# EUROPEAN PATENT APPLICATION

(11) **EP 1 881 081 A1**
(43) Date of publication of application: **23.01.2008**
(21) Application number: 06447094.1
(22) Date of filing: 20.07.2006
(51) Int. Cl.: C12Q 1/68

(54) **Combinations of markers for increased accuracy of diagnosis of rheumatoid arthritis**

(71) Applicant: Innogenetics N.V., 9052 Gent (BE)
(72) Inventor: Nuytinck, Lieve, 9031 Drongen (BE)
(74) Representative: Paemen, Liesbet R.J.

(57) **Abstract**

The present invention relates to methods for identifying a subject having, at risk of having or at risk of developing rheumatoid arthritis which comprise detecting the combined presence of a nucleic acid variant in a ficolin gene and another marker for rheumatoid arthritis in a subject. The invention further provides tools and kits for performing these methods and the use of these methods in differential diagnosis of arthritis and personalised therapy.

## Description

### FIELD OF THE INVENTION

The present invention relates to methods and kits for identifying a subject having, at risk of having, or at risk of developing, rheumatoid arthritis.

### BACKGROUND OF THE INVENTION

Rheumatoid arthritis (RA) is a chronic inflammatory disease, generally regarded as an autoimmune disorder, that affects up to 1% of the adult population. It is characterized primarily by inflammation of the peripheral joints, in many cases ultimately leading to destruction of these joints. However, RA is a systemic disease, as especially long-standing (and severe) cases also develop extra-articular manifestations of symptoms. As the structural damage is progressive and largely irreversible, it is important to diagnose RA as early as possible, to be able to start an adequate treatment. This holds especially true for patients at risk of or having severe RA, which is characterized e.g. by increased joint destruction (as measured by a higher radiological progression rate).

A correct diagnosis of RA is often difficult because the symptoms develop insidiously, or may resemble those of other diseases (e.g. osteoarthritis, arthritis due to infection or gout). Traditionally, RA is diagnosed using the revised American College of Rheumatology (ACR) classification criteria. According to the ACR, four of the following seven symptoms must be present; the first four have to be present for a minimum of six weeks:
- morning stiffness for at least one hour;
- arthritis of 3 or more of the following joints: right or left proximal interphalangeal (PIP), metacarpophalangeal (MCP), wrist, elbow, knee, ankle and metatarsophalangeal (MTP) joints;
- arthritis of wrist, MCP or PIP joint;
- symmetric involvement of joints;
- rheumatoid nodules over bony prominences, or extensor surfaces, or in juxta-articular regions;
- positive serum rheumatoid factor;
- radiographic changes including erosions or bony decalcification localized in or adjacent to the involved joints

The diagnosis according to this procedure is labour intensive and a significant amount of time passes before a definite diagnosis is made.

Several biochemical and genetic markers of RA have been proposed, allowing a more accurate diagnosis. Different autoantibodies are associated with RA; rheumatoid factor (RF), for instance, is detected in a majority of patients diagnosed with RA. Although presence of RF is part of the ACR criteria, it is not very specific: a significant group of patients are diagnosed with RA despite the absence of RF; and RF may also be present in subjects not having RA. Anti-citrullinated peptide antibodies were found to be more specific for RA. They include antiperinuclear factor, antikeratin antibodies and antifillagrin antibodies, all specific for epitopes containing citrulline, which is a post-translational modification of an arginine residue. These antibodies are highly specific for RA, and appear to have enough sensitivity to be clinically useful, although not all patients with RA react against the same citrullinated epitopes.

Genetic association studies have identified a link between several alleles and rheumatoid arthritis. The best studied association is that with the HLA-DRB1 shared epitope encoding allele. HLA (human leukocyte antigen) DRB1 is an antigen-presenting MHC type II molecule, thus implicated in immune processes. Several HLA-DRB1 alleles encode a conserved sequence at amino acid residues 70-74 (called the shared epitope or SE), and more than 90% of patients with severe RA carry at least one shared epitope encoding allele. Despite this significant association, its usefulness as a diagnostic marker for RA is hampered by the high prevalence of these alleles throughout the population (meaning also in subjects not having RA). Further combination of SE status with serologic markers did not increase diagnostic performance compared with the serologic markers alone (Hoffman et al., Clin Chem. 2005; 51(1):261-3).

Recently, a strong association was found between the PTPN22 R620W polymorphism and RA, especially RF positive RA. PTPN22 is a lymphoid specific phosphatase, also implicated in the immune response.

Ficolins are lectins that contain a collagen-like domain and a fibrinogen-like domain, which is similar to fibrinogen α- and γ-chains (Matsushita et al., J. Immunol. 2000; 164(5):2281-2284). Ficolins form oligomers of structural subunits, each of which is composed of three identical 35 kDa polypeptides. Each subunit is composed of an amino-terminal, cysteine-rich region, a collagen-like domain that consists of tandem repeats of Gly-Xaa-Yaa triplet sequences (where Xaa and Yaa represent any amino acid), a neck region, and a fibrinogen-like domain. The oligomers of ficolins comprise two or more subunits, especially a tetrameric form of ficolin has been observed.

Some of the ficolins trigger the activation of the complement system substantially in similar way as shown for other lectins. The fibrinogen-like domain of these ficolins has a function which is similar to that of the carbohydrate recognition domain (CRD) of C type lectins and accordingly these ficolins function as pattern-recognition receptors (PRR) to discriminate pathogens from self.

In human serum, two types of ficolin, known as L-ficolin (P35, ficolin L, ficolin 2 or hucolin) and H-ficolin (Hakata antigen, ficolin 3 or thermolabile b2-macroglycoprotein), have been identified, and both of them have lectin activity. L-ficolin recognises GlcNAc and H-ficolin recognises GalNAc. Another ficolin known as M-ficolin (P35-related protein, Ficolin 1 or Ficolin A) is not considered to be a serum protein and is found on leukocytes and in the lungs. The lectin activation of the complement system involves carbohydrate recognition by PRR, such as mannose binding lectin (MBL) and ficolins, and the subsequent activation of associated enzymes that are known as MBL-associated proteins (mannose binding protein serine protease or MASP). Both L-ficolin and H-ficolin activate the lectin-complement pathway in association with MASPs.

Hummelshoj T et al. (2003, Abstracts Molecular Immunology 40:171-228) identified a number of genetic polymorphisms in human ficolin genes, more particularly 7 polymorphisms in FCN1, 11 in FCN2 and 1 in FCN3. These are not the only known polymorphisms in ficolin genes however, as can be seen in the SNP database of NCBI. Currently, there are 90 polymorphisms identified in the FCN1 gene (63 of which are found in the human FCN1 gene), 60 polymorphisms in the FCN2 gene (all of them in the human gene) and 37 in the FCN3 gene (also all human).

Despite the different associations of genetic risk factors and autoantibodies with rheumatoid arthritis, there is a continuous search for more accurate genetic and/or biochemical markers that provide a reliable diagnosis or prediction of the risk to develop RA.

Moreover, it is has been found that, as for other diseases involving autoimmune reactions, subjects vary considerably in their susceptibility to rheumatoid arthritis and in their response to treatment thereof. These differences can be partially explained by polymorphisms of the genes encoding proteins involved in mediating and controlling the innate immune response, the inflammatory cascade, coagulation, and fibrinolysis. Molecular biology has revolutionized medicine by increasing our understanding of the pathophysiological mechanisms of disease and the ability to assess genetic risk. Recent data suggest an association between specific genotypes and the risk of adverse clinical outcomes.

The identification of allotypes and haplotypes linked with adverse clinical outcome in rheumatoid arthritis not only provides insight as to why the response to treatment varies amongst individuals, but can also potentially decrease morbidity and mortality through improved risk assessment and the administration of prophylactic or "personalized" medicine.

### SUMMARY OF THE INVENTION

It is an object of the present invention to provide methods and kits for identifying a subject having, at risk of having, or at risk of developing, rheumatoid arthritis.

It is an advantage of the present invention that a diagnosis of rheumatoid arthritis or of an increased susceptibility to developing rheumatoid arthritis can be made with improved accuracy, by the methods and tools of the invention. For example, the invention provides combinations of markers which ensure improved specificity for the diagnosis of rheumatoid arthritis or a susceptibility thereto.

It is a further advantage of the present invention that patients having been more accurately diagnosed with RA or a susceptibility to develop RA can be treated in a more timely and appropriate manner.

It is a further advantage of the present invention that subjects likely to develop a severe form of arthritis can be identified accurately and be treated in a more timely and appropriate manner, while at the same time excessive aggressive medication can be avoided for subjects identified as not likely to develop severe forms of the disease. Accordingly, the methods of the present invention can be applied in the differential diagnosis of arthritis and personalised therapy.

It is a further advantage of the present invention that the improved diagnosis can be performed by simple tests which can be developed into routine methods and standard kits.

It is a further advantage of particular embodiments of the present invention that the improved diagnosis can be performed using one analysis technique, in a time- and cost-efficient way.

A first aspect of the invention provides methods of gaining information about a subject having, or at risk of having, or developing rheumatoid arthritis, comprising:
- determining the presence of at least one nucleic acid variant in the FCN1 gene in that subject; and
- determining the presence of another marker for rheumatoid arthritis in said subject, the other marker being selected from the group consisting of: a HLA-DRB1 shared epitope encoding allele, a nucleic acid variant in the FCN2 gene, a nucleic acid variant in the PTPN22 gene, the presence of rheumatoid factor and the presence of anti-citrullinated peptide antibodies.

Another aspect of the invention provides methods of identifying a subject having, or at risk of having, or developing rheumatoid arthritis, comprising:
- determining the presence of at least one nucleic acid variant in a ficolin gene in that subject; and
- determining the presence of an other marker for rheumatoid arthritis in that subject, the other marker being selected from the group consisting of: a HLA-DRB1 shared epitope encoding allele, a nucleic acid variant in the PTPN22 gene, the presence of rheumatoid factor, the presence of anti-citrullinated peptide antibodies and the occurrence of a nucleic acid variant in another ficolin gene;
wherein the combined presence of the at least one nucleic acid variant in the ficolin gene and the other marker for rheumatoid arthritis is indicative of having or a risk of having or developing rheumatoid arthritis. Optionally, the method can be carried out in vitro or ex vivo.

According to a specific embodiment, the methods of the invention comprise the step of determining the presence of at least one nucleic acid variant in the FCN1 gene in that subject and determining the presence of an other marker for rheumatoid arthritis in that subject, the other marker being selected from the group consisting of: a HLA-DRB1 shared epitope encoding allele, a nucleic acid variant in the PTPN22 gene, the presence of rheumatoid factor, the presence of anti-citrullinated peptide antibodies and the occurrence of a nucleic acid variant in the FCN2 gene, wherein the combined presence of the at least one nucleic acid variant in the FCN1 gene and the other marker for rheumatoid arthritis is indicative of having or a risk of having or developing rheumatoid arthritis.

Specific embodiments of the above-cited methods of the invention comprise methods wherein the step of determining the occurrence of at least one nucleic acid variant in a ficolin gene in that subject comprises the step of determining the presence or absence of a single nucleotide polymorphism (SNP).

More specific embodiments, whereby the methods of the present invention involve the determination of a nucleic acid variant in the FCN1 gene, involve the detection of one or more SNPs located at position -1981 and/or position 7919 of the genomic DNA sequence of the FCN1 gene. Particularly, the SNP at position -1981 is a G/A polymorphism (dbSNP number rs2989727) and the SNP at position 7919 is an A/G polymorphism (dbSNP number rs1071583).

Further particular embodiments of the methods of the present invention involve determining whether the at least one nucleic acid variant in the ficolin gene, more particularly the at least one nucleic acid variant in the FCN1 gene, is present in 0, 1 or 2 copies, wherein the heterozygous or homozygous presence (or, where appropriate, absence) of the at least one nucleic acid variant is indicative of having or of a risk of having or developing rheumatoid arthritis.

In yet a further particular embodiment of these methods, for those subjects identified as at risk of developing RA based on the method of the present invention, homozygous presence (or absence) of a nucleic acid variant in the ficolin gene, more particularly homozygous presence of at least one nucleic acid variant in the FCN1 gene, is indicative of an increased risk of having or developing rheumatoid arthritis.

Specific embodiments of the methods of the present invention relate to the combined detection of a nucleic acid variant in a ficolin gene and the detection of the presence of a HLA-DRB1 shared epitope encoding allele, wherein the combined presence (or, where appropriate, absence) of the at least one nucleic acid variant in the ficolin gene and the presence of a HLA-DRB1 shared epitope encoding allele is indicative of having or a risk of having or developing rheumatoid arthritis.

In further specific embodiments, the methods comprise determining whether 0, 1 or 2 HLA-DRB1 shared epitope encoding alleles are present in the subject, wherein, for those subjects identified as at risk of developing RA based on the method of the present invention, the presence of 2 HLA-DRB1 shared epitope encoding alleles is indicative of an increased risk of having or developing rheumatoid arthritis.

Alternative specific embodiments of the methods of the present invention relate to the detection of the presence (or, where appropriate, absence) of a nucleic acid variant in a ficolin gene and of a nucleic acid variant in the PTPN22 gene, wherein the combined presence (or absence) of the at least one nucleic acid variant in a ficolin gene and a nucleic acid variant in the PTPN22 gene is indicative of having or a risk of having or developing rheumatoid arthritis.

In further specific embodiments, the methods comprise determining the presence of a nucleic acid variant in the PTPN22 gene, more particularly determining the occurrence of a SNP at position 1858 of the PTPN22 gene, most particularly the C/T polymorphism (dbSNP number rs2476601) at position 1858 of the PTPN22 gene.

In yet an alternative specific embodiment, the methods of the present invention relate to the combined detection of a nucleic acid variant in at least two ficolin genes, more particularly in the FCN1 and in the FCN2 gene.

In further specific embodiments, the methods comprising detecting a nucleic acid variant in the FCN2 gene comprise determining the absence of a SNP at position -64 of the genomic DNA sequence of the FCN2 gene and/or the absence of a SNP at position 6424 of the genomic DNA sequence of the FCN2 gene. Most particularly, the methods comprise determining the absence of an A/C polymorphism (dbSNP number rs7865453) at position -64 of the FCN2 gene and/or the absence of a G/T polymorphism (dbSNP number rs7851696) at position 6424 of the FCN2 gene. Other variants in the FCN2 gene which are detected in the methods of the methods of the present invention are the presence of a SNP at position -4 of the FCN2 gene (more particularly rs17514136) and/or the presence of a SNP at position 6359 of the FCN2 gene (more particularly rs17549193).

Further specific embodiments of the above-described methods comprises determining the combined presence of a nucleic acid variant in a ficolin gene and the presence of another genetic marker for rheumatoid arthritis, which other marker is the presence or absence of a nucleic acid variant in another ficolin gene or the presence of a nucleic acid variant in the PTPN22 gene, wherein the step of determining the other genetic marker for rheumatoid arthritis comprises determining whether the other marker for rheumatoid arthritis is occurs in 0, 1 or 2 copies. In these methods, homozygous presence (or absence) of the other genetic marker for RA is indicative of an increased risk of having or developing rheumatoid arthritis.

Further specific embodiments of the invention are methods comprising detecting the combined presence of a nucleic acid variant in a ficolin gene and a biochemical marker, such as rheumatoid factor or anti-citrullinated peptide antibodies.

Specific embodiments of the present invention relate to methods for determining the presence of a first marker which is a nucleic acid variant in a ficolin gene, such as FCN1 and the presence of a second marker for rheumatoid arthritis, to determine the combined presence of the first and the second marker, whereby the detection of the first and/or second marker is performed by one or more methods selected from the group consisting of DNA or RNA hybridization, sequencing, PCR, primer extension, multiplex ligation-dependent probe amplification (MLPA), oligonucleotide ligation assay (OLA) and/or restriction site analysis. More particularly the detection of a nucleic acid variant of the FCN1 gene is performed by DNA or RNA hybridization, sequencing, PCR, primer extension, multiplex ligation-dependent probe amplification (MLPA), oligonucleotide ligation assay (OLA) and/or restriction site analysis. Additionally or alternatively, the methods of the detection of the first and/or second marker in the methods of the present invention is performed by a method selected from the group consisting of ELISA, immunoblotting, immunofluorescence, an agglutination test, nephelometry or an immuno-assay. According to a particular embodiment, the methods of the present invention are performed in vitro on a biological sample of a subject of which the susceptibility to RA is to be determined.

In another aspect of the present invention methods and tools of the present invention are used for the identification of a subject having, at risk of having or at risk of developing an increased severity of rheumatoid arthritis. More particularly, susceptibility to increased severity of RA is determined based on the combined presence of a nucleic acid variant in the FCN1 gene and another marker for RA.

Yet another aspect of the present invention provides reagents, combinations of reagents and kits for the diagnosis of a subject having or a risk of having or developing rheumatoid arthritis, which kits comprise: one or more reagents for detecting the presence of at least one nucleic acid variant in a ficolin gene, such as in the FCN1 gene, and one or more reagents for specifically detecting the presence of an other marker for rheumatoid arthritis.

Particular embodiments of the kits of the present invention include one or more reagents for detecting the presence of a nucleic acid variant in the FCN1 gene and one or more reagents for detecting the presence of another genetic or biochemical marker for RA, which marker is selected from the group consisting of: a HLA-DRB1 shared epitope encoding allele, a nucleic acid variant in the PTPN22 gene, rheumatoid factor, anti-citrullinated peptide antibodies and the presence (or, where appropriate absence) of a nucleic acid variant in another ficolin gene, such as a nucleic acid variant in the FCN2 gene.

Specific embodiments of the kits of the present invention comprise (a) one or more allele specific primers or oligonucleotide probes for detecting the presence of at least one nucleic acid variant in the FCN1 gene, more particularly for detecting a nucleic acid variant at position -1981 and/or position 7919 of the genomic DNA sequence of the FCN1 gene; and (b) one or more allele specific primers or oligonucleotide probes for detecting a HLA-DRB1 shared epitope encoding allele.

Further specific embodiments of the kits of the present invention comprise: (a) one or more allele specific primers or oligonucleotide probes for detecting the presence of at least one nucleic acid variant in the FCN1 gene, such as one or more allele specific primers or oligonucleotide probes for detecting nucleic acid variants at position -1981 and/or position 7919 of the genomic DNA sequence of the FCN1 gene; and (b) one or more allele specific primers or oligonucleotide probes for detecting a nucleic acid in the PTPN22 gene, such as one or more allele specific primers or oligonucleotide probes for detecting a SNP at position 1858 of the PTPN22 gene, most particularly a C/T polymorphism (dbSNP number rs2476601) at position 1858 of the PTPN22 gene.

Further specific embodiments of the kits of the present invention comprise: (a) one or more allele specific primers or oligonucleotide probes for detecting the presence of at least one nucleic acid variant in the FCN1 gene, such as one or more allele specific primers or oligonucleotide probes for detecting nucleic acid variants at position -1981 and/or position 7919 of the genomic DNA sequence of the FCN1 gene; and (b) one or more allele specific primers or oligonucleotide probes for detecting the presence (or absence) of at least one nucleic acid variant in the FCN2 gene, such as one or more allele specific primers or oligonucleotide probes for detecting the presence of a SNP at position -64 of the genomic DNA sequence of the FCN2 gene, most particularly a C/A polymorphism at position -64 of the genomic DNA sequence of the FCN2 gene (dbSNP number rs7865453) and/or a SNP at position 6424 of the genomic DNA sequence of the FCN2 gene, most particularly a T/G polymorphism (dbSNP number rs7851696) at position 6424 of the genomic DNA sequence of the FCN2 gene.

Further specific embodiments of the kits of the present invention comprise: (a) one or more allele specific primers or oligonucleotide probes for detecting the presence of at least one nucleic acid variant in the FCN1 gene, such as one or more allele specific primers or oligonucleotide probes for detecting nucleic acid variants at position -1981 and/or position 7919 of the genomic DNA sequence of the FCN1 gene; and (b) one or more synthetic peptides, antigens or antibodies for detecting anti-citrullinated peptide antibodies or rheumatoid factor.

Further specific embodiments of the kits of the present invention comprise: (a) an antibody for detecting the presence of a first marker which is at least one nucleic acid variant in a ficolin gene, such as the FCN1 gene, and (b) at least one reagent for specifically detecting the presence of a second marker for rheumatoid arthritis, wherein the second marker for rheumatoid arthritis is selected from the group consisting of: a HLA-DRB1 shared epitope encoding allele, a nucleic acid variant in the PTPN22 gene, rheumatoid factor, anti-citrullinated peptide antibodies, or a nucleic acid variant in another ficolin gene, such as a nucleic acid variant in the FCN2 gene.

In specific embodiments, the at least one reagent for specifically detecting the presence of a second marker for rheumatoid arthritis of the kits of the invention described above comprises one or more allele specific primers or oligonucleotide probes for detecting a HLA-DRB1 shared epitope encoding allele. In further embodiments, the at least one reagent for specifically detecting the presence of a second marker for rheumatoid arthritis of the kits of the invention described above comprises one or more allele specific primers or oligonucleotide probes for detecting a SNP at position 1858 of the PTPN22 gene, most particularly a C/T polymorphism at position 1858 of the PTPN22 gene (dbSNP number rs2476601).

In further specific embodiments, the at least one reagent for specifically detecting the presence of a second marker for rheumatoid arthritis of the kits of the invention described above comprises one or more allele specific primers or oligonucleotide probes for detecting a SNP at position -64 of the genomic DNA sequence of the FCN2 gene, most particularly a C/A polymorphism at position -64 of the FCN2 gene (dbSNP number rs7865453) and/or a SNP at position 6424 of the genomic DNA sequence of the FCN2 gene, most particularly a T/G polymorphism at position 6424 of the FCN2 gene (dbSNP number rs7851696).

In further specific embodiments, the at least one reagent for specifically detecting the presence of a second marker for rheumatoid arthritis of the kits of the invention described above comprises one or more synthetic peptides, antigens or antibodies for detecting anti-citrullinated peptide antibodies or rheumatoid factor.

In yet another aspect of the present invention methods and tools of the present invention are used for the identification of a subject at risk of having or developing a modified response to rheumatoid arthritis therapy. More particularly, the likeliness of a subject to have a modified response to rheumatoid arthritis therapy is determined based on the combined presence of a nucleic acid variant in the FCN1 gene and another marker for RA.

Accordingly, methods are provided for identifying a subject at risk of having or developing a modified response to rheumatoid arthritis therapy, said method comprising:
- determining the presence of at least one nucleic acid variant in the FCN1 gene in said subject; and
- determining the presence of an other marker for rheumatoid arthritis in that subject, the other marker being selected from the group consisting of:
   a HLA-DRB1 shared epitope encoding allele, a nucleic acid variant in the FCN2 gene, a nucleic acid variant in the PTPN22 gene, or the presence of anti-citrullinated peptide antibodies;
   wherein the combined presence of the at least one nucleic acid variant in the FCN1 gene and the other marker identifies whether a subject is at risk of having or developing a modified response to rheumatoid arthritis therapy.

Yet another aspect of the present invention provides methods of treating a subject having an increased risk of having or developing rheumatoid arthritis, said method comprising
- determining the presence of at least one nucleic acid variant in a first ficolin gene, more particularly in the FCN1 gene in that subject; and
- determining the presence of an other marker for rheumatoid arthritis in that same subject, the other marker being selected from the group consisting of: a HLA-DRB1 shared epitope encoding allele, a nucleic acid variant in the PTPN22 gene, rheumatoid factor, the presence of anti-citrullinated peptide antibodies or the presence of a nucleic acid variant in another ficolin gene, such as a nucleic acid variant in the FCN2 gene;
wherein the combined presence of the at least one nucleic acid variant in the first ficolin gene, more particularly in the FCN1 gene and the other marker identifies whether a subject is at risk of, or has rheumatoid arthritis; and
- treating the so-identified subject with medicaments against rheumatoid arthritis.

The present description including the Examples is to be understood in conjunction with the following Figures intended to illustrate the invention without implying any limitation of the invention to the specific embodiments described therein.

### FIGURE LEGENDS

- Figure 1:: Genomic DNA sequence of the FCN1 gene, taken from the reverse complemented strand of NT_019501, position 709841-720740 (10900 bp). Exons are shaded in grey, the positions of rs2989727 and rs1071583 are indicated by boxes, the start and stop codon of the coding sequence are underlined.
- Figure 2:: Genomic DNA sequence of the FCN2 gene, taken from NT_019501, position 680465-689164 (8700 bp). Exons are shaded in grey, the positions of rs7865453, rs17514136, rs17549193 and rs7851696 are indicated by boxes, the start and stop codon of the coding sequence are underlined.
- Figure 3:: Genomic DNA sequence of the FCN3 gene, taken from the reverse complemented strand of NT_004610, position 10519501 to 10527700 (8200 bp). Exons are shaded in grey, the start and stop codon of the coding sequence are underlined.
- Figure 4:: Occurrence of the SNP rs2989727 vs. the occurrence of 0, 1 or 2 shared epitope encoding alleles and the corresponding positive predictive value (PPV) of RA according to an embodiment of the invention; continuous line: homozygous presence of the mutant FCN1 allele; dotted line: heterozygous presence of the mutant allele; dashed line: homozygous presence of the wild-type allele.
- Figure 5:: Occurrence of the SNP rs1071583 vs. the presence of 0, 1 or 2 shared epitope encoding alleles and the corresponding positive predictive value (PPV) of RA according to an embodiment of the invention; continuous line: homozygous presence of the mutant FCN1 allele; dotted line: heterozygous presence of the mutant allele; dashed line: homozygous presence of the wild-type allele.
- Figure 6:: Predictive probability of RA for the combination of the presence of anti-citrullinated peptide antibodies (expressed in PepA scan values) and 0 ("0"), 1 ("1") or 2 ("2") mutant FCN1 alleles (rs2989727) according to an embodiment of the invention.

### DETAILED DESCRIPTION OF THE INVENTION

Unless defined otherwise, all technical and scientific terms used herein have the same meaning as commonly understood by one of ordinary skill in the art. In any case the meaning of a term should not be limited to less than would be commonly understood by one of ordinary skill. The examples are only illustrative and not limiting.

### Definitions

As used herein the term "ficolin gene(s)" refers to one or more of the genes selected from the group consisting of the ficolin 1 gene (M-ficolin; FCN1), the ficolin 2 gene (L-ficolin; FCN2) and the ficolin 3 gene (H-ficolin; FCN3) and homologues thereof.

A homologue of a ficolin gene is a gene encoding a protein having at least about 60%, particularly 70%, more particularly 80%, most particularly at least about 90% sequence similarity or sequence identity with a wild type ficolin protein in a stretch of at least 30 amino acids, more particularly 50 amino acids, especially 100 amino acids, most particularly over the entire length of the shortest of the two proteins and which contains a collagen-like domain and a fibrinogen-like domain.

The term "variants" as used herein referring to a ficolin gene is to be understood as being any sequence having between 95 and 99.9% sequence identity to a wild type ficolin gene. Naturally occurring variants will generally have between 98 and 99.9% sequence identity with each other.

The percentage of sequence identity between two nucleotide or amino acid sequences as referred to herein can be determined by aligning the two sequences, and determining the number of positions with identical nucleotides or amino acids, divided by the total number of nucleotides or amino acids in the shorter of the sequences. The alignment of two nucleotide sequences is performed by the algorithm as described by Wilbur and Lipmann (1983) Proc. Natl. Acad. Sci. U.S.A. 80:726, using a window size of 20 nucleotides, a word length of 4 nucleotides, and a gap penalty of 4.

Two amino acids are considered as "similar" if they belong to one of the following groups GASTCP; VILM; YWF; DEQN; KHR. Thus, the percentage of sequence similarity between two protein sequences as referred to herein can be determined by aligning the two protein sequences and determining the number of positions with identical or similar amino acids divided by the total number of amino acids in the shorter of the sequences.

The term "ficolin protein" as used herein refers to any one of the proteins selected from the group consisting of ficolin 1, ficolin 2 and ficolin 3 (i.e. all isoforms thereof), and homologues or variants thereof.

The term "homologue" when referring to a ficolin protein" refers to a protein having at least 60%, particularly 70%, more particularly 80%, most particularly 90% sequence similarity or sequence identity with a wild type ficolin protein in a stretch of at least 30 amino acids, more particularly 50 amino acids, especially 100 amino acids, most particularly over the entire length of the shortest protein and which contains a collagen-like domain and a fibrinogen-like domain.

A "variant" of a ficolin protein is a ficolin protein comprising a sequence having between about 95 and about 99% sequence identity to a wild type ficolin protein.

Unless otherwise specified, a "gene" refers not only to the coding sequence, but to all sequences that are part of that gene: the introns and exons, the regulatory regions including the promoter region and possible other regulatory sequences, such as 5'UTR, 3'UTR or sequences further up- or downstream.

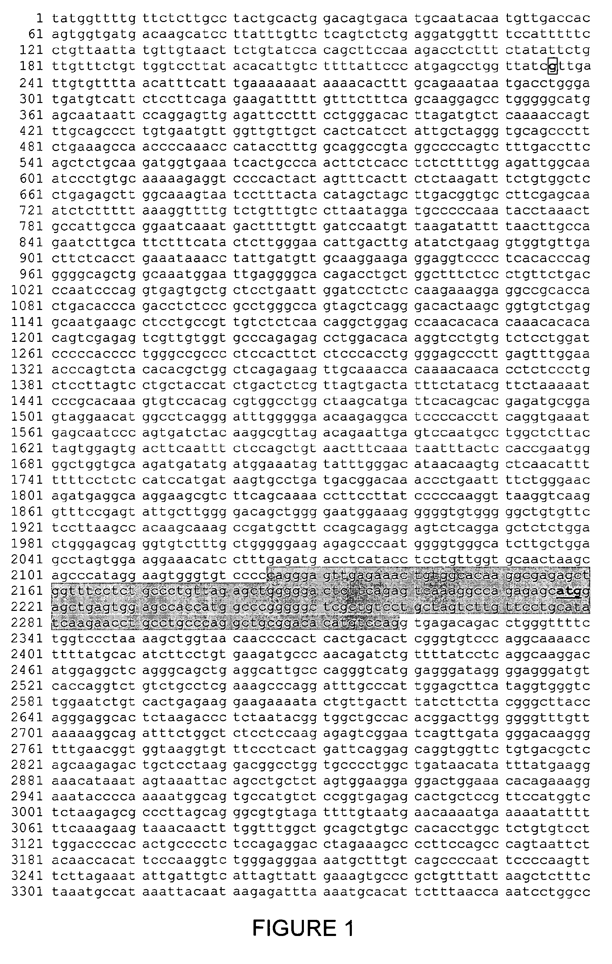

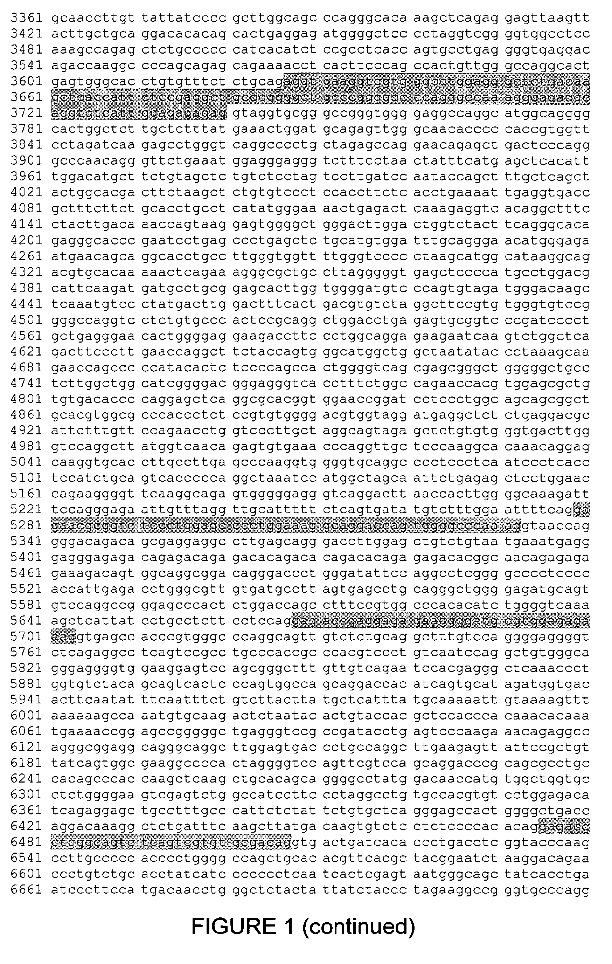

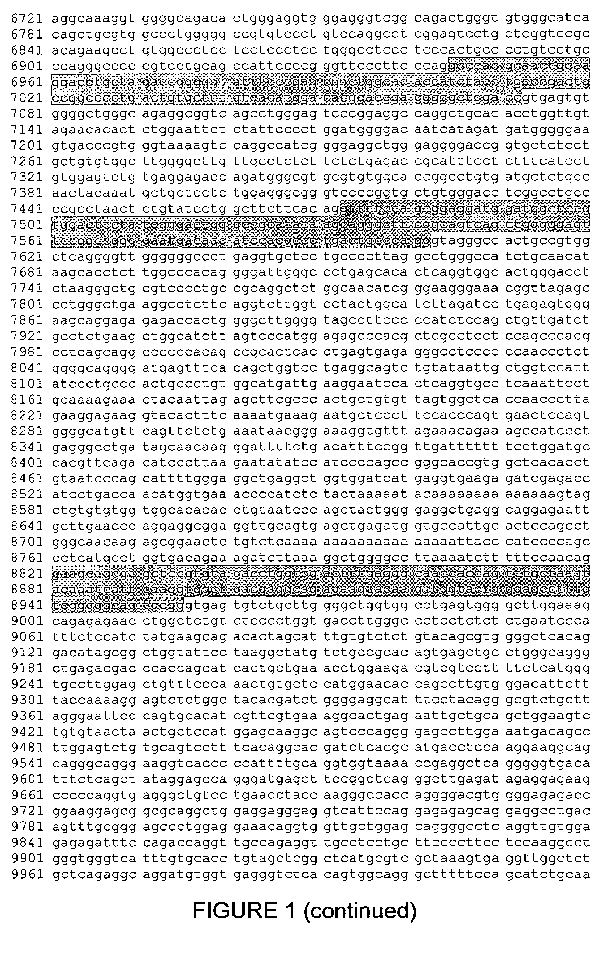

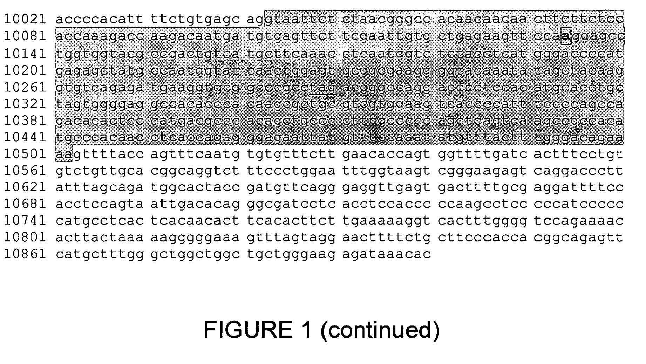

As used herein the term "FCN1 gene" or "ficolin 1 gene" refers to the reference nucleic acid sequence NT_019501 (gDNA; Version: NT_019501.13, Gl:89030070; SEQ ID NO: 1 and Figure 1)), and naturally occurring variants thereof. The mRNA reference sequence is NM_002003 (version NM_002003.2, GI:8051583. The reference FCN1 protein sequence is NP_001994 (version NP_001994.2, GI:8051584).

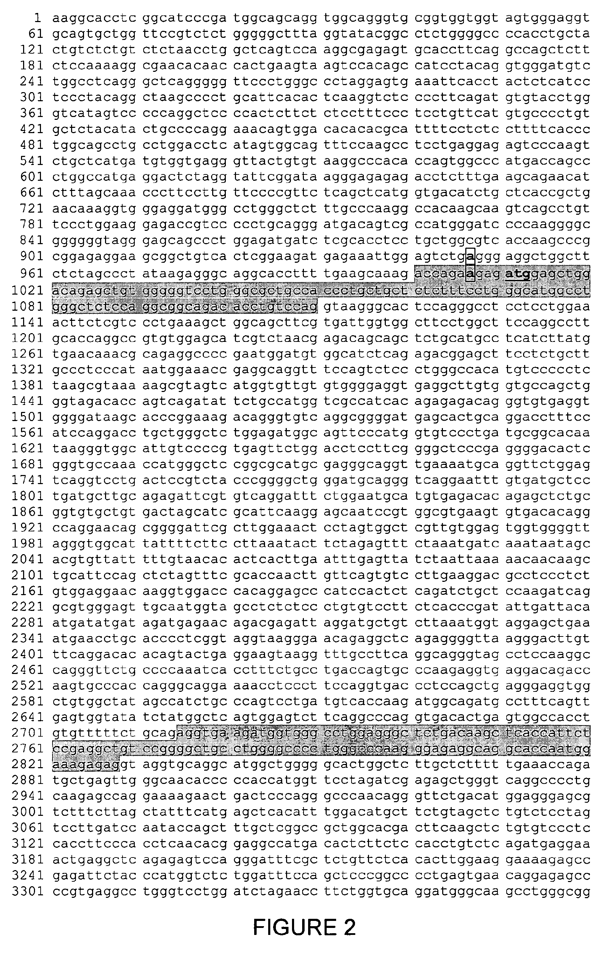

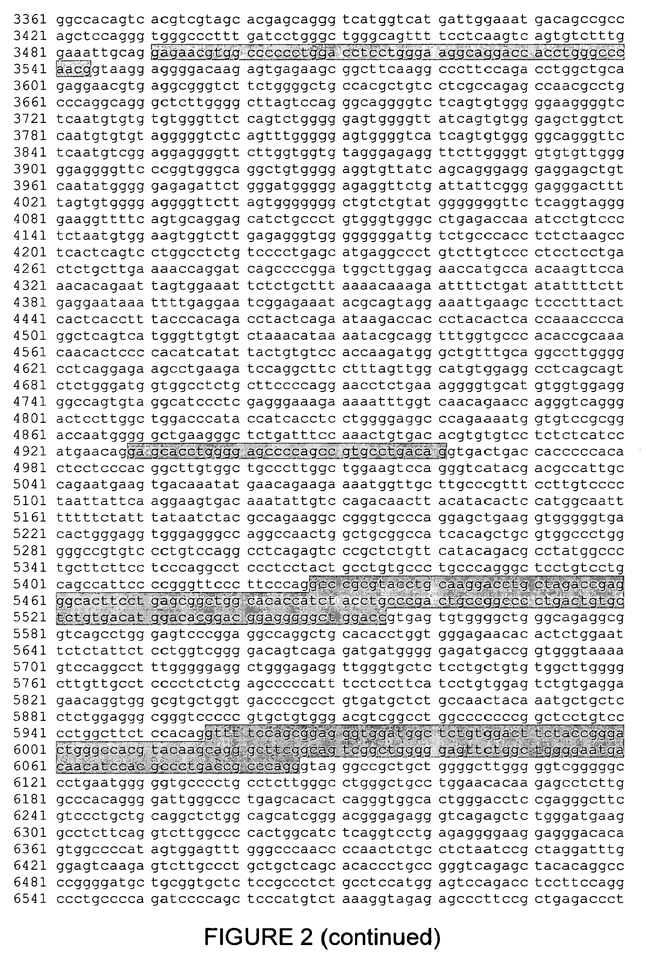

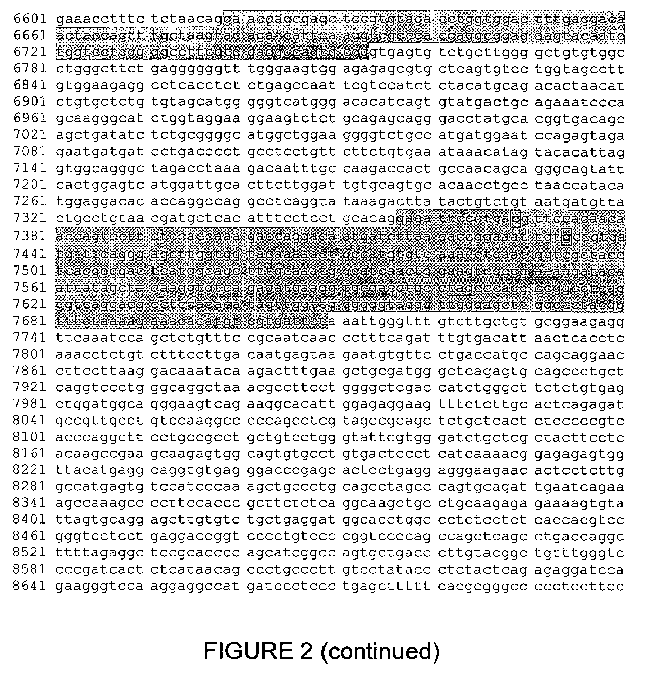

As used herein the term "FCN2 gene" or "ficolin 2 gene" refers to the reference nucleic acid sequence NT_019501 (gDNA; Version: NT_019501.13, Gl:89030070; SEQ ID NO: 2 and Figure 2) and naturally occurring variants thereof. Four different transcripts of the FCN2 gene arising from alternative splicing, and encoding different isoforms of the FCN2 protein, have been described. The splice variant SV0 is the most predominant FCN2 gene transcript in the liver and encodes a protein of 313 amino acids. It represents the longest FCN2 protein, isoform a. The reference mRNA sequence is NM_004108, and the protein encoded thereby is NP_004099. The splice variant SV1 is a minor FCN2 gene transcript in the liver, and results from the deletion of exon 2. Since the reading frame is unchanged by this splicing event, it encodes a shorter protein of 275 amino acids. The reference mRNA sequence is NM_015837, and the protein encoded thereby is NP_056652. The splice variant SV2 is a minor FCN2 gene transcript in the liver, and is generated by the persistence of the fifth intron between exons 5 and 6. It encodes the same 143 amino acids in the amino-terminal end as the SV0 transcript variant, plus an additional 39 amino acids from the intron sequence. The reference mRNA sequence is NM_015838, and the protein encoded thereby is NP_056653. The splice variant SV3 is a minor FCN2 gene transcript in the liver, and is generated by the persistence of introns 4 and 5. Exons 1-4 of this transcript encode a truncated protein of 102 amino acids, followed by an in-frame stop codon at the beginning of intron 4. The reference mRNA sequence is NM_015839, and the protein encoded thereby is NP_056654.

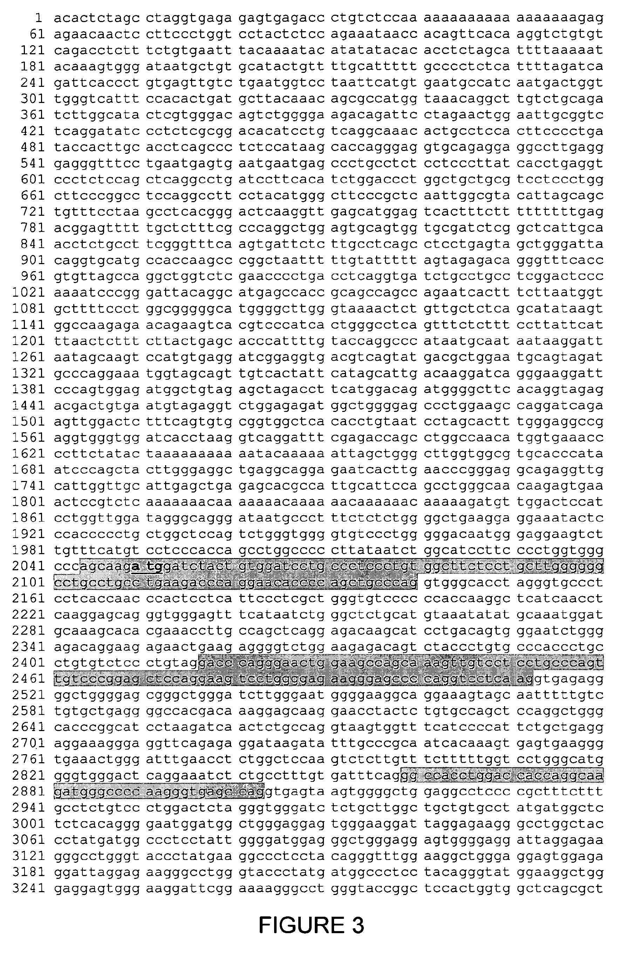

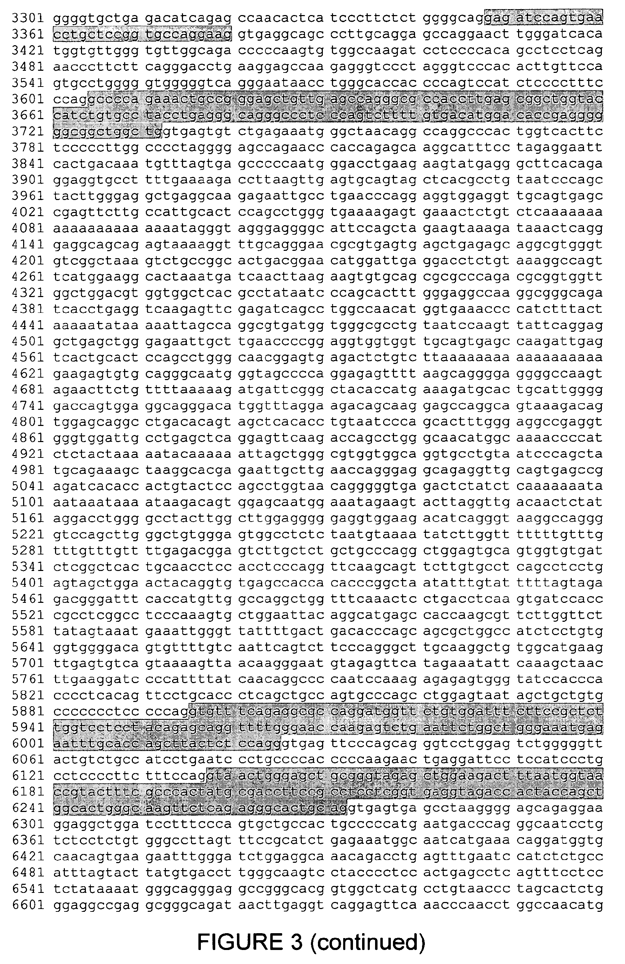

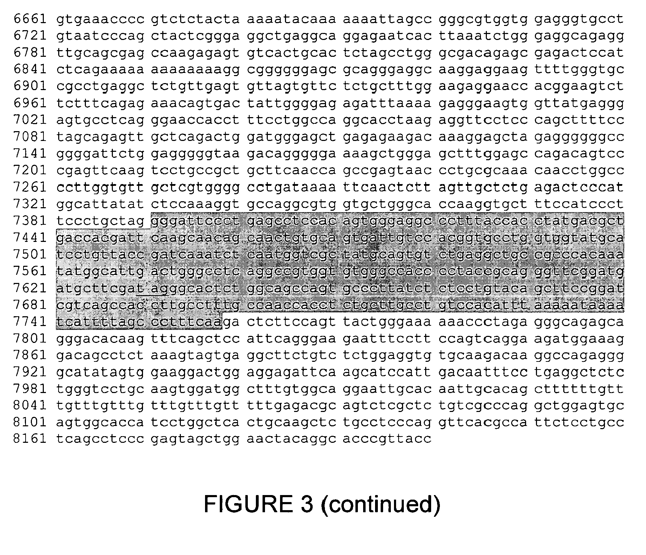

As used herein the term "FCN3 gene" or "ficolin 3 gene" refers to the the reference nucleic acid sequence NT_004610 (gDNA; Version: NT_004610.18; Gl:88942574; SEQ ID NO: 3 and Figure 3) and naturally occurring variants thereof. Alternative splicing occurs at this locus and two variants, each encoding a distinct isoform, have been identified. Transcript Variant 1 represents the longer transcript and encodes the longer isoform 1. The reference mRNA sequence is NM_003665. The protein encoded thereby is NP_003656. Transcript Variant 2 lacks an alternate in-frame exon, compared to variant 1, resulting in a shorter protein (isoform 2) that lacks an internal segment compared to isoform 1. The reference mRNA sequence is NM_173452 and the protein encoded thereby is NP_775628.

The term "HLA-DRB1 shared epitope encoding allele" or "HLA-DRB1 SE encoding allele" as used herein refers to one of a specific set of alleles of the human leukocyte antigen (HLA) beta chain (DRB1) genes, located on chromosome 6, which all encode a common sequence of amino acids (residues 70 to 74), generally referred to as the shared epitope (SE). There are three well described variations of the amino acid sequence of the shared epitope, referred to herein as HLA-DRB1-SE A corresponding to the sequence QRRAA (SEQ ID NO: 4), HLA-DRB1-SE B, corresponding to sequence QKRAA (SEQ ID NO: 6) and HLA-DRB1-SE C, corresponding to sequence RRRAA (SEQ ID NO: 8), which have been linked to rheumatoid arthritis. The sequence DERAA (SEQ ID NO: 10), herein also referred to as HLA-DRB1-SE D is not associated with having or an increased risk of developing RA and appears to have a protective effect.

The term "protein tyrosine phosphatase N22" or "PTPN22" as used herein is used to refer to the gene corresponding to the reference nucleic acid sequence NT_019273 (gDNA, version NT_019273.18, Gl:88942992). The PTPN22 gene on chromosome 1 encodes a protein tyrosine phosphatase which is expressed primarily in lymphoid tissues. Alternative splicing of this gene results in two transcript variants encoding distinct isoforms. Transcription variant 1 encodes the longer isoform which is 116 amino acids longer than isoform 2 and has a distinct C-terminus. The reference mRNA sequence is NM_015967 (SEQ ID NO: 11), the encoded protein is NP_057051. Transcript variant 2 uses an alternative splice site within the coding region, resulting in a frameshift and use of an upstream stop codon, as compared to variant 1. The reference mRNA sequence is NM_012411, the protein sequence NP_036543.

The term "Rheumatoid factor" as used herein refers to a kind of immunoglobulin (most often IgM, but sometimes also IgG or IgA) which can bind to the Fc portion of other antibodies in the body. Rheumatoid factor is an autoantibody.

As used herein, the term "wild-type" sequence is analogous to the reference sequence. The reference nucleic acid and protein sequences indicated in the current invention are derived from GeneBank (NCBI) and indicated by their respective accession number, as is well known to the person skilled in the art. The nomenclature for the nucleotide and amino acid changes as used herein is generally .accepted and recommended by den Dunnen and Antonarakis (Hum. Mut. 2000; 15:7-12). Frequent updates of the nomenclature for the description of sequence variations are provided on the web-site of the Human Genome Variation Society.
Accordingly, the nucleotide numbering of the coding DNA and RNA reference sequence is as follows:
• nucleotide +1 is the A of the ATG-translation initiation codon
• there is no nucleotide 0
• the nucleotide 5' of the ATG-translation initiation codon is -1.
To avoid confusion, the nucleotide number is preceded by "g." when a genomic or by "c." when a cDNA reference sequence is used. Substitutions are designated by ">".

The term "nucleic acid" refers to a single stranded or double stranded nucleic acid sequence and may consist of deoxyribonucleotides or ribonucleotides, nucleotide analogues or modified nucleotides, or may have been adapted for therapeutic purposes. There is no limitation in length. A nucleic acid that is about 100 nucleotides or less in length is often also referred to as an oligonucleotide.

The term "nucleic acid variant" or "polymorphism" as used in the present invention, refers to a position comprising one or more nucleotides in the nucleic acid sequence which differs relative to a reference nucleic acid sequence.

The most simple nucleic acid polymorphism is a polymorphism affecting a single nucleotide, i.e. a single nucleotide polymorphism or SNP. Nucleic acid polymorphisms further include any number of contiguous and/or non-contiguous differences in the primary nucleotide sequence of the nucleic acid under investigation relative to the primary nucleotide sequence of one or more reference nucleic acids. The term "polymorphic position" or "position" refers to the nucleic acid position at which a nucleic acid polymorphism arises. Nucleic acid sequences comprising at least one such polymorphism are referred to as "polymorphic nucleic acid sequences", "polymorphic polynucleotides", "polymorphic sequences" or the like. The polymorphism or nucleic acid variant can be an insertion, deletion, substitution, tandem repeat or similar.

The phrase "determining the presence", e.g. of a marker as used herein, refers to determining whether or not the relevant genetic, physiological and/or biochemical event, linked with the occurrence of a disease is present. In practice, both the absence and the presence of a certain event can function as markers. Accordingly, reference to determining the presence of a nucleic acid variant or a biochemical marker for rheumatoid arthritis, generally encompasses determining whether the marker is present, either based on the absence or the presence of the variant or biochemical marker in a sample. Moreover, this also includes the possible finding that the marker is not present in the sample, i.e. determining the absence (or presence) of a nucleic acid variant or a biochemical marker. In both cases determining the presence the marker can also be done indirectly, e.g., where the presence of a nucleic acid variant is linked to disease, the occurrence of this marker can (besides the direct detection of the nucleic acid variant) also be done by determining the homozygous presence of the corresponding allele not comprising the nucleic Similarly, allele specific oligonucleotide primers or probes for detecting the presence of a SNP can be specific for the allele where the SNP is not present.

The term "haplotype" means a particular pattern of sequential polymorphisms found on a single chromosome. As used herein, the term "allele" is one of several alternative forms of a gene or DNA sequence at a specific chromosomal location (locus). At each autosomal locus an individual possesses two alleles, one inherited from the father and one from the mother. The term "genotype" means the genetic constitution of an individual, either overall or at a specific locus, and defines the combination of alleles the individual carries. The term "homozygous" refers to having two of the same alleles at a locus; the term "heterozygous" refers to having different alleles at a locus. The term "allotype" refers to any of the genetically determined variants in the constant region of a given subclass of an immunoglobulin that is detectable as an antigen by members of the same species having a different constant region.

The term "Positive Predictive Value (PPV)" as used herein refers to the percentage of people with a positive test result who actually have the disease. The positive predictive value can be expressed as the number of true positives / (the number of true positives + false positives).

The term "Predictive probability" as used herein refers to the possibility to predict the disease status (diseased or not diseased) of the patient based on the test result (positive or negative). It includes the PPV as well as the NPV (negative predictive value). The NPV can be expressed as the number of true negatives / (the number of true negatives + false negatives).

The term "specificity" when referring to an assay herein refers to the probability of the test finding no disease among those who do not have the disease or the proportion of people free of a disease who have a negative test. Specificity can be expressed as the number of true negatives / (the number of true negatives + false positives).

The term "sensitivity" when referring to an assay herein refers to the probability of the test finding disease among those who have the disease or the proportion of people with disease who have a positive test result. Sensitivity can be expressed as the number of true positives / (the number of true positives + false negatives).

The term "rheumatoid arthritis" (RA) or "rheumatoid disease" as used herein refers to the autoimmune disease that causes chronic inflammation of the organs in the body, most particularly, the joints and the tissue around the joints. In rheumatoid arthritis, multiple joints are usually inflamed in a symmetrical pattern (both sides of the body affected). The small joints of both the hands and wrists are often involved. Also the small joints of the feet are often involved. More rarely, the cricoarytenoid joint is involved, causing a hoarseness of voice. The term RA however also covers conditions wherein the inflammation affects organs and areas of the body other than the joints, such as the glands of the eyes and mouth, this can cause dryness of these areas, referred to as Sjögren's syndrome. Rheumatoid inflammation of the lung lining (pleuritis) causes chest pain with deep breathing or coughing. The lung tissue itself can also become inflamed and sometimes nodules of inflammation (rheumatoid nodules) develop within the lungs. Inflammation around the heart (pericarditis) can cause a chest pain that typically changes in intensity when lying down or leaning forward. Rheumatoid disease can reduce the number of red blood cells (anemia), and white blood cells. Decreased white cells can be associated with an enlarged spleen (referred to as Felty's syndrome) and can increase the risk of infections. Firm lumps under the skin (rheumatoid nodules) can occur around the elbows and fingers where there is frequent pressure. Even though these nodules usually do not cause symptoms, occasionally they can become infected. A rare, serious complication, usually with long-standing rheumatoid disease, is blood vessel inflammation (vasculitis). Vasculitis can impair blood supply to tissues and lead to tissue death. This is most often initially visible as tiny black areas around the nail beds or as leg ulcers.

The terms "severe rheumatoid arthritis" or "increased severity RA" as used herein refers arthritis wherein the typical symptoms of RA are more pronounced. Often, severe forms of RA are associated with the occurrence of extra-articular manifestations, such as the rheumatoid nodules described above or inflammatory bowel disease. Typically, patients with mild disease have fewer than six joints involved, no bone erosion on X-rays and no RA activity outside of the joints. Patients with moderate disease have 6-20 involved joints and may have joint space narrowing or erosions on X-rays. Severe RA patients have more than 20 joints involved, anemia, rapid joint destruction on X-rays and RA activity outside the joints.results in restricted mobility and severe pain.

When referring to the presence of "anti-citrullinated peptide antibodies" or "ACPAs" herein, it is intended to refer to the presence, in a sample, of one or more antibodies selected from a group of RA associated autoantibodies that specifically target epitopes in which arginine has been converted by peptidylarginine deiminase into citrulline during a post-translational modification. ACPAs include antiperinuclear factor, antikeratin antibodies and antifilaggrin antibodies. The presence of anti-citrullinated peptide antibodies can be determined using natural or synthetic citrullinated peptides as described herein.

As used herein, an "immuno-assay" is an assay that utilizes a specific antibody to detect an antigen of interest or utilizes a specific antigen to detect an antibody of interest. An immuno-assay is thus characterized by detection of the specific binding of an antigen to an antibody.

The term "biological sample" means a tissue sample or a body fluid sample. A tissue sample includes (but is not limited to) buccal cells, a brain sample, a skin sample or organ sample (e.g. liver). The term "body fluid" refers to all fluids that are present in the body including but not limited to blood, plasma, serum, synovial fluid, lymph, urine, saliva or cerebrospinal fluid. The biological sample may also be obtained by subjecting it to a pre-treatment if necessary, for example, by homogenizing or extracting. Such a pre-treatment may be selected appropriately by those skilled in the art depending on the biological sample to be subjected.

The present invention is to be understood in conjunction with the Sequence Listing which contains the following sequences:
- SEQ ID NO: 1:: FCN1 genomic sequence
- SEQ ID NO: 2:: FCN2 genomic sequence
- SEQ ID NO: 3:: FCN3 genomic sequence
- SEQ ID NO: 4:: HLA-DRB1 shared epitope (A)
- SEQ ID NO: 5:: degenerate DNA sequence encoding a HLA-DRB1 shared epitope (A)
- SEQ ID NO: 6:: HLA-DRB1 shared epitope (B)
- SEQ ID NO: 7:: degenerate DNA sequence encoding a HLA-DRB1 shared epitope (B)
- SEQ ID NO: 8:: HLA-DRB1 shared epitope (C)
- SEQ ID NO: 9:: degenerate DNA sequence encoding a HLA-DRB1 shared epitope (C)
- SEQ ID NO: 10:: amino acid residues 70-74 of a HLA-DRB1 protein which are not a shared epitope (D)
- SEQ ID NO: 11:: mRNA sequence encoding PTPN22
- SEQ ID NO: 12:: oligonucleotide primer for amplifying a region of the FCN1 gene
- SEQ ID NO: 13:: oligonucleotide primer for amplifying a region of the FCN1 gene
- SEQ ID NO: 14:: oligonucleotide primer for amplifying a region of the FCN1 gene
- SEQ ID NO: 15:: oligonucleotide primer for amplifying a region of the FCN1 gene
- SEQ ID NO: 16:: oligonucleotide probe for detecting a SNP in the FCN1 gene
- SEQ ID NO: 17:: oligonucleotide probe for detecting a SNP in the FCN1 gene
- SEQ ID NO: 18:: oligonucleotide probe for detecting a SNP in the FCN1 gene
- SEQ ID NO: 19:: oligonucleotide probe for detecting a SNP in the FCN1 gene
- SEQ ID NO: 20:: oligonucleotide probe for detecting a SNP in the FCN1 gene
- SEQ ID NO: 21:: oligonucleotide probe for detecting a SNP in the FCN1 gene
- SEQ ID NO: 22:: oligonucleotide probe for detecting a SNP in the FCN1 gene
- SEQ ID NO: 23:: oligonucleotide probe for detecting a SNP in the FCN1 gene
- SEQ ID NO: 24:: DNA sequence encoding a HLA-DRB1 shared epitope (A)
- SEQ ID NO: 25:: DNA sequence encoding a HLA-DRB1 shared epitope (B)
- SEQ ID NO: 26:: DNA sequence encoding a HLA-DRB1 shared epitope (C)
- SEQ ID NO: 27:: DNA sequence encoding a HLA-DRB1 shared epitope (C)
- SEQ ID NO: 28:: DNA sequence of an allele encoding HLA-DRB 1 residues 70-74, which do not form a shared epitope (D)
- SEQ ID NO: 29: degenerate DNA sequence encoding residues 70-74 of HLA-DRB1, which do not form a shared epitope (D)

The present invention provides methods and tools for identifying subjects having, at risk of having, or at risk of developing rheumatoid arthritis.

The "subject" on which the method of the present invention can be carried out can be any vertebrate animal, more particularly any mammalian animal, and is most particularly a human. It is envisaged that the methods of the invention can be applied to a non-human subject such as (but not limited to) a cow, a pig, a sheep, a goat, a horse, especially racing horses, a monkey, a rabbit, a dog, a cat, a mouse, a rat, a hamster or a primate or any laboratory test animal.

In a first aspect, the present invention provides methods for identifying a subject at risk of having, having, or at risk of developing RA based on a combined determination of the presence in that subject of at least one nucleic acid variant in a ficolin gene and the presence of another marker for rheumatoid arthritis.

According to the present invention, determination of the occurrence of a polymorphism in a ficolin gene, in combination with determination of the presence of another suitable rheumatoid arthritis marker, allows the identification of a subject at risk of having or developing rheumatoid arthritis with improved accuracy, compared to the determination of the ficolin polymorphisms or the other markers alone. More particularly, the suitable arthritis markers in the context of the present invention are selected from the group consisting of: the presence of one or more HLA-DRB1 shared epitope encoding alleles, the presence of a nucleic acid variant in the PTPN22 gene, the detection of Rheumatoid factor (RF) and the presence of anti-citrullinated peptide antibodies.

According to a particular embodiment of the methods of the invention, the determination of the presence of one or more polymorphisms of a ficolin gene is ensured at the DNA level. Most particularly, the determination includes determining the occurrence of one variant allele of a ficolin gene (heterozygous for the variant ficolin allele) or the presence of two variant alleles of a ficolin gene (homozygous for the presence the variant ficolin allele). Determination of the occurrence of a variant ficolin allele at the DNA level is described in more detail below.

Additionally or alternatively, the determination of the presence of a variant ficolin allele can be performed at the protein level, based on quantitative expression of the expressed protein, qualitative assessment of the expressed ficolin protein (differences in protein sequence) and/or a functional assessment of the expressed ficolin protein (e.g. the ability to bind to GIcNAc). Methods for determining serum concentrations and altered function of a ficolin protein are described, for instance in Hummelshoj et al. (Hum Mol Genet. 2005;14(12):1651-8).

In a first embodiment, the methods of the present invention involve the determination of one or more polymorphisms in a ficolin gene, more particularly the determination of a polymorphism in the FCN1 gene. In a particular embodiment, the polymorphism is located within the FCN1 gene as identified in SEQ ID NO: 1, or in the corresponding cDNA or RNA sequence.

According to a particular embodiment, the polymorphism in the ficolin gene is one that is associated with the occurrence of RA, with an odds ratio of at least 1.2, 1.3 or 1.4, more specifically at least 1.5. Most particularly the lower limit of the confidence interval for this odds ratio is >1. According to a particular embodiment, the occurrence of RA is determined based on ACR criteria.

According to yet a further particular embodiment, the polymorphism in the FCN1 gene is one that is located in Exon 9, most particularly at position 7919 of the genomic DNA (gDNA) sequence. More specifically, the FCN1 variant determined in the methods of the present invention is a nucleic acid variant which is an A to G single nucleotide polymorphism at position 7919 (also referred to as g.7919A>G, herein; which includes complement T>C) that does not change the amino acid sequence (designated as Q275Q). This FCN1 variant is identified as rs1071583 (dbSNP NCBI).

Additionally or alternatively, specific embodiments of the methods of the present invention involve the identification of a polymorphism of the FCN1 gene located in the promoter region. More specifically, the FCN1 variant determined in the methods of the present invention is a nucleic acid variant which is present at nucleic acid position -1981 of the gDNA sequence. More particularly, this nucleic acid variant is a C to T polymorphism at position - 1981 (also referred to as g.-1981C>T herein; which includes complement G>A), identified as rs2989727 in the SNP database.

Within the FCN1 gene, a common haplotype has been identified. This haplotype was found in approximately 41% of investigated individuals. FCN1 Haplotype: FCN1-rs2989727 A allele --------FCN1-rs1071583 G allele.

Thus, according to a particular embodiment of the methods of the invention, the step of determining the presence of at least one nucleic acid variant in the FCN1 gene in a subject involves detecting whether one or more single nucleotide polymorphism (SNP) are present. In a specific embodiment, the methods involve the identification of one or more SNPs at position -1981 and/or position 7919 of the genomic DNA sequence of the FCN1 gene. In yet a further embodiment, the SNP at position -1981 of the FCN1 gene (located in the promoter region) is a G to A polymorphism, and the presence of at least one allele with an A nucleotide at that position is an indication that the subject carrying such allele is at risk of having or developing RA. In another specific embodiment, the SNP at position 7919 of the FCN1 gene (located in exon 9) is an A to G polymorphism and the presence of at least one allele with a G nucleotide at that position is an indication that the subject carrying such allele is at risk of having or developing RA.

In a second embodiment, the methods of the invention involve the combined determination of one or more polymorphisms in a ficolin gene which is the FCN2 gene and another marker for rheumatoid arthritis. In a particular embodiment, the polymorphism in the ficolin gene is located within the FCN2 gene as identified in SEQ ID NO: 2, or in the corresponding cDNA or RNA sequence.

In a specific embodiment of the invention, the methods involve determining the presence of at least one SNP in the FCN2 gene. The FCN2 gene has 8 exons. Exon 8 contains a nucleic acid variant at nucleic acid position 6424 of the gDNA sequence. The nucleic acid variant is g.6424G>T which results in the amino acid change A258S and is identified as rs7851696 in the NCBI SNP database. It is noted that for this polymorphism, the wild-type allele is associated with a risk of having or developing rheumatoid arthritis. Accordingly, methods involving the determination of a polymorphism at position 6424 of the gDNA sequence involve determining the absence of nucleic acid variant g.6424G>T, whereby the combined absence of this nucleic acid variant and the positive determination of another marker, is indicative of a risk of having or developing rheumatoid arthritis.

Exon 8 furthermore contains a nucleic acid variant at position 6359 of the gDNA sequence, which is g. 6359C>T and which results in the amino acid change T236M, identified as rs17549193 in the SNP database (dbSNP).

The promoter region of the FCN2 gene is also polymorphic. A nucleic acid variant has been identified at position -64 of the gDNA sequence. This nucleic acid variant is g.64A>C and is identified as rs7865453 (dbSNP). It is noted that also for this polymorphism, the wild-type allele is associated with a risk of having or developing rheumatoid arthritis. Accordingly, methods involving the determination of a polymorphism at position -64 of the gDNA sequence involve determining the absence of nucleic acid variant g.64A>C, whereby the combined absence of this nucleic acid variant and the positive determination of another marker, is indicative of a risk of having or developing rheumatoid arthritis.

A further nucleic acid variant is identified in the 5'UTR of the FCN2 gene, i.e. at position -4 of the gDNA sequence. More specific, the nucleic acid variant is -4A>G (dbSNP rs17514136).

Within the FCN2 gene, two common haplotypes were found using four DNA variations included in this invention.

Haplotype FCN2.1 occurred in 23% of normal individuals and is presented by:
FCN2-rs7865453 C allele ------------ FCN2 rs7851696 T allele

A second haplotype FCN2.2 occurred in 22% of normal individuals and is presented by: FCN2 -4A>G G-allele-------------FCN2 ex8-T236M T allele.

Accordingly, particular embodiments of the methods of the present invention relate to the determination of the absence of a SNP at position -64 and/or the presence of a SNP at position -4 and/or position 6359 and/or position 6424 of the genomic DNA sequence of the FCN2 gene. Most particularly, the methods involve determining the absence of an A/C polymorphism at position -64 (dbSNP nr. rs7865453), the presence of an A/G polymorphism at position -4 (dbSNP rs17514136), the presence of a C/T polymorphism at position 6359 (dbSNP rs17549193) and/or the absence of a G/T polymorphism at position 6424 (dbSNP nr. rs7851696).

In a further embodiment, the methods of the invention comprise the step of determining whether the one or more nucleic acid variants in the ficolin gene are present in 0, 1 or 2 copies, more particularly whether a nucleic acid variant in the ficolin gene is present in one or in both alleles. Thus, according to this embodiment it is determined not only whether an allele carrying the nucleic acid variation is present or absent, but if it is present, it is also determined whether there is only one allele carrying the nucleic acid variant (heterozygous presence) or whether both alleles have the nucleic acid variant (homozygous presence). Either heterozygous or homozygous presence of the at least one nucleic acid variant, in combination with the presence of another marker for rheumatoid arthritis, is indicative of having or a risk of having or developing rheumatoid arthritis.

It has been determined that the homozygous presence of a nucleic acid variant of the ficolin gene is more strongly indicative of the risk of having or developing RA than the identification of only one nucleic acid variant allele. This implies that a subject having two copies of a nucleic acid variant of a ficolin gene, in combination with another marker for rheumatoid arthritis is at higher risk of developing or having RA, or has an increased susceptibility to RA, than a subject carrying one copy of the nucleic acid variant in combination with another marker for rheumatoid arthritis, who in turn is at higher risk of developing or having RA than a subject not having the nucleic acid variant in combination with another marker for rheumatoid arthritis.

According to yet a further embodiment of the present invention, the methods of the invention comprise determining, in the genotype the presence of 0, 1 or 2 alleles comprising one or more nucleic acid variants of a ficolin gene, or where the absence of the nucleic acid variant is linked to rheumatoid arthritis, determining the absence of 1 or 2 alleles comprising the nucleic acid variant.

Different analytical procedures suitable for the detection of the presence or absence of the nucleic acid variants mentioned herein are known in the art.

Nucleic acid from any nucleated cell can be used as the starting point for such assay techniques and may be isolated according to standard nucleic acid preparation procedures well known to those of skill in the art. Many current methods for the detection of allelic variation are reviewed by Nollau et al. (Clin. Chem. 1997; 43(7):1114-1128), and in standard textbooks, for example "Laboratory Protocols for Mutation Detection", Ed. by U. Landegren, Oxford University Press, 1996 and "PCR", 2nd Edition" by Newton & Graham, BIOS Scientific Publishers Limited, 1997.

The step of determining the presence of a nucleic acid variant in a gene in the methods of the present invention can be carried out *in vivo* or *in vitro.* Most typically, however, detection of nucleic acid variants in the ficolin genes, more particularly in the FCN1 gene or the FCN2 gene are performed in *vitro* in a biological sample obtained from the subject.

Typically, a nucleic acid comprising a sequence of interest can be obtained from a biological sample, more particularly from a sample comprising DNA (e.g. gDNA or cDNA) or RNA (e.g. mRNA). Release, concentration and isolation of the nucleic acids from the sample can be done by any method known in the art. Currently, various commercial kits are available such as the QlAamp DNA Blood Kit from Qiagen (Hilden, Germany) for the isolation of nucleic acids from blood samples, or the 'High pure PCR Template Preparation Kit' (Roche Diagnostics, Basel, Switzerland) or the DNA purification kits (PureGene, Gentra, Minneapolis, US). Other, well-known procedures for the isolation of DNA or RNA from a biological sample are also available (Sambrook et al., Cold Spring Harbor Laboratory Press 1989, Cold Spring Harbor, NY, USA; Ausubel et al., Current Protocols in Molecular Biology 2003, John Wiley & Sons).

When the quantity of the nucleic acid is low or insufficient for the assessment, the nucleic acid of interest may be amplified. Such amplification procedures can be accomplished by those methods known in the art, including, for example, the polymerase chain reaction (PCR), ligase chain reaction (LCR), nucleic acid sequence-based amplification (NASBA), strand displacement amplification, rolling circle amplification, T7-polymerase amplification, and reverse transcription polymerase reaction (RT-PCR).

Accordingly the methods of the present invention optionally comprise the steps of isolating nucleic acids from the sample and/or an amplification step.

Numerous methods for detecting a single nucleotide anomaly in nucleic acid sequences are well-known in the art. The present invention is not limited by any particular method used to detect the target sequences disclosed herein. Examples of such methods are described by Gut (Hum. Mutat. 2001; 17:475-492) and Syvänen (Nat. Rev. Genet. 2001; 2:930-942), and include, but are not limited to, hybridization methods such as reverse dot blot, line probe assay (LiPA), GeneChip^{™} microarrays, dynamic allele-specific hybridization (DASH), peptide nucleic acid (PNA) and locked nucleic acid (LNA) probes, TaqMan^{™} (5'nuclease assay), and molecular beacons; allele-specific PCR methods such as intercalating dye, FRET primers, and Alphascreen^{™}; primer extension methods such as ARMS (amplification refractory mutation system), kinetic or real-time PCR, SNPstream^{™}, Genetic Bit Analysis^{™} (GBA), multiplex minisequencing, SnaPshot^{™}, Pyrosequencing^{™}, MassEXTEND^{™}, MassArray^{™}, GOOD assay, microarray minisequencing, APEX (arrayed primer extension), sequence specific priming (SSP), microarray primer extension, Tag arrays, coded microspheres, template-directed incorporation (TDI), fluorescence polarization; oligonucleotide ligation methods such as colorimetric OLA (oligonucleotide ligation assay), sequence-coded OLA, microarray ligation, ligase chain reaction, padlock probes, and rolling circle amplification; endonuclease cleavage methods such as restriction site analysis (RFLP) and Invader^{™} assay.

In a particular embodiment, the detection of the presence or absence of a nucleic acid variant is determined by DNA or RNA hybridization, sequencing, PCR, primer extension, multiplex ligation-dependent probe amplification (MLPA), oligonucleotide ligation assay (OLA) or restriction site analysis.

As indicated above, according to the present invention one or more of the above methods for the determination of a nucleic acid variant in a ficolin gene are combined with the determination of another marker for rheumatoid arthritis, so as to determine the combined presence of a nucleic acid variant in a ficolin gene and another marker.

Indeed, it has previously been demonstrated that the occurrence of nucleic acid variants in one or more of the ficolin genes, more particularly the occurrence of FCN1 and FCN2 nucleic acid variants, is associated with an altered risk of developing RA. In addition it has now been found that the determination of the combined presence of one or more nucleic acid variants in one of the ficolin genes and another genetic or biochemical marker indicative of a risk of having or developing RA allows a more accurate and reliable identification of a subject having or at risk of having or developing RA.

Suitable markers for determining the combined presence with the nucleic acid variants of a ficolin gene include the presence of HLA-DRB1 SE encoding alleles, the presence of nucleic variants in the PTPN22 gene, the presence of anti-citrullinated peptide antibodies and the presence of RF. Additionally it is envisaged that the improved diagnostic value is also obtained by determining the combined presence of different ficolin genes.

According to a particular embodiment, the methods of the invention comprise, in addition to the determination of the presence of a nucleic acid variant of a ficolin gene, the step of determining the presence of at least one HLA-DRB1 shared epitope encoding allele, whereby the methods comprise identifying a subject having or at risk of developing RA based on the combined presence of both markers. The human leukocyte antigen (HLA) genes, located on chromosome 6, are part of the major histocompatibility complex (MHC) and are involved in presentation of antigen to T cells. HLA-DRB1 is a specific MHC class II molecule that has many allelic variations. Some of these alleles show a strong association with RA, both with susceptibility to the disease as with severity. Those alleles all encode a highly similar sequence of amino acids (residues 70 to 74), generally referred to as the shared epitope (SE).

There are three well characterized variations of the amino acid sequence of the shared epitope associated with RA: QRRAA (HLA-DRB1-SE A, SEQ ID NO: 4), QKRAA (HLA-DRB1-SE B, SEQ ID NO: 6) and RRRAA (HLA-DRB1-SE C, SEQ ID NO: 8). The nature of residues 70 and 71 seems critical for the association with rheumatoid arthritis, as a HLA-DRB1 allele encoding the sequence DERAA (HLA-DRB1-SE D, SEQ ID NO: 10) appears to have protective effect, i.e. it occurs more frequently in healthy controls than in subjects having rheumatoid arthritis.

The association of the shared epitope with RA has long been known, and it is used as a diagnostic marker for RA, susceptibility to RA, or to assess the risk of severe RA. Moreover, the effect of the shared epitope encoding allele is thought to be dose-dependent: the presence of two alleles encoding the SE indicates that a subject is at higher risk of developing RA, having RA, or of developing severe RA when already diagnosed with RA, than a subject having only one SE encoding allele.

However, although the significant association between diagnosis of RA and SE status is recognized, the high prevalence of SE in controls limits its diagnostic usefulness. According to the present invention however, the combination of determining the presence of a HLA-DRB1 SE encoding allele, with the determination of the presence of a nucleic acid variant in a ficolin gene, more particularly in FCN 1, increases the accuracy of diagnosis of RA or susceptibility of developing RA. Indeed, the specificity of the assay increases, as the likeliness of a non-RA subject having the two markers decreases.

Accordingly, in one embodiment of the invention, methods are provided for identifying a subject at risk of, or having rheumatoid arthritis, wherein the combined presence of a nucleic acid variant in a ficolin gene, more particularly in the FCN1 gene, and the presence of a HLA shared epitope encoding allele is determined, to identify whether the subject is at risk of developing RA, or has RA. Accordingly, the methods of the present invention involve, determining the presence of a nucleic acid variant in a ficolin gene in a sample of a subject and simultaneously or consecutively, determining the presence of a HLA SE encoding allele in a sample of a subject and assessing whether the patient has, is at risk of having or at risk of developing rheumatoid arthritis based on the combined presence of a nucleic acid variant in a ficolin gene and a HLA SE encoding allele in the tested sample(s). More specific embodiments of the methods of the invention include methods which comprise the detection of the combined presence of a nucleic acid variant in the FCN1 gene and HLA-DRB1 SE encoding alleles, more particularly detecting the combined occurrence of either the SNP rs1071583 or the SNP rs2989727 of the FCN1 gene and HLA-DRB1 SE encoding alleles. Particular embodiments of the methods of the present invention comprise the step of determining the presence of one or more HLA-DRB1 SE encoding alleles which are alleles encoding at positions 70 to 74 of the human leukocyte antigen beta chain (HLA-DRB1) an amino acid sequence selected from the group consisting of: QRRAA (SEQ ID NO: 4), QKRAA (SEQ ID NO: 6) and RRRAA (SEQ ID NO: 8). According to a further specific embodiment, the methods of the invention comprise the step of determining the presence, in the nucleotide sequence encoding HLA-DRB1 sequence one of the sequences selected from the group consisting of SEQ ID NO: 5, SEQ ID NO: 7, and SEQ ID NO: 9. More particularly, this corresponds to determining the presence of a sequence selected from CAY WGV WGV GCN GCN (SEQ ID NO: 5), CAY AAY WGV GCN GCN (SEQ ID NO: 7), or WGV WGV WGV GCN GCN (SEQ ID NO: 9), whereby N = any nucleotide, W = A or C, Y = A or G and V = any nucleotide if the first nucleotide in codon is C; A or G if the first nucleotide in the codon is A, in the corresponding regions of the HLA-DRB1 SE encoding alleles.

According to this embodiment, the methods of the invention comprise determining the presence of a HLA-DRB1 encoding allele which has a protective effect, whereby presence of such an allele is indicative of a decreased risk of having or developing RA. Thus, alternatively or concomittantly, in the methods of the present invention, the presence of an allele encoding the sequence with protective effect on RA (DERAA, SEQ ID NO: 10) can be determined.

According to a further specific embodiment, the methods of the invention comprise the step of determining the presence, in the nucleotide sequence encoding HLA-DRB1, the presence of SEQ ID NO: 29. More particularly, this corresponds to determining the presence of GAS GAY WGV GCN GCN (SEQ ID NO: 29), whereby N = any nucleotide, S = T or C, W = A or C, Y = A or G and V = any nucleotide if the first nucleotide in codon is C; A or G if the first nucleotide in the codon is A, in the corresponding region of the HLA-DRB1 encoding allele.

It has further been determined, and is demonstrated herein, that the gene dosage effect of the HLA-DRB1 shared epitope encoding alleles is cumulative to the combined presence of a nucleic acid variant in a ficolin gene. Thus, a specific embodiment of the methods of the present invention comprises, determining the presence of a nucleic acid variant in a ficolin gene and determining whether there are 0, 1 or 2 HLA-DRB1 shared epitope encoding alleles present, wherein the presence of 2 HLA-DRB1 SE encoding alleles is indicative of an increased risk of having or developing rheumatoid arthritis. Accordingly, the methods of the present invention which comprise determining the genotype for the HLA-DRB1 SE encoding allele allows a further identification of patients having an increased susceptibility to RA. The increased risk of having or developing RA is associated with the presence of two HLA-DRB1 SE encoding alleles, which can be the same (homozygous) or different HLA-DRB1 SE encoding alleles. Accordingly a sample comprising 2 HLA-DRB1 alleles can comprise two different alleles encoding either the same or a different shared epitope. A particular embodiment of the methods of the present invention, the step of determining the presence of 0, 1 or 2 HLA-DRB1 SE encoding alleles (alternatively referred to as SE genotype = 0, 1 or 2 respectively), comprises determining the presence of 0, 1 or 2 alleles selected from the group consisting of alleles encoding, at amino acid position 70-74 of the HLA-DRB1 sequence, the sequence of SEQ ID NO: 4 (HLA-DRB1-SE1), SEQ ID NO: 6 (HLA-DRB1-SE2), or SEQ ID NO: 8 (HLA-DRB1-SE3), wherein the presence of 2 HLA-DRB1 SE encoding alleles is indicative of an increased risk of having or developing rheumatoid arthritis.

It has been established that the different HLA-DRB1 SE encoding alleles are differentially associated with the risk of having or developing rheumatoid arthritis. Accordingly, in yet a further embodiment of the methods of the present invention, the step of determining the presence of an HLA-DRB1 SE encoding allele comprises determining the number (i.e. 0, 1 or 2) and the nature (A, B, C, D or other) of the HLA-DRB1 SE allele(s) encoded, so as to further characterize the nature of the risk which has be identified. More particularly, the methods comprise the step of determining whether the HLA-DRB1-SE encoding allele(s) encode HLA-DRB1-SE A, HLA-DRB1-SE B or HLA-DRB1-SE C, so as to allow a further characterization of the susceptibility of the subject to having or developing RA, in accordance with Table 9 herein. Alternatively, the methods comprise the step of determining whether a HLA-DRB1-SE D encoding allele is present, which is associated with a decreased susceptibility of the subject to having or developing RA (see Table 8).

In a further embodiment of the methods of the invention, both the number of copies of the at least one nucleic acid variant in the FCN1 gene and the number of HLA-DRB1 SE encoding alleles are determined, to allow further stratification of the risk of developing or having rheumatoid arthritis.

The nature of the method used in the context of the present invention for determining the HLA genotype is not critical to the invention. Such methods are known to the skilled person and include many of the methodologies listed above for the determining of the presence of a nucleic acid variant of a ficolin gene. Particularly suited methods again include DNA or RNA hybridization, sequencing, PCR, primer extension, MLPA, OLA or restriction site analysis, or a combination thereof. An example of such combination is a line probe assay (LiPA), which combines hybridization and PCR. LiPA kits for the detection of the HLA genotype are commercially available, e.g. the InnoLiPA^{™} kit.

It is envisaged that the step of determining the presence of another marker for RA (here a HLA-DRB1 shared epitope encoding allele) of the methods of the present invention can be carried out either *in vivo* and/or *in vitro.* Most typically, however, detection is performed *in vitro* in a biological sample obtained from the subject which is to be diagnosed.

The methods of the present invention envisage both simultaneous and sequential determining of the presence of the nucleic acid variants in the ficolin gene and the presence of HLA-DRB1 SE encoding alleles. Accordingly, the methods of the invention include both methods wherein both markers are assessed using the same methodology and methods wherein each of the markers is determined by a different method. Accordingly, one or both markers can be determined *in vitro* or *in vivo.*

According to a particular embodiment of the present invention, the same sample (optionally divided in two) is used in both steps for the determination of each of the markers. Alternatively however, the methods of the invention involve determining the presence of a nucleic acid variant in a ficolin gene in a first sample and determining the presence of a second marker for RA in a second sample of a patient, wherein the nature of the sample can be the same or different. Typically however, the genotyping of both markers will be done starting from the same biological sample, although the sample may be processed differently, depending on the methods used to detect either marker.

The HLA shared epitope encoding alleles have not only been implicated in the susceptibility to rheumatoid arthritis, but are also associated with an increased risk of the development of severe RA. Based on the present observations, it is believed that the determination of the combined presence of one or more nucleic acid variants in a ficolin gene, more particularly in FCN1, and of one or more alleles encoding HLA-DRB1 shared epitopes can be used to identify whether the subject is at risk of having or developing an increased severity of RA.

In another embodiment, the methods of the present invention involving determining the combined presence of one or more nucleic acid variants in a ficolin gene, more particularly in FCN1, and one or more alleles encoding HLA-DRB1 shared epitopes are used to assess the risk of having or developing a modified response to rheumatoid arthritis therapy.

According to yet another embodiment of the invention, methods are provided wherein samples are analysed for the combined presence of one or more variants of a ficolin gene, more particularly FCN1, and the presence of a nucleic acid variant of the PTPN22 gene.

The PTPN22 gene, comprising 21 exons, has been described to contain a nucleic acid variant in exon 14 (present in both transcription variants), which is associated with an increased risk of having or developing RA. According to a particular embodiment, the methods of the invention comprise the step of determining the presence of the single nucleotide polymorphism at position 1858 of the cDNA sequence: c.1858C>T, resulting in the amino acid change R620W of the PTPN22 gene. Although there are two differing transcriptional variants (and thus 2 possible cDNA sequences), the SNP is at the same position in both sequences. For the purpose of the present invention, we herein define the reference nucleotide of the SNP as present in the reference mRNA sequence (SEQ ID NO: 11). Although this differs from the one present in the reference genomic sequence, this is in line with the way it is usually referred to. The wild type nucleotide at position c.1858 thus is C.

One embodiment of the methods of the invention thus comprises determining the combined presence (or absence) of a nucleic acid variant in a ficolin gene and at least one SNP in the PTPN22 gene

A specific embodiment of the methods of the invention comprises determining the combined presence of a nucleic acid variant in the FCN1 gene and at least one SNP in the PTPN22 gene, more particularly, the SNP in the PTPN22 gene located at position 1858 of the cDNA sequence of the PTPN22 gene. Most particularly, the SNP in the PTPN22 gene is a C/T polymorphism at position 1858 (dbSNP nr. rs2476601).

In a further embodiment, the methods of the present invention comprise determining the combined presence of a nucleic acid variant in a ficolin gene and at least one nucleic acid variant in the PTPN22 gene wherein the step of determining the presence of at least one nucleic acid variant in the PTPN22 gene comprises determining whether such a nucleic acid variant is present in 0, 1 or 2 copies, wherein homozygous presence of the nucleic acid variant in the PTPN22 gene is indicative of an increased risk of having or developing rheumatoid arthritis.

In the methods of the present invention, any suitable technique can be used for determining the presence or absence of the nucleic acid variant in the PTPN22 gene. For instance, the methods described above, suitable for the determination of the presence of a. nucleic acid variant in a ficolin gene, can be applied in the detection of a nucleic acid variant in the PTPN22 gene. In a specific embodiment, detecting the presence of a nucleic acid variant in the PTPN22 gene is determined by sequencing, hybridization, PCR, primer extension, MLPA, OLA or restriction site analysis.

As for the detection of the other markers described above, determining the presence of nucleic acid variants in the PTPN22 gene can be carried out *in vivo* or *in vitro.* Most typically, however, detection of is performed *in vitro* in a biological sample obtained from the subject.

Again, the combined determation of the occurrence of a nucleic acid variant in the ficolin gene, more particularly in the FCN1 gene, and the nucleic acid variant in the PTPN22 gene does not need to be performed simultaneously or in the same way. Accordingly, the sample may be processed differently, depending on the methods used to detect either marker. Accordingly, methods are envisaged wherein different samples from the same subject are used to determine the presence of both markers, or wherein the detection of the presence of one marker is performed *in vitro* and the other *in vivo.* Typically however, the genotyping of both markers will be done starting from the same biological sample, more particularly using the same technique, most particularly allowing simultaneous determination of both markers.

According to yet a further embodiment of the invention, methods are provided wherein samples are analysed for the combined presence of one or more variants of a ficolin gene, more particularly FCN1, and the presence of a biochemical marker for RA. Suitable biochemical markers which can be used in the context of the present invention include the presence of anti-citrullinated peptide antibodies and Rheumatoid factor (RF).

One specific embodiment of the methods of the present invention envisages the detection of the combined presence (or, where appropriate, absence) in a sample of at least one nucleic acid variant in a ficolin gene and the occurrence of anti-citrullinated peptide antibodies.

Anti-citrullinated peptide antibodies (or ACPAs) form a group of RA associated autoantibodies that specifically target epitopes in which arginine is converted by peptidylarginine deiminase into citrulline during a post-translational modification. ACPAs include antiperinuclear factor, antikeratin antibodies and antifilaggrin antibodies. They are used as biomarkers for RA, with increased specificity compared to RF, and recently it was demonstrated that they also enhance severity of disease (Kuhn et al., J. Clin. Invest. 2006, 116(4): 961-973). It has nevertheless been found that the predictive value of the presence of anti-citrullinated peptide antibodies is further increased, when combined with the presence of at least one nucleic acid variant of a ficolin gene, more particularly when combined with the presence of a nucleic acid variant in the FCN1 gene.

Thus, in a further embodiment of the invention, methods are provided for identifying a subject at risk of having or developing RA, comprising determining the presence of at least one nucleic acid variant in a ficolin gene, more particularly in the FCN1 gene in said subject; and determining the presence of anti-citrullinated peptide antibodies, whereby the combined presence (or, where appropriate, absence) of the nucleic acid variant in a ficolin gene and of anti-citrullinated peptide antibodies is used to identify those subjects having, at risk of having or developing RA. More specific embodiments of the methods of the invention include methods which comprise the detection of the combined presence of a nucleic acid variant in the FCN1 gene and anti-citrullinated peptide antibodies, more particularly detecting the combined occurrence of either the SNP rs1071583 or the SNP rs2989727 of the FCN1 gene and anti-citrullinated peptide antibodies.

The present invention further provides methods for identifying a subject having or at risk of developing increased severity of RA. According to one embodiment of this aspect of the invention, the combined presence of a nucleic acid variant in a ficolin gene, more particularly in the FCN1 gene and anti-citrullinated peptide antibodies is indicative of a risk of having or developing an increased severity of RA. Similarly, the methods can be used to assess the risk of having or developing a modified response to rheumatoid arthritis therapy, whereby the combined presence (or where appropriate, absence) of a nucleic acid variant in a ficolin gene and anti-citrullinated peptide antibodies is indicative of a modified response to rheumatoid arthritis therapy.

Different methods have been described for determining the presence of ACPAs in a subject, and the nature of the method used in the context of the present invention is not critical to the invention. According to one embodiment, the detection of these antibodies is done by serological tests using synthetic citrullinated substrates.

According to a specific embodiment, the methods of the invention comprise the detection of the presence of anti-citrullinated peptide antibodies in a sample by immunoassay. Immunoassays for anti-citrullinated peptide antibodies can be based on detecting the binding with an antigen known to be recognized by these antibodies, i.e. a natural or synthetic citrullinated peptide. Binding of the anti-citrullinated peptide antibodies to the antigen can be detected e.g. by a labelled secondary antibody, e.g. a fluorescent labelled secondary antibody. Immuno-assays may be either competitive or non-competitive. Non-competitive immunoassays are assays in which the amount of captured analyte is directly measured. In competitive assays, the amount of analyte present in the sample is measured indirectly by measuring the amount of an added (exogenous) analyte displaced (or competed away) from a capture agent by the analyte present in the sample. Suitable immunological methods include but are not limited to enzyme-linked immunosorbent assays (ELISA), immunoblotting, immunospotting (such as line immunoassays or LIA), radioimmunoassays (RIA), fluid or gel precipitation reactions, immunodiffusion (single or double), agglutination assays, immunoelectrophoresis, time-resolved immunofluorometric assay (TRIFMA), Western blots, liposome immunoassays, complement-fixation assays, immunoradiometric assays, fluorescent immunoassays, protein A immunoassays or immunoPCR. An overview of different immunoassays is given in Wild (Nature Press, 2001, London, UK), Ghindilis et al. (Humana Press, 2002, Totowa, NJ, US) and Kilpatrick (Transfusion Medicine, 2002; 12:335-351).

Several ELISAs have been developed: e.g. using a single cyclic citrullinated peptide (CCP) recognised by anti-CCP1 antibodies (e.g. the Immunoscan RA anti-CCP test kit (Euro-Diagnostica B.V., Arnhem, The Netherlands), or using citrullinated recombinant rat filaggrin or deiminated fibrinogen. The presence of ACPAs can also be assessed using indirect immunofluorescence, using fluorescent-labelled secondary antibodies. Based on the epitope mapping of human filaggrin, two synthetic citrullinated peptides have been developed, termed peptide A and B (pepA and pepB), for the detection of anti-pepA and anti-pepB antibodies in a line immuno-assay based test.

The methods of the present invention envisage determining the presence of ACPAs either *in vivo* or *in vitro.* Most typically, however, detection of is performed *in vitro* in a biological sample obtained from the subject.

Yet a further specific embodiment of the methods of the present invention envisages the detection of the combined presence (or, where appropriate, absence) in a sample of at least one nucleic acid variant in a ficolin gene and the occurrence of Rheumatoid factor.

Rheumatoid factor (RF) is an immunoglobulin which can bind to the Fc portion of other antibodies. This autoantibody is not normally found in healthy subjects, but is associated with several conditions, infections and autoimmune diseases (e.g. osteomyelitis, hepatitis, mononucleosis, systemic lupus erythematosus), but most notably with rheumatoid arthritis. Even though it is not highly specific for rheumatoid arthritis, and not all patients diagnosed with RA are RF positive, it is one of the most common markers used to diagnose RA and presence of RF is part of the ACR criteria. RA patients are commonly categorised as either RF positive or RF negative (the distribution being 80% and 20% of RA cases, respectively), whereby RF+ patients usually have a worse prognosis, i.e. a higher chance of developing severe RA. Due to its low specificity however, presence of RF alone is not conclusive for an accurate diagnosis of RA.

According to this embodiment of the invention methods are provided wherein the combined presence (or, where appropriate, absence) of a nucleic acid variant of a ficolin gene and the occurrence of RF is used to identify a subject having, at risk of having, or at risk of developing rheumatoid arthritis. Accordingly, the present invention relates to methods for identifying, within the population of RF+ patients, subjects with a further increased risk of having or developing RA.

Different methods for determining the presence of RF have been described, including the agglutination tests (e.g. a Waaler-Rose assay or sheep cell agglutination test, or a test with latex particles coated with human IgG), rate or laser nephelometry, ELISA, capillary precipitation, or an immuno-assay.

Yet another embodiment of the invention provides methods wherein the identification of a subject having, at risk of having or at risk of developing RA is based on the combined presence of a nucleic acid variant in two or more ficolin genes, or where the absence of a nucleic acid variant in a ficolin gene is linked to RA, the combined presence of a nucleic acid variant in a first ficolin gene and absence of a nucleic acid variant in another ficolin gene. Thus, according to this embodiment, the methods comprise the determination of the occurrence of a nucleic acid variant in at least two different ficolin genes, whereby the combined occurrence/absence of two or more nucleic acid variants linked to RA in at least two ficolin genes is considered as indicative of RA or the risk of developing RA.

More particular embodiments of the invention provide methods and tools for identifying subjects having or at risk of having or developing RA based on the combined presence (and/or absence) of a nucleic acid variant in the FCN1 gene and the FCN2 gene.

Further particular embodiments of the invention relate to methods which comprise determining the combined occurrence of either the SNP rs1071583 or the SNP rs2989727 in the FCN1 gene and one or more of the nucleic acid variants in the FCN2 gene selected from the group consisting of the absence of SNP rs7865453, the absence of SNP rs7851696,. The presence of SNP rs17514136 and the presence of SNP rs17549193.

While the methods of the invention are particularly suited for identifying a subject at risk of having or developing rheumatoid arthritis, another aspect of the invention envisages the use of the provided methods to identify a subject at risk of having or developing an increased severity of rheumatoid arthritis. Increased severity of RA is typically associated with more pronounced structural joint damage, typically measured by radiology. Assessing severity of RA, or the likelihood of developing severe RA, is important to determine which therapy is most appropriate, or will have most effect.

RA usually requires lifelong treatment, including medications, physical therapy, exercise, education, and possibly surgery. A multitude of different medications are available (including disease-modifying anti-rheumatic drugs (DMARDs) such as methotrexate or leflunomide; non-steroidal antiinflammatory drugs (NSAIDs), such as ibuprofen or indomethacin; COX-2 inhibitors; antimalarial medications such as hydroxychloroquine and sulfasalazine; immune-suppressing drugs such as azathioprine, cyclophosphamide or TNF or IL-1 inhibitors; and corticosteroids), but many of these are associated with detrimental side effects. The use of such aggressive medications or therapies is only advised if the benefits outweigh the disadvantages. For example, drugs that suppress the immune system are normally reserved for severe RA patients who have failed other therapies.

Therefore, another aspect of the invention is the use of the present methods to identify a subject at risk of having or developing a modified response to rheumatoid arthritis therapy, as patients having or developing severe RA usually only respond to aggressive therapies. An increasing number of studies confirms that some immunotherapies fail to work in patients with mutations in specific genes, while others stay effective. Identifying the most effective treatment by first determining the presence of specific allotypes, haplotypes or genotypes may lead to a considerable decrease in morbidity. The methods of the invention thus provide a tool for improved risk assessment and may help to determine the most appropriate, "personalized" therapy.

Another aspect of the invention relates to a kit for identifying a subject at risk of having or developing rheumatoid arthritis, comprising one or more reagents for detecting the presence (or, where appropriate absence) of at least one nucleic acid variant in a ficolin gene, and one or more reagents for detecting the presence of an other marker for rheumatoid arthritis. More particularly the kits of the present invention provide the tools for the detection of one or more variants in a ficolin gene and at least one other marker for RA being selected from the group consisting of a HLA-DRB1 shared epitope encoding allele, a nucleic acid variant in the PTPN22 gene, anti-citrullinated peptide antibodies, RF, or a nucleic acid variant in another ficolin gene.

Although different reagents suitable for detecting the presence of at least one nucleic acid variant in a ficolin gene, such as the FCN1 gene can be envisaged, in a particular embodiment they will include an oligonucleotide probe suitable for detection of a target ficolin polynucleic acid and/or an oligonucleotide pair suitable for amplification of a target ficolin polynucleic acid. Specific embodiments of the kits of the present invention comprise an oligonucleotide probe suitable for detection of a sequence within the FCN1 gene and/or an oligonucleotide pair suitable for amplification of a sequence within a FCN1 polynucleic acid.

Similarly, the one or more reagents for the detection of a HLA-DRB1 shared epitope encoding allele(s), or a nucleic acid variant in the PTPN22 gene typically will include an oligonucleotide probe suitable for detection of and/or an oligonucleotide pair suitable for amplification of a HLA-DRB1 shared epitope encoding allele, or a target PTPN22 polynucleic acid respectively.

Oligonucleotides for use in the kits or methods of the present invention typically are isolated nucleic acid molecules comprising at least 8 nucleotides and specifically hybridizing with a target nucleic acid sequence, e.g. the wild type or variant sequence of the FCN1 gene including position -1981 and/or position 7919, or the complementary thereof.

More particularly, the oligonucleotide comprises at least 9, 10, 11, 12, 13, 14 or 15 nucleotides and up to 40, 30, 25, 24, 23, 22, 21, or 20 nucleotides. The oligonucleotides can be used as a primer or probe. In general such primers or probes will comprise nucleotide sequences entirely complementary to the corresponding target sequence, e.g. a wild type or variant locus in the target gene. Examples of such primers and probes for the FCN1 gene are given in Tables 1 and 2. Of course, specific length and sequence of the probes and primers will depend on the complexity of the required nucleic acid target, as well as on the reaction conditions such as temperature and ionic strength.

**Table 1. Examples of primers suitable for amplifying parts of the FCN1 gene**

| **Gene** | **Region** | **Forward primer** | **Reverse primer** |
|---|---|---|---|
| FCN1 | Promoter | agtggtgatgacaagcatcc (SEQ ID NO 12) | agacatctaagtgtcccagg (SEQ ID NO 13) |
| | exon 9 | gctcagaggcaggatgtggt (SEQ ID NO 14) | tggaggggtcctggcccgtc (SEQ ID NO 15) |

**Table 2. Examples of probes hybridizing to specific sequences in the FCN1 gene**

| **Gene** | **Position SNP** | **Nt** | **Wt probe** | **Mutant probe** |
|---|---|---|---|---|
| FCN1 | Promoter, g.-1981 | G/A | cacaatcaacgataaccagg (SEQ ID NO: 16) or cctggttatcgttgattgtg (SEQ ID NO 17) | cacaatcaatgataaccagg (SEQ ID NO 18) or cctggttatcattgattgtg (SEQ ID NO: 19) |
| | exon 9, g.7919 | A/G | accaggctccttggaacttc (SEQ ID NO: 20) or gaagttccaaggagcctggt (SEQ ID NO: 21) | accaggctccctggaacttc (SEQ ID NO: 22) or gaagttccagggagcctggt (SEQ ID NO: 23) |

An oligonucleotide primer (or primer pair) designed to specifically recognize and amplify either a wild type or variant allele at a locus is referred to as an allele specific primer (or primer pair). The same applies for an allele specific probe, i.e. an oligonucleotide probe that specifically hybridizes to either a wild type or variant allele.

For the detection of specific alleles, or for the detection of specific nucleic acid variants, the hybridization conditions are to be stringent as known in the art. "Stringent" refers to conditions under which a nucleotide sequence will no longer bind to unrelated or non-specific sequences. For example, high temperature and lower salt increases stringency such that non-specific binding or binding with low melting temperature will be prevented or dissolved.

The primers or probes may carry one or more labels to facilitate detection. The nature of the label is not critical to the invention and may be fluorescent, chemiluminescent, enzymatic, radioactive, chemical or other, provided it doesn't interfere with correct hybridizing of the oligonucleotide.

Typically, the kits of the present invention, will comprise, depending on whether the detection of the occurrence of a nucleic acid variant in the FCN1 gene, a HLA-DRB1 shared epitope encoding allele, a nucleic acid variant in the FCN2 gene and/or a nucleic acid variant in the PTPN22 gene is envisaged, one or more oligonucleotide primers or probes specific for the one or more allele(s) containing the nucleic acid variant(s) or encoding the shared epitope(s). However, in another embodiment, it is envisaged that the kits for detection according to the methods of the present invention comprise one or more oligonucleotide primers or probes specific for the wild type allele, not containing the nucleic acid variant or encoding shared epitope, whereby an indication of the presence of a wild type allele is indicative of the absence of a nucleic acid variant. In a further embodiment, the kits of the present invention comprise both oligonucleotide primers (or probes) specific for the mutant and the wild type allele. The latter embodiment is particularly suited to determine the copy number of the mutant alleles.

Accordingly, in specific embodiments, the one or more allele specific primers or probes for detecting the nucleic acid variants will typically comprise at least part of the nucleic acid sequence as identified by SEQ ID Nos 1 (for FCN1), 2 (for FCN2), 3 (for FCN3), or the complementary thereof, wherein the part of the nucleic acid sequence is envisaged to potentially comprise one or more nucleic acid variants. More specifically, kits are envisaged which comprise combinations of primers or probes capable of hybridizing to and/or amplifying the region comprising
- position -1981 and/or 7919 of SEQ ID NO: 1;
- position -64 and/or 6424 of SEQ ID NO: 2;
- position 1858 of SEQ ID NO: 11;
wherein the designated positions either have the wild type nucleotides or nucleic acid variants thereof.

Where the kits of the present invention are to be used for the detection of the HLA SE encoding allele, the one or more allele specific primers or probes for detecting the nucleic acid variants will typically be able to hybridize to and/or amplify the region encoding amino acids 70-74 of a HLA-DRB1 encoding allele, or the complementary thereof. More particular, they will comprise at least part of the nucleic acid sequence as identified by SEQ ID Nos 5, 7 and 9, or the complementary thereof.
Accordingly, the present invention also provides diagnostic kits comprising:
- one or more allele specific primers or oligonucleotide probes for detecting the presence of at least one nucleic acid variant in the FCN1 gene at position -1981 and/or position 7919 of the genomic DNA sequence of the FCN1 gene; and
- one or more allele specific primers or oligonucleotide probes for detecting a HLA-DRB1 shared epitope encoding allele.

In an alternative embodiment, diagnostic kits are provided comprising:
- one or more allele specific primers or oligonucleotide probes for detecting the presence of at least one nucleic acid variant in the FCN1 gene at position -1981 and/or position 7919 of the genomic DNA sequence of the FCN1 gene; and
- one or more allele specific primers or oligonucleotide probes for detecting the absence of a SNP at position -64 of the genomic DNA sequence of the FCN2 gene and/or the absence of a SNP at position 6424 of the genomic DNA sequence of the FCN2 gene. Optionally, the kits further comprise one or more allele specific primers or oligonucleotide probes for detecting the presence of a SNP at position -4 of the genomic DNA sequence of the FCN2 gene and/or one or more allele specific primers or oligonucleotide probes for detecting the presence of a SNP at position 6359 of the genomic DNA sequence of the FCN2 gene.

In yet an alternative embodiment, diagnostic kits are provided comprising:
- one or more allele specific primers or oligonucleotide probes for detecting the presence of at least one nucleic acid variant in the FCN1 gene at position -1981 and/or position 7919 of the genomic DNA sequence of the FCN1 gene; and
- one or more allele specific primers or oligonucleotide probes for detecting a SNP at position 1858 of the cDNA sequence of the PTPN22 gene.

Further embodiments of the diagnostic kits of the present invention include combinations of allele specific primers or oligonucleotide probes for detecting the presence or absence of at least one nucleic acid variant in the FCN2 gene (as described above) in combination with one or more allele specific primers or oligonucleotide probes selected from the group consisting of:
- one or more allele specific primers or oligonucleotide probes for detecting a HLA-DRB1 shared epitope encoding allele.
- one or more allele specific primers or oligonucleotide probes for detecting a SNP at position 1858 of the cDNA sequence of the PTPN22 gene.

Where the kits of the invention envisage the combined detection of a nucleic acid variant in a ficolin gene and a biochemical marker, the one or more reagents for the detection of the presence of a biochemical marker is either an antigen or antibody. Suitable reagents for the detection of anti-citrullinated peptide antibodies include synthetic peptides or antigens recognized by ACPAs. Alternatively, the kits of the present invention can comprise antibodies that cross-react with ACPAs.

Thus, one embodiment of the invention provides a diagnostic kits comprising:
- one or more allele specific primers or oligonucleotide probes for detecting the presence of at least one nucleic acid variant in the FCN1 gene at position -1981 and/or position 7919 of the genomic DNA sequence of the FCN1 gene; and one or more additional reagents selected from the group consisting of:

- one or more antibodies, antigens or synthetic peptides for detecting anti-citrullinated peptide antibodies, and
- one or more antibodies, antigens or synthetic peptides for detecting the presence of rheumatoid factor.

Further embodiments of the diagnostic kits of the present invention include combinations of allele specific primers or oligonucleotide probes for detecting the presence of at least one nucleic acid variant in the FCN2 gene (as described above) in combination with one or more additional reagents selected from the group consisting of:
- one or more antibodies, antigens or synthetic peptides for detecting anti-citrullinated peptide antibodies, and
- one or more antibodies, antigens or synthetic peptides for detecting the presence of rheumatoid factor.

As indicated above, the presence of nucleic acid variants in a ficolin gene of a subject may also be detected at the protein level, i.e. based on the modified concentration, structure and functionality of the ficolin protein in the serum or plasma of said subject. Therefore, the present invention also encompasses kits for performing the methods of the invention wherein the nucleic acid variants in the ficolin genes are detected by their protein phenotype. Thus, another aspect of the invention relates to a kit based on the detection of a FCN1 protein in combination with another marker for rheumatoid arthritis. Illustrative embodiments of the diagnostic kits according to this embodiment comprise an antibody for detecting the presence of FCN1 protein, and one or more reagents for specifically detecting the presence of another marker for RA, selected from the group consisting of: a HLA-DRB1 shared epitope encoding allele, a nucleic acid variant in the FCN2 gene, a nucleic acid variant in the PTPN22 gene, the presence of RF and the presence of anti-citrullinated peptide antibodies.

As the FCN1 protein may not be present in serum, but likely occurs more cell-membrane bound, detecting the presence of FCN1 protein should be performed on a suitable sample, e.g. obtained from lungs, kidney or arterial tissues.

Apart from the one or more specific reagents for detecting the presence of markers for RA described above, the kits of the invention optionally contain a variety of other reagents, e.g. depending on the detection procedure. The following is a non-exhaustive list of reagents that may be part of the kit's contents, the person skilled in the art will understand that this merely is illustrative of the possibilities: an agent for denaturing nucleic acids, an enzyme capable of modifying a double stranded or single stranded nucleic acid molecule, a hybridization buffer, or components necessary for producing a hybridization buffer, a wash solution, or components necessary for producing a wash solution, a means for detecting partially or completely denatured polynucleic acids, a means for detecting hybrids formed in the preceding hybridization, a means for detecting enzymatic modifications of nucleic acids, a means for attaching an oligonucleotide to a known location on a solid support, a labelled antibody, and/or a means for attaching an antigen to a known location on a solid support.

In yet another aspect of the invention, methods are provided for treating a subject at risk of having or developing RA, which method comprises identifying the subject as being at risk of having or developing RA. Thus, methods are provided for treating a subject having an increased risk of having or developing rheumatoid arthritis, said method comprising:
- determining the presence of at least one nucleic acid variant in a ficolin gene, such as the FCN1 gene, in said subject; and
- determining the presence of an other marker for rheumatoid arthritis in that subject, said the other marker being selected from the group consisting of:
   a HLA-DRB1 shared epitope encoding allele, a nucleic acid variant in another ficolin gene, a nucleic acid variant in the PTPN22 gene, the presence of RF, or the presence of anti-citrullinated peptide antibodies;
wherein the combined presence (or, where appropriate, absence) of the at least one nucleic acid variant in a ficolin gene and one of the other markers identifies whether a subject is at risk of, or has rheumatoid arthritis; and
- treating said the identified subject with medicaments against rheumatoid arthritis.

Other arrangements of the methods and tools embodying the invention will be obvious for those skilled in the art.

It is to be understood that although preferred embodiments, specific constructions and configurations, as well as materials, have been discussed herein for the methods and tools according to the present invention, various changes or modifications in form and detail may be made without departing from the scope and spirit of this invention.

The present invention is illustrated by the following Examples, which should not be understood to limit the scope of the invention to the specific embodiments therein.

### EXAMPLES

### Statistical analysis

For the present examples, the statistical analysis of the data is based on the determination of odds ratios (OR) using standard procedures. An odds ratio is calculated by dividing the odds in the treated or exposed (case) group by the odds in the control group. The odds of an event are calculated as the number of events divided by the number of non-events. If the odds of an event are greater than one the event is more likely to happen than not (the odds of an event that is certain to happen are infinite); if the odds are less than one the chances are that the event won't happen (the odds of an impossible event are zero). In the present examples, the strength of association was reported as odds ratios (OR) (with 95% lower (LCL) and upper (UCL) confidence limit), indicating the factor by which the risk of developing a disorder or disease is increased (OR>1), or indicating the factor for a protective effect on the risk of developing a disorder or disease (OR<1).

The 95% confidence interval (95% Cl) is the range of numerical values in which we can be confident (to a computed probability, here 95%) that the population value being estimated will be found. Confidence intervals indicate the strength of evidence; where confidence intervals are wide, they indicate less precise estimates of effect. The larger the trial's sample size, the larger the number of outcome events and the greater becomes the confidence that the true relative risk reduction is close to the value stated. Thus the confidence intervals get narrower and "precision" is increased. To confidently accept a calculated OR as reliable, important or clinically significant, the lower boundary of the confidence interval, or lower confidence limit, should be >1 if the OR>1, or the upper boundary of the confidence interval should be <1 if the OR<1.

### Example 1. Detection of nucleic acid variants in ficolin genes from patients with longstanding rheumatoid arthritis (RA) and from control subjects

### Patient samples

In the study, blood samples were taken from 164 longstanding RA patients (disease duration over 4 years, median 9 years).

The control group (C) consisted of 205 healthy individuals. From each individual from which a blood sample was taken, informed consent to participate in the study is available.

### Detection of nucleic acid polymorphisms

To determine the presence or absence of nucleic acid variants in the FCN1 and FCN2 gene, the relevant promoter and coding sequence regions of the genes under study were amplified using biotinylated oligonucleotides. The polymorphisms were detected by use of a reverse hybridization method (Dot Probe Assay) with probes designed to recognize the polymorphisms as given in Table 3. After stringent washing at 56°C, hybridized probes were incubated with a streptavidine-alkaline phosphatase conjugate. The presence of a hybridized probe was revealed using NBIT/BCIP colour development. Details on the reverse hybridization are described in Stuyver et al. (1996, J. Clin. Microbiol. 34:2259-2266), Stuyver et al. (1997, Antimicrob. Agents Chemoter. 41:284-291) and Van Geyt et al. (1998, in: Therapies of viral Hepatitis. International Medical Press, London, UK pp. 139-145).

**Table 3: identification of the ficolin nucleic acid variants studied in the present examples.**

| Gene | Region | SNP | Position gDNA | protein |
|---|---|---|---|---|
| FCN1 | Promoter | rs2989727 | -1981G>A | na* |
| | exon 9 | rs1071583 | 7919A>G | Q275Q |
| FCN2 | promoter | rs7865453 | -64A>C | na* |
| | promoter | rs17514136 | -4A>G | na* |
| | exon 8 | rs17549193 | 6359C>T | T236M |
| | exon 8 | rs7851696 | 6424G>T | A258S |

| | | | | |
|---|---|---|---|---|
| *na: not applicable | | | | |

### Statistical analysis

194 patients with Rheumatoid arthritis and 205 C-diagnosed subjects were genotyped for 2 SNPs in both the FCN1 gene and the FCN2 gene.

The strength of association was reported as odds ratios (OR), indicating the factor by which the risk of having RA is increased.

The 95% confidence interval (95% CI) is the interval computed from the sample data which, were the study repeated multiple times, would contain the true effect 95% of the time.

### Frequencies and odds ratios of FCN genotypes in patients with Rheumatoid arthritis vs healthy controls

Using the above-described detection methods, it was found that:
1. The presence of the A allele for the FCN1-rs2989727 and the G allele of the FCN1-rs1071583 polymorphism resulted in a higher risk of having or developing RA [OR 1,45 (95% CI 1,09-1,94) and OR 1,51 (1,13-2,02) respectively].
2. The presence of the C allele for the FCN2-rs7865453 polymorphism and the T allele for the FCN2-rs7851696 resulted in a lower risk of having or developing RA; these nucleic acid variations have a protective effect [OR 0.56 (95% CI 0.36-0.86) and OR 0.55 (95% CI 0.36-0.84) respectively]. Alternatively, it can be said that the absence of a SNP at these positions is associated with a higher risk of having or developing RA.
3. The presence of the G allele for the FCN2-rs17514136 and the T allele of the FCN2-rs17549193 polymorphism resulted in a higher risk of having or developing RA [OR 1,44 (95% CI 1,05-1,98) and OR 1,48 (1,08-2,03) respectively].
4. When comparing the ORs of the WT/Mut vs. WT/WT genotype with those of the Mut/Mut vs. WT/WT genotype, it is clear that there is a dosage effect for these alleles, i.e. an individual having two copies of the mutant allele has an increased risk of having or developing RA (or in the case of rs7865453 and rs7851696: having two copies of the wild type allele).

The results are provided in Table 4

**Table 4: genotypes, allele frequencies and Odds Ratios for SNPs in the FCN1 and FCN 2 gene in patients with longstanding RA**

| **SNP** | **n** | **FCN1 (Prom) rs2989727 WT: G-allele Mut: A-allele** | **FCN1 (Ex9)QQ rs1071583 WT: A-allele Mut: G-allele** | **FCN2 (Prom) rs7865453 WT: A-allele Mut: C-allele** | **FCN2 (Prom) rs17514136 WT: A-allele Mut: G-allele** | **FCN2 (Ex8)TM rs17549193 WT: C-allele Mut: T-allele** | **FCN2 (Ex8)AS rs7851696 WT: G-allele Mut: T-allele** |
|---|---|---|---|---|---|---|---|
| Controls | 205 | GG:38 | AA: 36 | AA:146 | AA: 123 | CC: 122 | GG: 143 |
| | | GA: 96 | AG: 96 | AC:56 | AG: 72 | CT:73 | GT:58 |
| | | AA:71 | GG:73 | CC:3 | GG:10 | TT:10 | TT: 4 |
| | | G allele: 172 | A allele: 168 | A allele: 348 | A allele: 318 | C allele: 317 | G allele: 344 |
| | | A allele: 238 | G allele: 242 | C allele: 62 | G allele: 92 | T allele: 93 | T allele: 66 |
| RA | 194 | GG: 18 | AA: 18 | AA: 160 | AA: 97 | CC: 95 | GG: 158 |
| | | GA:93 | AG:86 | AC: 33 | AG:80 | CT: 82 | GT: 37 |
| | | AA: 83 | GG: 90 | CC:1 | GG:17 | TT:18 | TT:0 |
| | | G allele: 129 | A allele: 122 | A allele: 353 | A allele: 274 | C allele: 272 | G allele: 353 |
| | | A allele: 259 | G allele: 266 | C allele: 35 | G allele: 114 | T allele: 118 | T allele: 37 |
| OR (95% Cl) | | | | | | | |
| WT/Mut vs WT/WT | | 2.05 (1.09-3.84) | 1.79 (0.95-3.39) | 0.54 (0.33-0.87) | 1.41 (0.93-2.13) | 1.44 (0.95-2.18) | 0.58 (0.36-0.92) |
| Mut/Mut vs WT/WT (WT/Mut or Mut/Mut) vs | | 2.47 (1.30-4.70) | 2.47 (1.29-4.70) | 0.30 (0.03-2.96) | 2.16 (0.94-4.92) | 2.31 (1.02-5.24) | / |
| WT/WT (WT/WT or WT/Mut) vs | | 2.22 (1.22-4.05) | 2.08 (1.14-3.81) | 0.53 (0.33-0.85) | 1.50 (1.01-2.23) | 1.55 (1.04-2.30) | 0.54 (0.34-0.86) |
| Mut/Mut | | 0.71 (0.47-1.06) | 0.64 (0.43-0.96) | 2.87 (0.30-27.8) | 0.53 (0.24-1.20) | 0.50 (0.23-1.12) | / |
| | | | | | | | |
| Mut allele vs WT allele | | 1.45 (1.09-1.94) | 1.51 (1.13-2.02) | 0.56 (0.36-0.86) | 1.44 (1.05-1.98) | 1.48 (1.08-2.03) | 0.55 (0.36-0.84) |

### Example 2. Detection of nucleic acid variants in ficolin genes from patients newly diagnosed with rheumatoid arthritis (RA) and from control subjects

### Patient samples

A consecutive cohort of 528 patients presented themselves with a new diagnostic problem with RA in the differential diagnosis. After 1 year, diagnoses established 138 RA patients and 390 non-RA patients (control population).

The non-RA control population (n=629) had the following diagnoses: osteoarthritis, abarticular rheumatic symptoms, spondyloarthropathy, connective tissue diseases including polymyalgia rheumatica, psoriatic arthritis, crystal induced arthritis, fibromyalgia and aspecific arthralgias, other and undifferentiated diseases including infections, malignancies, and neurological disorders. It can be estimated that in about 50% of this control population, pathways of innate immunity may be involved.

From each individual from which a blood sample was taken, informed consent to participate in the study is available.

### Detection of nucleic acid polymorphisms

To determine the presence or absence of nucleic acid variants in the FCN1 and FCN2 gene, the relevant promoter and coding sequence regions of the genes under study were amplified using biotinylated oligonucleotides. The polymorphisms were detected by use of a reverse hybridization method (Dot Probe Assay) with probes designed to recognize the polymorphisms as given in Table 3. After stringent washing at 56°C, hybridized probes were incubated with a streptavidine-alkaline phosphatase conjugate. The presence of a hybridized probe was revealed using NBIT/BCIP colour development. Details on the reverse hybridization are described in Stuyver et al. (1996, J. Clin. Microbiol. 34:2259-2266), Stuyver et al. (1997, Antimicrob. Agents Chemoter. 41:284-291) and Van Geyt et al. (1998, in: Therapies of viral Hepatitis. International Medical Press, London, UK pp. 139-145).

### Statistical analysis

138 patients with Rheumatoid arthritis and 390 C-diagnosed subjects were genotyped for 2 SNPs in both the FCN1 gene and the FCN2 gene.

The strength of association was reported as odds ratios (OR), indicating the factor by which the risk of developing RA is increased.

The 95% confidence interval (95% Cl) is the interval computed from the sample data which, were the study repeated multiple times, would contain the true effect 95% of the time.

### Frequencies and odds ratios of FCN genotypes in patients with Rheumatoid arthritis vs non-RA patients

Based on the screening of the above-described patient groups, it was found that:
1. The presence of the A allele for the FCN1-rs2989727 polymorphism resulted in a higher risk of having or developing RA [OR 1,34 (95% CI 1,00-1,79)]
2. The presence of at least one G allele for the FCN1-rs1071583 polymorphism significantly increases the risk of developing or having RA when compared to subjects homozygous for the A allele [OR 1,91 (1,00-3,68)].
3. The presence of the C allele for the FCN2-rs7865453 polymorphism and the T allele for the FCN2-rs7851696 seem to protect against RA, but the result is not statistically significant [OR 0.86 (0.54-1.37) and OR 0.95 (0.61-1.47) respectively]. However, this lack of statistical significance may for a large part be due to the low numbers of C or T alleles respectively in the RA group when compared to the control group. Indeed, higher numbers are needed to reach a definite conclusion. To have a better indication, the pooled Odds Ratio was calculated, which relates to the association of a mutant allele with RA in the combined populations of Example 1 and 2 (relative to the combined control populations of Example 1 and 2). This leads to an OR of 0,72 (CI 0,53-0,99) for the C allele of the FCN2-rs7865453 polymorphism, and an OR of 0,75 (0,55-1,01) for the T allele of FCN2-rs7851696. These data, together with the strong association found in example 1, indicate that these SNPs in FCN2 indeed have protective effect, or that the absence of these SNPs is indicative of a risk of having or developing RA.

The results are summarized in Table 5.

**Table 5: Genotypes, allele frequencies and Odds Ratios for SNPs in the FCN1 and FCN 2 gene in patients with RA compared with non-RA patients**

| **SNP** | **n** | **FCN1 (Prom) rs2989727 WT: G-allele Mut: A-allele** | **FCN1 (Ex9)QQ rs1071583 WT: A-allele Mut: G-allele** | **FCN2 (Prom) rs7865453 WT: A-allele Mut: C-allele** | **FCN2 (Ex8)AS rs7851696 WT: G-allele Mut: T-allele** |
|---|---|---|---|---|---|
| Controls | 390 | GG: 67 | AA: 60 | AA: 312 | GG: 306 |
| | | GA:173 | AG:175 | AC: 72 | GT: 79 |
| | | AA:150 | GG: 154 | CC: 6 | TT:5 |
| | | G allele: 307 | A allele: 295 | A allele: 696 | G allele: 691 |
| | | A allele: 473 | G allele: 483 | C allele: 84 | T allele: 89 |
| | | | | | |
| RA | 138 | GG:11 | AA: 12 | AA:112 | GG:110 |
| | | GA: 68 | AG: 66 | AC: 26 | TG: 26 |
| | | AA: 59 | GG: 60 | CC: 0 | TT:2 |
| | | G allele: 90 | A allele: 90 | A allele: 250 | G allele: 246 |
| | | A allele: 186 | G allele: 186 | C allele: 26 | T allele: 30 |
| | | | | | |
| OR (95% Cl) | | | | | |
| WT/Mut vs WT/WT | | 2.39 (1.19-4.80) | 1.89 (0.95-3.73) | 1.01 (0.61-1.65) | 0.92 (0.56-1.50) |
| Mut/Mut vs WT/WT | | 2.40 (1.18-4.85) | 1.95 (0.98-3.88) | / | 1.11 (0.21-5.82) |
| (WT/Mut or Mut/Mut) vs WT/WT | | 2.39 (1.23-4.68) | 1.91 (1.00-3.68) | 0.93 (0.57-1.52) | 0.93 (0.57-1.50) |
| (WT/WT or WT/Mut) vs Mut/Mut | | 0.84 (0.56-1.24) | 0.85 (0.58-1.26) | / | 0.88 (0.17-4.61) |
| | | | | | |
| Mut allele vs WT allele | | 1.34 (1.00-1.79) | 1.26 (0.94-1.69) | 0.86 (0.54-1.37) | 0.95 (0.61-1.47) |

In addition to the importance of the ficolin SNPs in the diagnosis of RA (versus healthy controls), the above data also show the value of said SNPs in the differential diagnosis of RA versus other diseases such as osteoarthritis, abarticular rheumatic symptoms, spondyloarthropathy, connective tissue diseases including polymyalgia rheumatica, psoriatic arthritis, crystal induced arthritis, fibromyalgia and aspecific arthralgias, being other and undifferentiated diseases including infections, malignancies, and neurological disorders.

### Example 3. Evidence of linkage between two SNPs in the FCN1 gene

As the haplotype data were not available for both patient and control groups, the distribution of genotypes was assessed to see whether linkage between the two SNPs in the FCN 1 gene was likely. The results are shown in Table 6 for the patient and control group from example 1 and in Table 7 for the patient and control group from example 2. Both populations show a high agreement in the copy number of SNPs present, indicating that linkage between both SNPs is probable.

**Table 6. Assessment of gene linkage in patient cohort 1**

| | | | copy number rs1071583 | | | Total |
|---|---|---|---|---|---|---|
| | | | 0 | 1 | 2 | |
| control | copy | 0 | 35 | 2 | 1 | 38 |
| | number | 1 | 0 | 91 | 5 | 96 |
| | rs2989727 | 2 | 1 | 3 | 67 | 71 |
| Total | | | 36 | 96 | 73 | 205 |
| RA | copy | 0 | 17 | 1 | 0 | 18 |
| | number | 1 | 0 | 85 | 8 | 93 |
| | rs2989727 | 2 | 1 | 0 | 82 | 83 |
| Total | | | 18 | 86 | 90 | 194 |

**Table 7. Assessment of gene linkage in patient cohort 2**

| | | | copy number rs1071583 | | | Total |
|---|---|---|---|---|---|---|
| | | | 0 | 1 | 2 | |
| control | copy | 0 | 59 | 7 | 1 | 67 |
| | number | 1 | 1 | 167 | 4 | 172 |
| | rs2989727 | 2 | 0 | 1 | 149 | 150 |
| Total | | | 60 | 175 | 154 | 389 |
| RA | copy | 0 | 11 | 0 | 0 | 11 |
| | number | 1 | 1 | 66 | 1 | 68 |
| | rs2989727 | 2 | 0 | 0 | 59 | 59 |
| Total | | | 12 | 66 | 60 | 138 |

### Example 4. The combined presence of a SNP in the FCN1 gene and a HLA-SE encoding allele is highly indicative of the risk of developing or having RA.

### Patient samples

The patient and control group are the same as described in Example 1.

### Detection of nucleic acid polymorphisms and the HLA SE encoding allele

Detection of the FCN1 nucleic acid polymorphisms was already described in Example 1. Presence of a HLA-SE encoding allele was determined using the INNO-LiPA^{™} HLA-DRB1 Plus kit (Innogenetics NV, Ghent, Belgium) according to manufacturer's instructions.

The first four nucleotide sequences shown in Table 8 are classified as encoding a shared epitope (QRRAA, QKRAA or RRRAA) and form part of a HLA-DRB1 allele. Table 9 shows the possible genotypes of these alleles and the associated risk of RA of these combinations.

**Table 8: Exemplary sequences encoding the shared epitope and the associated risk of RA.**

| **Nucleotide sequence encoding AA 70- 74 of HLA-DRB1** | **AA sequence** | **SE** | **Risk of RA** |
|---|---|---|---|
| CAG AGG CGG GCC GCN (SEQ ID NO: 24) | QRRAA (SEQ ID NO: 4) | A | Risk |
| CAG AAG CGG GCC GCN (SEQ ID NO: 25) | QKRAA (SEQ ID NO: 6) | B | High risk |
| CGG AGG CGG GCC GCN (SEQ ID NO: 26) | RRRAA (SEQ ID NO: 8) | C | Risk |
| CGG AGG CGT GCC GCN (SEQ ID NO: 27) | RRRAA (SEQ ID NO: 8) | C | Risk |
| GAC GAG CGG GCC GCN (SEQ ID NO: 28) | DERAA (SEQ ID NO: 10) | D | Protective |
| All sequences not encoding a shared epitope | | E | No risk |

| | | | |
|---|---|---|---|
| (N = any nucleic acid) | | | |

**Table 9. Possible genotypes for SE encoding alleles and the associated risk of RA.**

| **SE** | **Associated risk** | **combined SE** |
|---|---|---|
| **SE=0** | | |
| | No risk | D/D D/E E/E |

| **SE = 1 (single load)** | | |
|---|---|---|
| | Low risk | A/D A/E C/D C/E |
| | Medium risk | B/D B/E |

| **SE = 2 (double load)** | | |
|---|---|---|
| | High risk | A/A A/C C/C |
| | Very high risk | A/B B/C |
| | Extreme high risk | B/B |

| **SE=-1** | | |
|---|---|---|
| | mixed SE result | |

The positive predictive value (PPV) for the HLA-SE encoding alleles - meaning the likelihood for a subject to effectively develop or have RA if a HLA-SE encoding allele is present - is 18%, 30% and 56% for the SE genotype 0, 1 and 2 of HLA-DRB1, respectively. Although it is clear that there is a strong association between HLA-SE and risk of RA, it could be demonstrated that, when assessing both the presence of a polymorphism in the FCN1 gene and the presence of a HLA-SE encoding allele, the specificity of the assay is tremendously increased.

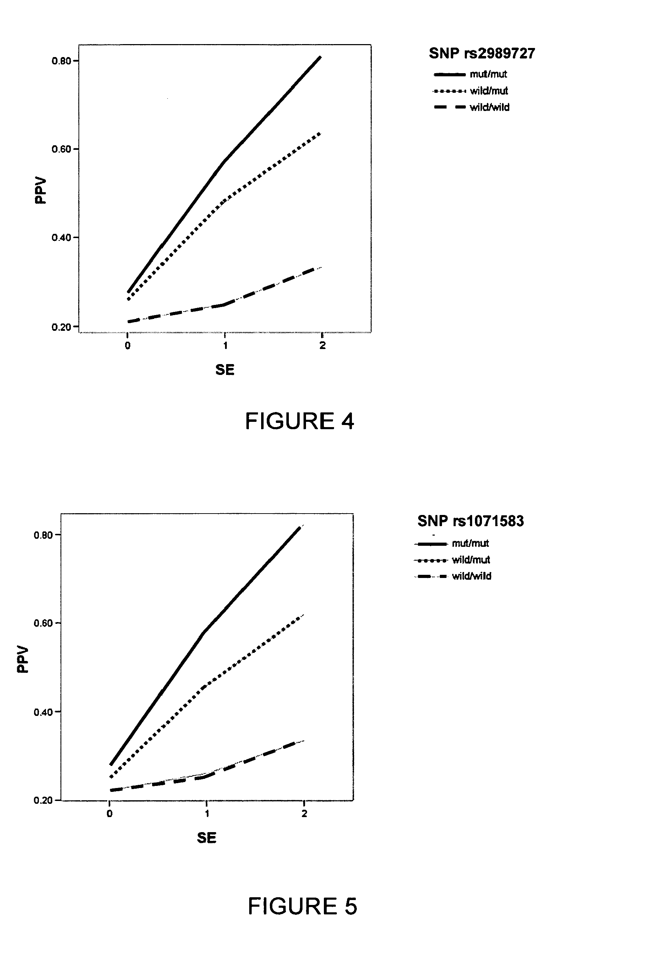

As is shown in Figure 4, the positive predictive value can be further stratified. If no A allele is present at position -1981 of the genomic DNA sequence of the FCN1 gene, the PPV remains below 35%, even when 2 HLA-SE encoding alleles are present. Similarly, the presence of one or two A alleles does not significantly increase the risk of RA if no HLA-SE encoding alleles are present. However, if both at least one A allele for FCN1 SNP rs2989727 and at least one HLA-SE encoding allele are present, the PPV is always above 45%. Remarkably, it reaches 80% when both the two A alleles comprising the nucleic acid variant at position -1981 of the FCN1 gene and two HLA-SE encoding alleles are present. This PPV is considerably more reliable than when only the occurrence of two HLA-SE encoding alleles is taken into account (PPV of 56%, see above).

The same was observed for the A/G polymorphism at position 7919 of the genomic DNA sequence of the FCN1 gene: the presence of at least one G allele for SNP rs1071583 and at least one HLA-SE encoding allele significantly raises PPV, and 80% PPV is reached when both the FCN1 marker is present in two copies and there are two HLA-SE encoding alleles. This is shown in Figure 5.

### Example 5. The combined presence of a SNP in the FCN1 gene and anti-citrullinated peptide antibodies is indicative of the risk of developing or having RA.

### Patient samples

The patient and control group are the same as described in Example 2.

### Detection of nucleic acid polymorphisms and anti-citrullinated peptide antibodies

Detection of the FCN1 nucleic acid polymorphisms was performed as described in Example 1. Anti-pepA antibodies were detected using the lmmunoscan RA anti-CCP Test Kit (Euro-Diagnostica B.V., Arhnem, The Netherlands). The assay was performed according to the manufacturer's instructions. The strips were scanned to allow quantification of the visual intensity. Scan values were correlated to predictive probability (PP) for RA using a logistic regression approach.

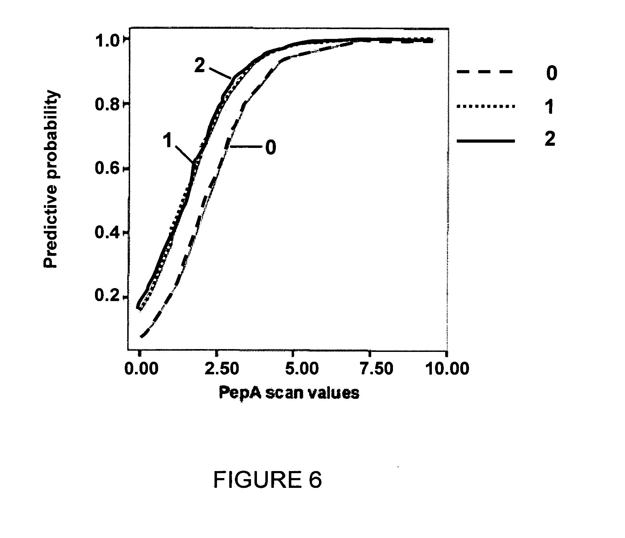

As is shown in Figure 6, the presence of anti-citrullinated peptide antibodies (ACPAs) is in itself a marker for the risk of RA. For example, a pepA scan value of 2,50 corresponds to a PP for RA of over 50% in case no FCN1 rs2989727 variant is present. However, when at least one A allele for FCN1 SNP rs2989727 is also present, the pepA scan value of 2,50 corresponds to a PPV of about 80%, thus significantly increasing specificity.

## Claims

1. A method of identifying a subject having or at risk of having or developing rheumatoid arthritis, comprising:
- determining the presence of at least one nucleic acid variant in the FCN1 gene in said subject; and
- determining the presence of an other marker for rheumatoid arthritis in said subject, said marker being selected from the group consisting of: a HLA-DRB1 shared epitope encoding allele, the occurrence of a nucleic acid variant in the FCN2 gene, a nucleic acid variant in the PTPN22 gene, the presence of rheumatoid factor and the presence of anti-citrullinated peptide antibodies;
wherein the combined presence of the at least one nucleic acid variant in the FCN1 gene and the other marker is indicative of having or a risk of having or developing rheumatoid arthritis.

2. The method of claim 1, wherein one or more of said at least one nucleic acid variant in the FCN1 gene is a single nucleotide polymorphism (SNP).

3. The method of claim 2, wherein the one or more SNPs are located at position -1981 and/or position 7919 of the genomic DNA sequence of the FCN1 gene.

4. The method of claim 3, wherein the SNP at position -1981 is a G/A polymorphism (dbSNP number rs2989727) and the SNP at position 7919 is an A/G polymorphism (dbSNP number rs1071583).

5. The method of any one of claims 1 to 4, which comprises determining whether said at least one nucleic acid variant in the FCN1 gene is present in 0, 1 or 2 copies, wherein the heterozygous or homozygous presence of the at least one nucleic acid variant in combination with said other marker is indicative of having or a risk of having or developing rheumatoid arthritis.

6. The method of any one of claims 1 to 5, which comprises determining whether said at least one nucleic acid variant in the FCN1 gene is present in 0, 1 or 2 copies, wherein homozygous presence is indicative of an increased risk of having or developing rheumatoid arthritis.

7. The method of any one of claims 1 to 6, wherein the other marker for rheumatoid arthritis is the presence of a HLA-DRB1 shared epitope encoding allele.

8. The method of any one of claims 1 to 7, which comprises determining whether 0, 1 or 2 HLA-DRB1 shared epitope encoding alleles are present in said subject, wherein the presence of 1 or 2 HLA-DRB1 shared epitope encoding alleles in combination with at least one nucleic acid variant in the FCN1 gene is indicative of having or a risk of having or developing rheumatoid arthritis.

9. The method of claim 8, which comprises determining whether 0, 1 or 2 HLA-DRB1 shared epitope encoding alleles are present in said subject,
wherein the presence of 2 HLA-DRB1 shared epitope encoding alleles is indicative of an increased risk of having or developing rheumatoid arthritis.

10. The method of any one of claims 1 to 6, wherein the other marker for rheumatoid arthritis is selected from the group consisting of: the absence of a SNP at position -64 of the genomic DNA sequence of the FCN2 gene, the absence of a SNP at position 6424 of the genomic DNA sequence of the FCN2 gene, and the presence of a SNP at position 1858 of the PTPN22 gene.

11. The method of claim 10, wherein the SNP at position -64 of the FCN2 gene is an A/C polymorphism (dbSNP number rs7865453), the SNP at position 6424 of the FCN2 gene is a G/T polymorphism (dbSNP number rs7851696) and the SNP at position 1858 of the PTPN22 gene is a C/T polymorphism (dbSNP number rs2476601).

12. The method of claim 10 or 11, which comprises determining whether said other marker is present in 0, 1 or 2 copies in said subject wherein the heterozygous or homozygous presence of said other marker in combination with at least one nucleic acid variant in the FCN1 gene is indicative of having or a risk of having or developing rheumatoid arthritis

13. The method of claim 10 or 11, which comprises determining whether said other marker for rheumatoid arthritis is present in 0, 1 or 2 copies, wherein homozygous presence of said other marker is indicative of an increased risk of having or developing rheumatoid arthritis.

14. The method of any one of claims 1 to 6, wherein said other marker for rheumatoid arthritis is the presence of rheumatoid factor or anti-citrullinated peptide antibodies.

15. The method of any one of claims 1 to 13, wherein the step of detecting the presence of an other marker for rheumatoid arthritis is performed by DNA or RNA hybridization, sequencing, PCR, primer extension, multiplex ligation-dependent probe amplification (MLPA), oligonucleotide ligation assay (OLA) and/or restriction site analysis.

16. The method of claim 14, wherein said other marker for rheumatoid arthritis is detected via ELISA, immunoblotting, immunofluorescence, an agglutination test, nephelometry or an immuno-assay.

17. The method of any one of claims 1 to 16, wherein the step of determining the presence of at least one nucleic acid variant in the FCN1 gene is performed by DNA or RNA hybridization, sequencing, PCR, primer extension, multiplex ligation-dependent probe amplification (MLPA), oligonucleotide ligation assay (OLA) and/or restriction site analysis.

18. The method of any one of claims 1 to 17, wherein the combined presence of the at least one nucleic acid variant in the FCN1 gene and the other marker for rheumatoid arthritis is indicative of having or the risk of having or developing an increased severity of rheumatoid arthritis.

19. The method of any one of claims 1 to 17, wherein the combined presence of the at least one nucleic acid variant in the FCN1 gene and the other marker for rheumatoid arthritis is indicative of having or the risk of having or developing, a modified response to rheumatoid arthritis therapy.

20. The method of any one of claims 1 to 19, wherein the step of determining of the presence of the at least one nucleic acid variant in the FCN1 gene and/or the presence of an other marker for rheumatoid arthritis is performed in vitro in a biological sample obtained from said subject.

21. A kit for the diagnosis of having or a risk of having or developing rheumatoid arthritis comprising: one or more reagents for detecting the presence of at least one nucleic acid variant in the FCN1 gene, and one or more reagents for specifically detecting the presence of an other marker for rheumatoid arthritis, said marker selected from the group consisting of: a HLA-DRB1 shared epitope encoding allele, the occurrence of a nucleic acid variant in the FCN2 gene, a nucleic acid variant in the PTPN22 gene, rheumatoid factor, and anti-citrullinated peptide antibodies.

22. A diagnostic kit comprising :
- one or more allele specific primers or oligonucleotide probes for detecting the presence of at least one nucleic acid variant in the FCN1 gene at position -1981 and/or position 7919 of the genomic DNA sequence of the FCN1 gene; and
- one or more allele specific primers or oligonucleotide probes for detecting the presence of a HLA-DRB1 shared epitope encoding allele.

23. A diagnostic kit comprising:
- one or more allele specific primers or oligonucleotide probes for detecting the presence of at least one nucleic acid variant in the FCN1 gene at position -1981 and/or position 7919 of the genomic DNA sequence of the FCN1 gene; and
- one or more allele specific primers or oligonucleotide probes for detecting a SNP at position -64 of the genomic DNA sequence of the FCN2 gene, a SNP at position 6424 of the genomic DNA sequence of the FCN2 gene, a SNP at position 1858 of the PTPN22 gene.

24. The kit of claim 23, wherein the SNP at position -64 of the FCN2 gene is a A/C polymorphism (dbSNP number rs7865453), the SNP at position 6424 of the FCN2 gene is a G/T polymorphism (dbSNP number rs7851696) and the SNP at position 1858 of the PTPN22 gene is a C/T polymorphism (dbSNP number rs2476601).

25. A diagnostic kit comprising:
- one or more allele specific primers or oligonucleotide probes for detecting the presence of at least one nucleic acid variant in the FCN1 gene at position -1981 and/or position 7919 of the genomic DNA sequence of the FCN1 gene; and
- one or more synthetic peptides, antigens or antibodies for detecting the presence of anti-citrullinated peptide antibodies or rheumatoid factor.

26. The kit of any one of claims 21 to 25, wherein one or more of said at least one nucleic acid variant in the FCN1 gene is a SNP.

27. The kit of claim 26, wherein the one or more SNPs are located at position - 1981 and/or position 7919 of the genomic DNA sequence of the FCN1 gene.

28. The kit of claim 27, wherein the SNP at position -1981 is a G/A polymorphism (dbSNP number rs2989727) and the SNP at position 7919 is an A/G polymorphism (dbSNP number rs1071583).

29. A kit for the diagnosis of having or a risk of having or developing rheumatoid arthritis, comprising: an antibody for detecting the presence of FCN1 protein, and at least one reagent for specifically detecting the presence of an other marker for rheumatoid arthritis, said marker selected from the group consisting of: the presence of a HLA-DRB1 shared epitope encoding allele, the occurrence of a nucleic acid variant in the FCN2 gene, the presence of a nucleic acid variant in the PTPN22 gene, rheumatoid factor , and anti-citrullinated peptide antibodies.

30. A diagnostic kit comprising:
- at least one antibody for detecting the presence of FCN1 protein; and
- one or more allele specific primers or oligonucleotide probes for detecting a HLA-DRB1 shared epitope encoding allele.

31. A diagnostic kit comprising:
- at least one antibody for detecting the presence of FCN1 protein; and
- one or more allele specific primers or oligonucleotide probes for detecting a SNP at position -64 of the genomic DNA sequence of the FCN2 gene, a SNP at position 6424 of the genomic DNA sequence of the FCN2 gene, a SNP at position 1858 of the PTPN22 gene.

32. The kit of claim 31, wherein the SNP at position -64 of the FCN2 gene is an A/C polymorphism (dbSNP number rs7865453), the SNP at position 6424 of the FCN2 gene is a G/T polymorphism (dbSNP number rs7851696) and the SNP at position 1858 of the PTPN22 gene is a C/T polymorphism (dbSNP number rs2476601).

33. A diagnostic kit comprising:
- at least one antibody for detecting and/or quantifying the presence of FCN1 protein; and
- one or more synthetic peptides, antigens or antibodies for detecting anti-citrullinated peptide antibodies or rheumatoid factor.

34. A method of treating a subject having an increased risk of having or developing rheumatoid arthritis, said method comprising
- determining the presence of at least one nucleic acid variant in the FCN1 gene in said subject; and
- determining the presence of an other marker for rheumatoid arthritis in said subject, said marker being selected from the group consisting of: a HLA-DRB1 shared epitope encoding allele, a nucleic acid variant in the FCN2 gene, a nucleic acid variant in the PTPN22 gene, or the presence of anti-citrullinated peptide antibodies;
wherein the combined presence of the at least one nucleic acid variant in the FCN1 gene and the other marker identifies whether a subject is at risk of, or has rheumatoid arthritis
- treating said subject with medicaments against rheumatoid arthritis.

35. A method of identifying a subject at risk of having or developing a modified response to rheumatoid arthritis therapy, said method comprising:
- determining the presence of at least one nucleic acid variant in the FCN1 gene in said subject; and
- determining the presence of an other marker for rheumatoid arthritis in said subject, said marker being selected from the group consisting of: a HLA-DRB1 shared epitope encoding allele, a nucleic acid variant in the FCN2 gene, a nucleic acid variant in the PTPN22 gene, or the presence of anti-citrullinated peptide antibodies;
wherein the combined presence of the at least one nucleic acid variant in the FCN1 gene and the other marker identifies whether a subject is at risk of having or developing a modified response to rheumatoid arthritis therapy.


SEQUENCE LISTING

<110>  Innogenetics

<120>  COMBINATIONS OF MARKERS FOR INCREASED ACCURACY OF DIAGNOSIS OF
       RHEUMATOID ARTHRITIS

<130>  I3863-EP

<160>  29

<170>  PatentIn version 3.3

<210>  1
<211>  10900
<212>  DNA
<213>  Homo sapiens

<400>  1
tatggttttg ttctcttgcc tactgcactg gacagtgaca tgcaatacaa tgttgaccac      60

agtggtgatg acaagcatcc ttatttgttc tcagtctctg aggatggttt tccatttttc     120

ctgttaatta tgttgtaact tctgtatcca cagcttccaa agacctcttt ctatattctg     180

ttgtttctgt tggtccttat acacattgtc ttttattccc atgagcctgg ttatcgttga     240

ttgtgtttta acatttcatt tgaaaaaaat aaaacacttt gcagaaataa tgacctggga     300

tgatgtcatt ctccttcaga gaagattttt gtttctttca gcaaggagcc tgggggcatg     360

agcaataatt ccaggagttg agattccttt cctgggacac ttagatgtct caaaaccagt     420

ttgcagccct tgtgaatgtt ggttgttgct cactcatcct attgctaggg tgcagccctt     480

ctgaaagcca accccaaacc catacctttg gcaggccgta ggccccagtc tttgaccttc     540

agctctgcaa gatggtgaaa tcactgccca acttctcacc tctcttttgg agattggcaa     600

atccctgtgc aaaaagaggt ccccactact agtttcactt ctctaagatt tctgtggctc     660

ctgagagctt ggcaaagtaa tcctttacta catagctagc ttgacggtgc cttcgagcaa     720

atctcttttt aaaggttttg tctgtttgtc cttaatagga tgcccccaaa tacctaaact     780

gccattgcca ggaatcaaat gacttttgtt gatccaatgt taagatattt taacttgcca     840

gaatcttgca ttctttcata ctcttgggaa cattgacttg atatctgaag gtggtgttga     900

cttctcacct gaaataaacc tattgatgtt gcaaggaaga ggaggtcccc tcacacccag     960

ggggcagctg gcaaatggaa ttgaggggca cagacctgct ggctttctcc ctgttctgac    1020

ccaatcccag gtgagtgctg ctcctgaatt ggatcctctc caagaaagga ggccgcacca    1080

ctgacaccca gacctctccc gcctgggcca gtagctcagg gacactaagc ggtgtctgag    1140

gcaatgaagc ctcctgccgt tgtctctcaa caggctggag ccaacacaca caaacacaca    1200

cagtcgagag tcgttgtggt gcccagagag cctggacaca aggtcctgtg tctcctggat    1260

cccccacccc tgggccgccc ctccacttct ctcccacctg gggagccctt gagtttggaa    1320

acccagtcta cacacgctgg ctcagagaag ttgcaaacca caaaacaaca cctctccctg    1380

ctccttagtc ctgctaccat ctgactctcg ttagtgacta tttctatacg ttctaaaaat    1440

cccgcacaaa gtgtccacag cgtggcctgg ctaagcatga ttcacagcac gagatgcgga    1500

gtaggaacat ggcctcaggg atttggggga acaagaggca tccccacctt caggtgaaat 1560
gagcaatccc agtgatctac aaggcgttag acagaattga gtccaatgcc tggctcttac 1620
tagtggagtg acttcaattt ctccagctgt aactttcaaa taatttactc caccgaatgg 1680
ggctggtgca agatgatatg atggaaatag tatttgggac ataacaagtg ctcaacattt 1740
ttttcctctc catccatgat aagtgcctga tgacggacaa accctgaatt ttctgggaac 1800
agatgaggca aggaagcgtc ttcagcaaaa ccttccttat cccccaaggt taaggtcaag 1860
gtttccgagt attgcttggg gacagctggg gaatggaaag ggggtgtggg ggctgtgttc 1920
tccttaagcc acaagcaaag ccgatgcttt ccagcagagg agtctcagga gctctctgga 1980
ctgggagcag ggtgtctttg ctgggggaag agagcccaat ggggtgggca tcttgctgga 2040
gcctagtgga aggaaacatc ctttgagatg acctcatacc tcctgttggt gcaactaagc 2100
agcccatagg aagtgggtgt cccccaggga gttgagaaac tgtggcacaa ggcgagagct 2160
ggtttcctct gccctgttag agctggggga ctcttcagag tcaaaggcca gagagcatgg 2220
agctgagtgg agccaccatg gcccgggggc tcgctgtcct gctagtcttg ttcctgcata 2280
tcaagaacct gcctgcccag gctgcggaca catgtccagg tgagacagac ctgggttttc 2340
tggtccctaa aagctggtaa caaccccact cactgacact cgggtgtccc aggcaaaacc 2400
ttttatgcac atcttcctgt gaagatgccc aacagatctg ttttatcctc aggcaaggac 2460
atggaggctc agggcagctg aggcattgcc cagggtcatg gagggatagg ggagggatgt 2520
caccaggtct gtctgcctcg aaagcccagg atttgcccat tggagcttca taggtgggtc 2580
tggaatctgt cactgagaag gaagaaaata ctgttgactt tatcttctta cgggcttacc 2640
agggaggcac tctaagaccc tctaatacgg tggctgccac acggacttgg ggggtttgtt 2700
aaaaaggcag atttctggct ctcctccaag agagtcggaa tcagttgata gggacaaggg 2760
tttgaacggt ggtaaggtgt ttccctcact gattcaggag caggtggttc tgtgacgctc 2820
agcaagagac tgctcctaag gacggcctgg tgcccctggc tgataacata tttatgaagg 2880
aaacataaat agtaaattac agcctgctct agtggaagga ggactggaaa cacagaaagg 2940
aaatacccca aaaatggcag tgccatgtct ccggtgagag cactgctccg ttccatggtc 3000
tctaagagcg cccttagcag ggcgtgtaga ttttgtaatg aacaaaatga aaaatatttt 3060
ttcaaagaag taaacaactt tggtttggct gcagctgtgc cacacctggc tctgtgtcct 3120
tggaccccac actgcccctc tccagaggac ctagaaagcc ccttccagcc cagtaattct 3180
acaaccacat tcccaaggtc tgggagggaa aatgctttgt cagccccaat tccccaagtt 3240
tcttagaaat attgattgtc attagttatt gaaagtgccc gctgtttatt aagctctttc 3300
taaatgccat aaattacaat aagagattta aaatgcacat tctttaacca aatcctggcc 3360
gcaaccttgt tattatcccc gcttggcagc ccagggcaca aagctcagag gagttaagtt 3420
acttgctgca ggacacacag cactgaggag atggggctcc cctaggtcgg ggtggcctcc 3480
aaagccagag ctctgccccc catcacatct ccgcctcacc agtgcctgag gggtgaggac 3540

agaccaaggc cccagcagag cagaaaacct cacttcccag ccactgttgg gccaggcact 3600
gagtgggcac ctgtgtttct ctgcagaggt gaaggtggtg ggcctggagg gctctgacaa 3660
gctcaccatt ctccgaggct gcccggggct gcccggggcc ccagggccaa agggagaggc 3720
aggtgtcatt ggagagagag gtaggtgcgg gccgggtggg gaggccaggc atggcagggg 3780
cactggctct tgctctttat gaaactggat gcagagttgg gcaacacccc caccgtggtt 3840
cctagatcaa gagcctgggt caggcccctg ctagagccag gaacagagct gactcccagg 3900
gcccaacagg gttctgaaat ggagggaggg tctttcctaa ctatttcatg agctcacatt 3960
tggacatgct tctgtagctc tgtctcctag tccttgatcc aataccagct ttgctcagct 4020
actggcacga cttctaagct ctgtgtccct ccaccttctc acctgaaaat tgaggtgacc 4080
gctttcttct gcacctgcct catatgggaa aactgagact caaagaggtc acaggctttc 4140
ctacttgaca aaccagtaag gagtggggct gggacttgga ctggtctact tcagggcaca 4200
gagggcaccc gaatcctgag ccctgagctc tgcatgtgga tttgcaggga acatgggaga 4260
atgaacagca ggcacctgcc ttgggtggtt ttgggtcccc ctaagcatgg cataaggcag 4320
acgtgcacaa aaactcagaa agggcgctgc cttagggggt gagctcccca tgcctggacg 4380
cattcaagat gatgcctgcg gagcacttgg tggggatgtc ccagtgtaga tgggacaagc 4440
tcaaatgtcc ctatgacttg gactttcact gacgtgtcta ggcttccgtg tgggtgtccg 4500
gggccaggtc ctctgtgccc actccgcagg ctggacctga gagtgcggtc ccgatcccct 4560
gctgagggaa cactggggag gaagaccttc cctggcagga gaagaatcaa gtctggctca 4620
gacttccctt gaaccaggct tctaccagtg ggcatggctg gctaatatac cctaaagcaa 4680
gaaccagccc ccatacactc tccccagcca ctggggtcag cgagcgggct gggggctgcc 4740
tcttggctgg catcggggac gggagggtca cctttctggc cagaaccacg tggagcgctg 4800
tgtgacaccc caggagctca ggcgcacggt ggaaccggat cctccctggc agcagcggct 4860
gcacgtggcg cccaccctct ccgtgtgggg acgtggtagg atgaggctct ctgaggacgc 4920
attctttgtt ccagaacctg gtcccttgct aggcagtaga gctctgtgtg ggtgacttgg 4980
gtccaggctt atggtcaaca gagtgtgaaa cccaggttgc tcccaaggca caaacaggag 5040
caaggtgcac cttgccttga gcccaaggtg gggtgcaggc ccctccctca atccctcacc 5100
tccatctgca gtcaccccca ggctaaatcc atggctagca attctgagag ctcctggaac 5160
cagaaggggt tcaaggcaga gtgggggagg gtcaggactt aaccacttgg ggcaaagatt 5220
tccagggaga attgtttagg ttgcattttt ctcagtgata tgtctttgga attttcagga 5280
gaacgcggtc tccctggagc ccctggaaag gcaggaccag tggggcccaa aggtaaccag 5340
gggacagaca gcgaggaggc cttgagcagg gaccttggag ctgtctgtaa tgaaatgagg 5400
gagggagaga cagagacaga gacacagaca cagacacaga gagacacggc aacagagaga 5460
gaaagacagt ggcaggcgga cagggaccct gggatattcc aggcctcggg gcccctcccc 5520
accattgaga cctgggcgtt gtgatgcctt agtgagcctg cagggctggg gagatgcagt 5580

gtccaggccg ggagcccact ctggaccagc ctttccgtgg cccacacatc tggggtcaaa 5640
agctcattat cctgcctctt cctccaggag accgaggaga gaaggggatg cgtggagaga 5700
aaggtgagcc acccgtgggc ccaggcagtt gtctctgcag gctttgtcca ggggaggggt 5760
ctcagaggcc tcagtccgcc tgcccaccgc ccacgtccct gtcaatccag gctgtgggca 5820
gggaggggtg gaaggagtcc agcgggcttt gttgtcagaa tccacgaggg ctcaaaccct 5880
ggtgtctaca gcagtcactc ccagtggcca gcaggaccac atcagtgcat agatggtgac 5940
acttcaatat ttcaatttct gtcttactta tgctcattta tgcaaaaatt gtaaaagttt 6000
aaaaaagcca aatgtgcaag actctaatac actgtaccac gctccaccca caaacacaaa 6060
tgaaaaccgg agccgggggc tgagggtccg ccgatacctg agtcccaaga aacagaggcc 6120
agggcggagg cagggcaggc ttggagtgac cctgccaggc ttgaagagtt attccgctgt 6180
tatcagtggc gaaggcccca ctaggggtcc agttcgtcca gcaggacccg cagcgcctgc 6240
cacagcccac caagctcaag ctgcacagca ggggcctatg gacaaccatg tggctggtgc 6300
ctctggggaa gtcgagtctg gccatccttc cctaggcctg tgccacgtgt cctggagaca 6360
tcagaggagc tgcctttgcc cattctctat tctgtgctca gggagccact ggggctgacc 6420
aggacaaagg ctctgatttc aagcttatga caagtgtctc ctctccccac acaggagacg 6480
ctgggcagtc tcagtcgtgt gcgacaggtg actgatcaca ccctgacctc ggtacccaag 6540
ccttgccccc acccctgggg ccagctgcac acgttcacgc tacggaatct aaggacagaa 6600
ccctgtctgc acctatcatc cccccctcaa tcactcgagt aatgggcagc tatcacctga 6660
atcccttcca tgacaacctg ggctctacta ttatctaccc tagaaggccg ggtgcccagg 6720
aggcaaaggt ggggcagaca ctgggaggtg ggagggtcgg cagactgggt gtgggcatca 6780
cagctgcgtg gccctggggg ccgtgtccct gtccaggcct cggagtcctg ctcggtccgc 6840
acagaagcct gtggccctcc tcctccctcc tgggcctccc tcccactgcc cctgtcctgc 6900
ccagggcccc cgtcctgcag ccattccccg ggttcccttc ccaggcccac gcaactgcaa 6960
ggacctgcta gaccgggggt atttcctgag cggctggcac accatctacc tgcccgactg 7020
ccggcccctg actgtgctct gtgacatgga cacggacgga gggggctgga ccgtgagtgt 7080
ggggctgggc agaggcggtc agcctgggag tcccggaggc caggctgcac acctggttgt 7140
agaacacact ctggaattct ctattcccct ggatggggac aatcatagat gatgggggaa 7200
gtgacccgtg ggtaaaagtc caggccatcg gggaggctgg gaggggaccg gtgctctcct 7260
gctgtgtggc ttggggcttg ttgcctctct tctctgagac cgcatttcct ctttcatcct 7320
gtggagtctg tgaggagacc agatgggcgt gcgtgtggca ccggcctgtg atgctctgcc 7380
aactacaaat gctgctcctc tggagggcgg gtccccggtg ctgtgggacc tcggcctgcc 7440
ccgcctaact ctgtatcctg gcttcttcac aggttttcca gcggaggatg gatggctctg 7500
tggacttcta tcgggactgg gccgcataca agcagggctt cggcagtcag ctgggggagt 7560
tctggctggg gaatgacaac atccacgccc tgactgccca gggtagggcc actgccgtgg 7620

ctcaggggtt ggggggccct gaggtgctcc tgccccttag gcctgggcca tctgcaacat 7680
aagcacctct tggcccacag gggattgggc cctgagcaca ctcaggtggc actgggacct 7740
ctaagggctg cgtcccctgc cgcaggctct ggcaacatcg ggaagggaaa cggttagagc 7800
cctgggctga aggcctcttc aggtcttggt cctactggca tcttagatcc tgagagtggg 7860
aagcaggaga gagaccactg gggcttgggg tagccttccc ccatctccag ctgttgatct 7920
gcctctgaag ctggcatctt agtcccatgg agagcccacg ctcgcctcct ccagcccacg 7980
cctcagcagg ccccccacag ccgcactcac ctgagtgaga gggcctcccc ccaaccctct 8040
ggggcagggg atgagtttca cagctggtcc tgaggcagtc tgtataattg ctggtccatt 8100
atccctgccc actgccctgt ggcatgattg aaggaatcca ctcaggtgcc tcaaattcct 8160
gcaaaagaaa ctacaattag agcttcgccc actgctgtgt tagtggctca ccaaccctta 8220
gaaggagaag gtacactttc aaaatgaaag aatgctccct tccacccagt gaactccagt 8280
ggggcatgtt cagttctctg aaataacggg aaaggtgttt agaaacagaa agccatccct 8340
gagggcctga tagcaacaag ggattttctg acatttccgg ttgatttttt tcctggatgc 8400
cacgttcaga catcccttaa gaatatatcc atccccagcc gggcaccgtg gctcacacct 8460
gtaatcccag catttgggga ggctgaggct ggtggatcat gaggtgaaga gatcgagacc 8520
atcctgacca acatggtgaa accccatctc tactaaaaat acaaaaaaaa aaaaaagtag 8580
ctgtgtgtgg tggcacacac ctgtaatccc agctactggg gaggctgagg caggagaatt 8640
gcttgaaccc aggaggcgga ggttgcagtg agctgagatg gtgccattgc actccagcct 8700
gggcaacaag agcggaactc tgtctcaaaa aaaaaaaaaa aaaaattacc catccccagc 8760
cctcatgcct ggtgacagaa agatcttaaa ggctggggcc ttaaaatctt tttccaacag 8820
gaagcagcga gctccgtgta gacctggtgg actttgaggg caaccaccag tttgctaagt 8880
acaaatcatt caaggtggct gacgaggcag agaagtacaa gctggtactg ggagcctttg 8940
tcgggggcag tgcgggtgag tgtctgcttg gggctggtgg cctgagtggg gcttggaaag 9000
cagagagaac ctggctctgt ctcccctggt gaccttgggc cctcctctct ctgaatccca 9060
tttctccatc tatgaagcag acactagcat ttgtgtctct gtacagcgtg gggctcacag 9120
gacatagcgg ctggtattcc taaggctatg tctgccgcac agtgagctgc ctgggcaggg 9180
ctgagacgac ccaccagcat cactgctgaa acctggaaga cgtcgtcctt ttctcatggg 9240
tgccttggag ctgtttccca aactgtgctc catggaacac cagccttgtg ggacattctt 9300
taccaaaagg agtctctggc tacacgatct ggggaggcat ttcctacagg gcgtctgctt 9360
agggaattcc cagtgcacat cgttcgtgaa aggcactgag aattgctgca gctggaagtc 9420
tgtgtaacta actgctccat ggagcaaggc agtcccaggg gagccttgga aatgacagcc 9480
ttggagtctg tgcagtcctt tcacaggcac gatctcacgc atgacctcca aggaaggcag 9540
cagggcaggg aaggtcaccc ccattttgca ggtggtaaaa ccgaggctca gggggtgaca 9600
tttctcagct ataggagcca gggatgagct tccggctcag ggcttgagat agaggagaag 9660

cccccaggtg agggctgtcc tgaacctacc aagggccacc aggggacgtg gggagagacc 9720
ggaaggagcg gcgcaggctg gaggagggag gtcattccag gagagagcag gaggcctgac 9780
agtttgcggg agccctggag gaaacaggtg gttgctggag caggggcctc aggttgtgga 9840
gagagatttc cagaccaggt tgccagaggt tgcctcctgc ttccccttcc tccaaggcct 9900
gggtgggtca tttgtgcacc tgtagctcgg ctcatgcgtc gctaaagtga ggttggctct 9960
gctcagaggc aggatgtggt gagggtctca cagtggcagg gctttttcca gcatctgcaa 10020
accccacatt ttctgtgagc aggtaattct ctaacgggcc acaacaacaa cttcttctcc 10080
accaaagacc aagacaatga tgtgagttct tcgaattgtg ctgagaagtt ccaaggagcc 10140
tggtggtacg ccgactgtca tgcttcaaac ctcaatggtc tctacctcat gggaccccat 10200
gagagctatg ccaatggtat caactggagt gcggcgaagg ggtacaaata tagctacaag 10260
gtgtcagaga tgaaggtgcg gcccgcctag acgggccagg acccctccac atgcacctgc 10320
tagtggggag gccacaccca caagcgctgc gtcgtggaag tcaccccatt tccccagcca 10380
gacacactcc catgacgccc acagctgccc ctttgccccc agctcagtca agccgccaca 10440
tgcccacaac ctcaccagag ggagaattat gtttctaaat atgtttactt tgggacagaa 10500
aagttttacc agtttcaatg tgtgtttctt gaacaccagt ggttttgatc actttcctgt 10560
gtctgttgca cggcaggtct ttccctggaa tttggtaagt cgggaagagt caggaccctt 10620
atttagcaga tggcactacc gatgttcagg gaggttgagt gacttttgcg aggattttcc 10680
acctccagta attgacacag ggcgatcctc acctccaccc ccaagcctcc cccatccccc 10740
catgcctcac tcacaacact tcacacttct tgaaaaaggt cactttgggg tccagaaaac 10800
acttactaaa aagggggaaa gtttagtagg aacttttctg cttcccacca cggcagagtt 10860
catgctttgg gctggctggc tgctgggaag agataaacac 10900

<210> 2
<211> 8700
<212> DNA
<213> Homo sapiens

<400> 2
aaggcacctc ggcatcccga tggcagcagg tggcagggtg cggtggtggt agtgggaggt 60
gcagtgctgg ttccgtctct gggggcttta ggtatacggc ctctggggcc ccacctgcta 120
ctgtctctgt ctctaacctg gctcagtcca aggcgagagt gcaccttcag gccagctctt 180
ctccaaaagg cgaacacaac cactgaagta agtccacagc catcctacag gtgggatgtc 240
tggcctcagg gctcaggggg ttccctgggc cctaggagtg aaattcacct actctcatcc 300
tccctacagg ctaagcccct gcattcacac tcaaggtctc cccttcagat gtgtacctgg 360
gtcatagtcc cccaggctcc ccactcttct ctcctttccc tcctgttcat gtgcccctgt 420
gctctacata ctgccccagg aaacagtgga cacacacgca ttttcctctc cttttcaccc 480
tggcagcctg cctggacctc atagtggcag tttccaagcc tcctgaggag agtcccaagt 540

ctgctcatga tgtggtgagg gttactgtgt aaggcccaca ccagtggccc atgaccagcc 600
ctggccatga ggactctagg tattcggata agggagagag acctctttga agcagaacat 660
ctttagcaaa cccttccttg ttccccgttc tcagctcatg gtgacatctg ctcaccgctg 720
aacaaaggtg ggaggatggg cctgggctct ttgcccaagg ccacaagcaa gtcagcctgt 780
tccctggaag gagaccgtcc ccctgcaggg atgacagtcg ccatgggatc cccaaggggc 840
ggggggtagg gagcagccct ggagatgatc tcgcacctcc tgctggcgtc accaagcccg 900
cggagaggaa gcggctgtca ctcggaagat gagaaattgg agtctgaggg aggctggctt 960
ctctagccct ataagagggc aggcaccttt tgaagcaaag accagaagag atggagctgg 1020
acagagctgt gggggtcctg ggcgctgcca ccctgctgct ctctttcctg ggcatggcct 1080
gggctctcca ggcggcagac acctgtccag gtaagggcac tccagggcct cctcctggaa 1140
acttctcgtc cctgaaagct ggcagcttcg tgattggtgg cttcctggct tccaggcctt 1200
gcaccaggcc gtgtggagca tcgtctaacg agacagcagc tctgcatgcc tcatcttatg 1260
tgaacaaacg cagaggcccc gaatggatgt ggcatctcag agacggagct tcctctgctt 1320
gccctcccat aatggaaacc gaggcaggtt tccagtctcc ctgggccaca tgtccccctc 1380
taagcgtaaa aagcgtagtc atggtgttgt gtggggaggt gaggcttgtg gtgccagctg 1440
ggtagacacc agtcagatat tctgccatgg tcgccatcac agagagacag ggtgtgaggt 1500
ggggataagc acccggaaag acagggtgtc aggcggggat gagcactgca ggacctttcc 1560
atccaggacc tgctgggctc tggagatggc agttcccatg gtgtccctga tgcggcacaa 1620
taagggtggc attgtccccg tgagttctgg acctccttcg gggctcccga ggggacactc 1680
gggtgccaaa ccatgggctc cggcgcatgc gagggcaggt tgaaaatgca ggttctggag 1740
tcaggtcctg actccgtcta cccggggctg ggatgcaggg tcaggaattt gtgatgctcc 1800
tgatgcttgc agagattcgt gtcaggattt ctggaatgca tgtgagacac agagctctgc 1860
ggtgtgctgt gactagcatc gcattcaagg agcaatccgt ggcgtgaagt gtgacacagg 1920
ccaggaacag cggggattcg cttggaaact cctagtggct cgttgtggag tggtggggtt 1980
agggtggcat tattttcttc cttaaatact tctagagttt ctaaatgatc aaataatagc 2040
acgtgttatt tttgtaacac actcacttga atttgagtta tctaattaaa aacaacaagc 2100
tgcattccag ctctagtttc gcaccaactt gttcagtgtc cttgaaggac gcctccctct 2160
gtggaggaac aaggtggacc cacaggagcc catccactct cagatctgct ccaagatcag 2220
gcgtgggagt tgcaatggta gcctctctcc ctgtgtcctt ctcacccgat attgattaca 2280
atgatatgat agatgagaac agacgagatt aggatgctgt cttaaatggt aggagctgaa 2340
atgaacctgc acccctcggt aggtaaggga acagaggctc agaggggtta agggacttgt 2400
ttcaggacac acagtactga ggaagtaagg tttgccttca ggcagggtag cctccaaggc 2460
cagggttctg ccccaaatca cctttctgcc tgaccagtgc ccaagaggtg aggacagacc 2520
aagtgcccac cagggcagga aaacctccct tccaggtgac cctccagctg agggaggtgg 2580

ctgtggctat agccatctgc ccagtcctga tgtcaccaag atggcagatg cctttcagtt 2640
gagtggtata tctatggctc agtggagtct tcaggcccag gtgacactga gtggccacct 2700
gtgtttttct gcagaggtga agatggtggg cctggagggc tctgacaagc tcaccattct 2760
ccgaggctgt ccggggctgc ctggggcccc tgggcccaag ggagaggcag gcaccaatgg 2820
aaagagaggt aggtgcaggc atggctgggg gcactggctc ttgctctttt tgaaaccaga 2880
tgctgagttg ggcaacaccc ccaccatggt tcctagatcg agagctgggt caggcccctg 2940
caagagccag gaaaagaact gactcccagg gcccaacagg gttctgacat ggagggagcg 3000
tctttcttag ctatttcatg agctcacatt tggacatgct tctgtagctc tgtctcctag 3060
tccttgatcc aataccagct ttgctcggcc gctggcacga cttcaagctc tgtgtccctc 3120
caccttccca cctcaacacg gaggccatga cactcttctc cacctgtctc agatgaggaa 3180
actgaggctc agagagtcca gggatttcgc tctgttctca cacttggaag gaaaagagcc 3240
gagattctac ccatggtctc tggatttcca gctcccggcc cctgagtgaa caggagagcc 3300
ccgtgaggcc tgggtcctgg atctagaacc ttctggtgca ggatgggcaa gcctgggcgg 3360
ggccacagtc acgtcgtagc acgagcaggg tcatggtcat gattggaaat gacagccgcc 3420
agctccaggg tgggcccttt gatcctgggc tgggcagttt tcctcaagtc agtgtctttg 3480
gaaattgcag gagaacgtgg cccccctgga cctcctggga aggcaggacc acctgggccc 3540
aacggtaagg aggggacaag agtgagaagc ggcttcaagg cccttccaga cctggctgca 3600
gaggaacgtg aggcgggtct tctggggctg ccacgctgtc ctcgccagag ccaacgcctg 3660
cccaggcagg gctcttgggg cttagtccag ggcaggggtc tcagtgtggg ggaaggggtc 3720
tcaatgtgtg tgtgggttct cagtctgggg gagtggggtt atcagtgtgg gagctggtct 3780
caatgtgtgt agggggtctc agtttggggg agtggggtca tcagtgtggg ggcagggttc 3840
tcaatgtcgg aggaggggtt cttggtggtg tagggagagg ttcttggggt gtgtgttggg 3900
ggaggggttc ccggtgggca ggctgtgggg aggtgttatc agcagggagg gaggagctgt 3960
caatatgggg gagagattct gggatggggg agaggttctg attattcggg gagggacttt 4020
tagtgtgggg aggggttctt agtggggggg ctgtctgtat gggggggttc tcaggtaggg 4080
gaaggttttc agtgcaggag catctgccct gtgggtgggc ctgagaccaa atcctgtccc 4140
tctaatgtgg aagtggtctt gagagggtgg ggggggattg tctgcccacc tctctaagcc 4200
tcactcagtc ctggcctctg tcccctgagc atgaggccct gtcttgtccc ctcctcctga 4260
ctctgcttga aaaccaggat cagccccgga tggcttggag aaccatgcca acaagttcca 4320
aacacagaat tagtggaaat tctctgcttt aaaacaaaga attttctgat atattttctt 4380
gaggaataaa ttttgaggaa tcggagaaat acgcagtagg aaattgaagc tccctttact 4440
cactcacctt tacccacaga cctactcaga ataagaccac cctacactca ccaaacccca 4500
ggctcagtca tgggttgtgt ctaaacataa aatacgcagg tttggtgccc acaccgcaaa 4560
caacactccc cacatcatat tactgtgtcc accaagatgg gctgtttgca ggccttgggg 4620

cctcaggaga agcctgaaga tccaggcttc ctttagttgg catgtggagg cctcagcagt 4680
ctctgggatg gtggcctctg cttccccagg aacctctgaa aggggtgcat gtggtggagg 4740
ggccagtgta ggcatccctc gagggaaaga aaaatttggt caacagaacc agggtcaggg 4800
actccttggc tggacccata ccatcacctc ctggggaggc ccagaaaatg gtgtccgcgg 4860
accaatgggg gctgaagggc tctgatttcc aaactgtgac acgtgtgtcc tctctcatcc 4920
atgaacagga gcacctgggg agccccagcc gtgcctgaca ggtgactgac cacccccaca 4980
ctcctcccac ggcttgtggc tgcccttggc tggaagtcca gggtcatacg acgccattgc 5040
cagaatgaag tgacaaatat gaacagaaga aaatggttgc ttgcccgttt ccttgtcccc 5100
taattattca aggaagtgac aaatattgtc cagacaactt acatacactc catggcaatt 5160
tttttctatt tataatctac gccagaaggc cgggtgccca ggagctgaag gtgggggtga 5220
cactgggagg tgggagggcc aggccaactg gctgcggcca tcacagctgc gtggccctgg 5280
gggccgtgtc cctgtccagg cctcagagtc ccgctctgtt catacagacg cctatggccc 5340
tgcttcttcc tcccaggcct ccctcctact gcctgtgccc tgcccagggc tcctgtcctg 5400
cagccattcc ccgggttccc ttcccaggcc cgcgtacctg caaggacctg ctagaccgag 5460
ggcacttcct gagcggctgg cacaccatct acctgcccga ctgccggccc ctgactgtgc 5520
tctgtgacat ggacacggac ggagggggct ggaccgtgag tgtggggctg ggcagaggcg 5580
gtcagcctgg gagtcccgga ggccaggctg cacacctggt gggagaacac actctggaat 5640
tctctattct cctggtcggg gacagtcaga gatgatgggg gagatgaccg gtgggtaaaa 5700
gtccaggcct ttgggggagg ctgggagagg ttgggtgctc tcctgctgtg tggcttgggg 5760
cttgttgcct cccctctctg agcccccatt tcctccttca tcctgtggag tctgtgagga 5820
gaacaggtgg gcgtgctggt gaccccgcct gtgatgctct gccaactaca aatgctgctc 5880
ctctggaggg cgggtccccc gtgctgtggg acgtcggcct ggcccccccg gctcctgtcc 5940
cctggcttct ccacaggttt tccagcggag ggtggatggc tctgtggact tctaccggga 6000
ctgggccacg tacaagcagg gcttcggcag tcggctgggg gagttctggc tggggaatga 6060
caacatccac gccctgaccg cccagggtag ggccgctgct ggggcttggg ggtcgggggc 6120
cctgaatggg ggtgcccctg cctcttgggc ctgggctgcc tggaacacaa gagcctcttg 6180
gcccacaggg gattgggccc tgagcacact cagggtggca ctgggacctc cgagggcttc 6240
gtccctgctg caggctctgg cagcatcggg acgggagagg gtcagagctc tgggatgaag 6300
gcctcttcag gtcttggccc cactggcatc tcaggtcctg agaggggaag gagggacaca 6360
gtggccccat agtggagttt gggcccaacc ccaactctgc ctctaatccg ctaggatttg 6420
ggagtcaaga gtcttgccct gctgctcagc acaccctgcc gggtcagagc tacacaggcc 6480
ccggggatgc tgcggtgctc tccgccctct gcctccatgg agtccagacc tccttccagg 6540
ccctgcccca gatccccagc tcccatgtct aaaggtagag agcccttccg ctgagaccct 6600
gaaacctttc tctaacagga accagcgagc tccgtgtaga cctggtggac tttgaggaca 6660

actaccagtt tgctaagtac agatcattca aggtggccga cgaggcggag aagtacaatc 6720
tggtcctggg ggccttcgtg gagggcagtg cgggtgagtg tctgcttggg gctgtgtggc 6780
ctgggcttct gaggggggtt tgggaagtgg agagagcgtg ctcagtgtcc tggtagcctt 6840
gtggaagagg cctcacctct ctgagccaat tcgtccatct ctacatgcag acactaacat 6900
ctgtgctctg tgtagcatgg gggtcatggg acacatcagt gtatgactgc agaaatccca 6960
gcaagggcat ctggtaggaa ggaagtctct gcagagcagg gacctatgca cggtgacagc 7020
agctgatatc tctgcggggc atggctggaa ggggtctgcc atgatggaat ccagagtaga 7080
gaatgatgat cctgacccct gcctcctgtt cttctgtgaa ataaacatag tacacattag 7140
gtggcagggc tagacctaaa gacaatttgc caagaccact gccaacagca gggcagtatt 7200
cactggagtc atggattgca cttcttggat tgtgcagtgc acaacctgcc taaccataca 7260
tggaggacac accaggccag gcctcaggta taaagactta tactgtctgt aatgatgtta 7320
ctgcctgtaa cgatgctcac atttcctcct gcacaggaga ttccctgacg ttccacaaca 7380
accagtcctt ctccaccaaa gaccaggaca atgatcttaa caccggaaat tgtgctgtga 7440
tgtttcaggg agcttggtgg tacaaaaact gccatgtgtc aaacctgaat ggtcgctacc 7500
tcagggggac tcatggcagc tttgcaaatg gcatcaactg gaagtcgggg aaaggataca 7560
attatagcta caaggtgtca gagatgaagg tgcgacctgc ctagcccagg ccggcctcag 7620
ggtcaggacg cctccacaca tagttggttg gggggtaggg ttgggagctt ggccctacgg 7680
tttgtaaaag aaacacatgt cgtgattcta aattgggttt gtcttgctgt gcggaagagg 7740
ttcaaatcca gctctgtttc cgcaatcaac cctttcagat ttgtgacatt aactcacctc 7800
aaacctctgt ctttccttga caatgagtaa gaatgtgttc ctgaccatgc cagcaggaac 7860
cttccttaag gacaaataca agactttgaa gctgcgatgg gctcagagtg cagccctgct 7920
caggtccctg ggcaggctaa acgccttcct ggggctcgac catctgggct tctctgtgag 7980
ctggatggca gggaagtcag aaggcacatt ggagaggaag tttctcttgc actcagagat 8040
gccgttgcct gtccaaggcc cccagcctcg tagccgcagc tctgctcact ctcccccgtc 8100
acccaggctt cctgccgcct gctgtcctgg gtattcgtgg gatctgctcg ctacttcctc 8160
acaagccgaa gcaagagtgg cagtgtgcct gtgactccct catcaaaacg gagagagtgg 8220
ttacatgagg caggtgtgag ggacccgagc actcctgagg agggaagaac actcctcttg 8280
gccatgagtg tccatcccaa agctgccctg cagcctagcc cagtgcagat tgaatcagaa 8340
agccaaagcc ccttccaccc gcttctctca ggcaagctgc ctgcaagaga gaaaagtgta 8400
ttagtgcagg agcttgtgtc tgctgaggat ggcacctggc cctctcctct caccacgtcc 8460
gggtcctcct gaggaccggt cccctgtccc cggtccccag ccagctcagc ctgaccaggc 8520
ttttagaggc tccgcacccc agcatcggcc agtgctgacc cttgtacggc tgtttgggtc 8580
cccgatcact ctcataacag ccctgccctt gtcctatacc ctctactcag agaggatcca 8640
gaagggtcca aggaggccat gatccctccc tgagcttttt cacgcgggcc ccctccttcc 8700

<210> 3
<211> 8200
<212> DNA
<213> Homo sapiens

<400> 3
acactctagc ctaggtgaga gagtgagacc ctgtctccaa aaaaaaaaaa aaaaaaagag 60

agaacaactc cttccctggt cctactctcc agaaataacc acagttcaca aggtctgtgt 120

cagacctctt tctgtgaatt tacaaaatac atatatacac acctctagca ttttaaaaat 180

acaaagtggg ataatgctgt gcatactgtt ttgcattttt gcccctctca ttttagatca 240

gattcaccct gtgagttgtc tgaatggtcc taattcatgt gaatgccatc aatgactggt 300

tgggtcattt ccacactgat gcttacaaac agcgccatgg taaacaggct tgtctgcaga 360

tcttggcata ctcgtgggac agtctgggga agacagattc ctagaactgg aattgcggtc 420

tcaggatatc cctctcgcgg acacatcctg tcaggcaaac actgcctcca cttcccctga 480

taccacttgc acctcagccc tctccataag caccagggag gtgcagagga ggccttgagg 540

gagggtttcc tgaatgagtg aatgaatgag ccctgcctct cctcccttat cacctgaggt 600

ccctctccag ctcaggcctg atccttcaca tctggaccct ggctgctgcg tcctccctgg 660

cttcccggcc tccaggcctt cctacatggg cttcccgctc aattggcgta cattagcagc 720

tgtttcctaa gcctcacggg actcaaggtt gagcatggag tcactttctt tttttttgag 780

acggagtttt tgctctttcg cccaggctgg agtgcagtgg tgcgatctcg gctcattgca 840

acctctgcct tcgggtttca agtgattctc ttgcctcagc ctcctgagta gctgggatta 900

caggtgcatg ccaccaagcc cggctaattt ttgtattttt agtagagaca gggtttcacc 960

gtgttagcca ggctggtctc gaacccctga cctcaggtga tctgcctgcc tcggactccc 1020

aaaatcccgg gattacaggc atgagccacc gcagccagcc agaatcactt tcttaatggt 1080

gcttttccct ggcgggggca tggggcttgg gtaaaactct gttgctctca gcatataagt 1140

ggccaagaga acagaagtca cgtcccatca ctgggcctca gtttctcttt ccttattcat 1200

ttaactcttt cttactgagc acccattttg taccaggccc ataatgcaat aataaggatt 1260

aatagcaagt ccatgtgagg atcggaggtg acgtcagtat gacgctggaa tgcagtagat 1320

gcccaggaaa tggtagcagt tgtcactatt catagcattg acaaggatca gggaaggatt 1380

cccagtggag atggctgtag agctagacct tcatggacag atggggcttc acaggtagag 1440

acgactgtga atgtagaggt ctggagagat ggctggggag ccctggaagc caggatcaga 1500

agttggactc tttcagtgtg cggtggctca cacctgtaat cctagcactt tgggaggccg 1560

aggtgggtgg atcacctaag gtcaggattt cgagaccagc ctggccaaca tggtgaaacc 1620

ccttctatac taaaaaaaaa aatacaaaaa attagctggg cttggtggcg tgcacccata 1680

atcccagcta cttgggaggc tgaggcagga gaatcacttg aacccgggag gcagaggttg 1740

cattggttgc attgagctga gagcacgcca ttgcattcca gcctgggcaa caagagtgaa 1800

actccgtctc aaaaaaacaa aaaaacaaaa aacaaaaaac aaaaagatgt tggactccat 1860

cctggttgga tagggcaggg ataatgccct ttctctctgg ggctgaagga ggaaatactc 1920
ccaccccctg ctggctccag tctgggtggg gtgtccctgg gggacaatgg gaggaagtct 1980
tgtttcatgt cctcccacca gcctggcccc cttataatct ggcatccttc ccctggtggg 2040
cccagcaaga tggatctact gtggatcctg ccctccctgt ggcttctcct gcttgggggg 2100
cctgcctgcc tgaagaccca ggaacacccc agctgcccag gtgggcacct agggtgccct 2160
cctcaccaac ccactcctca ttccctcgct gggtgtcccc ccaccaaggc tcatcaacct 2220
caaggagcag ggtgggagtt tcataatctg ggctctgcat gtaaatatat gcaaatggat 2280
gcaaagcaca cgaaaccttg ccagctcagg agacaagcat cctgacagtg ggaatctggg 2340
agacaggaag agaactgaag aggggtctgg aagagacagt ctaccctgtg cccaccctgc 2400
ctgtgtctcc ctgtaggacc cagggaactg gaagccagca aagttgtcct cctgcccagt 2460
tgtcccggag ctccaggaag tcctggggag aagggagccc caggtcctca aggtgagagg 2520
ggctggggag cgggctggga tcttgggaat ggggaaggca ggaaagtagc aatttttgtc 2580
tgtgctgagg ggccacgaca aaggagcaag gaacctactc tgtgccagct ccaggctggg 2640
cacccggcat cctaagatca actctgccag gtaagtggtt tcatccccat tctgctgagg 2700
aggaaaggga ggttcagaga ggataagata tttgcccgca atcacaaagt gagtgaaggg 2760
tgaaactggg atttgaacct ctggctccaa gtctcttgtt tctttttggt cctgggcatg 2820
gggtgggact caggaaatct ctgcctttgt gatttcaggg ccacctggac caccaggcaa 2880
gatgggcccc aagggtgagc caggtgagta agtggggctg gaggcctccc cgctttcttt 2940
gcctctgtcc ctggactcta gggtgggatc tctgcttggc tgctgtgcct atgatggctc 3000
tctcacaggg gaatggatgg ctgggaggag tgggaaggat taggagaagg gcctggctac 3060
cctatgatgg ccctcctatt ggggatggag ggctgggagg agtggggagg attaggagaa 3120
gggcctgggt accctatgaa ggccctccta cagggtttgg aaggctggga ggagtggaga 3180
ggattaggag aagggcctgg gtaccctatg atggccctcc tacagggtat ggaaggctgg 3240
gaggagtggg aaggattcgg aaaagggcct gggtaccggc tccactggtg gctcagcgct 3300
ggggtgctga gacatcagag ccaacactca tcccttctct ggggcaggag atccagtgaa 3360
cctgctccgg tgccaggaag gtgaggcagc ccttgcagga gccaggaact tgggatcaca 3420
tggtgttggg tgttggcaga cccccaagtg tggccaagat cctccccaca gcctcctcag 3480
aacccttctt cagggacctg aaggagccaa gagggtccct agggtcccac acttgttcca 3540
gtgcctgggg gtgggggtca gggaataacc tgggcaccac cccagtccat ctcccctttc 3600
ccaggcccca gaaactgccg ggagctgttg agccagggcg ccaccttgag cggctggtac 3660
catctgtgcc tacctgaggg cagggccctc ccagtctttt gtgacatgga caccgagggg 3720
ggcggctggc tggtgagtgt ctgagaaatg ggctaacagg ccaggcccac tggtcacttc 3780
tcccccttgg cccctagggg agccagaacc caccagagca aggcatttcc tagaggaatt 3840
cactgacaaa tgtttagtga gcccccaatg ggacctgaag aagtatgagg gcttcacaga 3900

ggaggtgcct tttgaaaaga ccttaagttg agtgcagtag ctcacgcctg taatcccagc 3960
tacttgggag gctgaggcaa gagaattgcc tgaacccagg aggtggaggt tgcagtgagc 4020
cgagttcttg ccattgcact ccagcctggg tgaaaagagt gaaactctgt ctcaaaaaaa 4080
aaaaaaaaaa aaaatagggt agggaggggc attccagcta gaagtaaaga taaactcagg 4140
gaggcagcag agtaaaaggt ttgcagggaa cgcgtgagtg agctgagagc aggcgtgggt 4200
gtcggctaaa gtctgccggc actgacggaa catggattga ggacctctgt aaaggccagt 4260
tcatggaagg cactaaatga tcaacttaag aagtgtgcag cgcgcccaga cgcggtggtt 4320
ggctggacgt ggtggctcac gcctataatc ccagcacttt gggaggccaa ggcgggcaga 4380
tcacctgagg tcaagagttc gagatcagcc tggccaacat ggtgaaaccc catctttact 4440
aaaaatataa aaattagcca ggcgtgatgg tgggcgcctg taatccaagt tattcaggag 4500
gctgagctgg gagaattgct tgaaccccgg aggtggtggt tgcagtgagc caagattgag 4560
tcactgcact ccagcctggg caacggagtg agactctgtc ttaaaaaaaa aaaaaaaaaa 4620
gaagagtgtg cagggcaatg ggtagcccca ggagagtttt aagcagggga ggggccaagt 4680
agaacttctg ttttaaaaag atgattcggg ctacaccatg aaagatgcac tgcattgggg 4740
gaccagtgga ggcagggaca tggtttagga agacagcaag gagccaggca gtaaagacag 4800
tggagcaggc ctgacacagt agctcacacc tgtaatccca gcactttggg aggccgaggt 4860
gggtggattg cctgagctca ggagttcaag accagcctgg gcaacatggc aaaaccccat 4920
ctctactaaa aatacaaaaa attagctggg cgtggtggca ggtgcctgta atcccagcta 4980
tgcagaaagc taaggcacga gaattgcttg aaccagggag gcagaggttg cagtgagccg 5040
agatcacacc actgtactcc agcctggtaa cagggggtga gactctatct caaaaaaata 5100
aataaataaa ataagacagt ggagcaatgg aaatagaagt acttaggttg acaactctat 5160
aggacctggg gcctacttgg cttggagggg gaggtggaag acatcagggt aaggccaggg 5220
gtccagcttg ggctgtggga gtggcctctc taatgtaaaa tatcttggtt tttttgtttg 5280
tttgtttgtt ttgagacgga gtcttgctct gctgcccagg ctggagtgca gtggtgtgat 5340
ctcggctcac tgcaacctcc acctcccagg ttcaagcagt tcttgtgcct cagcctcctg 5400
agtagctgga actacaggtg tgagccacca cacccggcta atatttgtat ttttagtaga 5460
gacgggattt caccatgttg gccaggctgg tttcaaactc ctgacctcaa gtgatccacc 5520
cgcctcggcc tcccaaagtg ctggaattac aggcatgagc caccaagcgt tcttggttct 5580
tatagtaaat gaaattgggt tattttgact gacacccagc agcgctggcc atctcctgtg 5640
ggtggggaca gtgttttgtc aattcagtct tcccagggct tgcaaggctg tggcatgaag 5700
ttgagtgtca gtaaaagtta acaagggaat gtagagttca tagaaatatt caaagctaac 5760
ttgaaggatc cccattttat caacaggccc caatccaaag agagagtggg tatccaccca 5820
cccctcacag ttcctgcacc ctcagctgcc agtgcccagc ctggagtaat agctgctgtg 5880
ccccccctcc cccaggtgtt tcagaggcgc caggatggtt ctgtggattt cttccgctct 5940

tggtcctcct acagagcagg ttttgggaac caagagtctg aattctggct gggaaatgag 6000
aatttgcacc agcttactct ccagggtgag ttcccagcag ggtcctggag tctgggggtt 6060
actgtctgcc atcctgaatc cctgccccac ccccaagaac tgaggattcc tccatccctg 6120
cctccccttc tttccaggta actgggagct gcgggtagag ctggaagact ttaatggtaa 6180
ccgtactttc gcccactatg cgaccttccg cctcctcggt gaggtagacc actaccagct 6240
ggcactgggc aagttctcag agggcactgc aggtgagtga gcctaagggg agcagaggaa 6300
ggaggctgga tcctttccca gtgctgccac tgcccccatg aatgacccag ggcaatcctg 6360
tctcctctgt gggccttagt ttccgcatct gagaaatggc aatcatgaaa caggatggtg 6420
caacagtgaa gaatttggga tctggaggca aacagacctg agtttgaatc catctctgcc 6480
atttagtact tatgtgacct tgggcaagtc ctacccctcc actgagcctc agtttcctcc 6540
tctataaaat gggcagggag gccgggcacg gtggctcatg cctgtaaccc tagcactctg 6600
ggaggccgag gcgggcagat aacttgaggt caggagttca aacccaacct ggccaacatg 6660
gtgaaacccc gtctctacta aaaatacaaa aaaattagcc gggcgtggtg gagggtgcct 6720
gtaatcccag ctactcggga ggctgaggca ggagaatcac ttaaatctgg gaggcagagg 6780
ttgcagcgag ccaagagagt gtcactgcac tctagcctgg gcgacagagc gagactccat 6840
ctcagaaaaa aaaaaaaagg cggggggagc gcagggaggc aaggaggaag ttttgggtgc 6900
cgcctgaggc tctgttgagt gttagtgttc tctgctttgg aagaggaacc acggaagtct 6960
tctttcagag aaacagtgac tattggggag agatttaaaa gagggaagtg gttatgaggg 7020
agtgcctcag ggaaccacct ttcctggcca ggcacctaag aggttcctcc cagcttttcc 7080
tagcagagtt gctcagactg gatgggagct gagagaagac aaaggagcta gagggggccc 7140
ggggattctg gagggggtaa gacaggggga aaagctggga gctttggagc cagacagtcc 7200
cgagttcaag tcctgccgct gcttcaacca gccgagtaac cctgcgcaaa caacctggcc 7260
ccttggtgtt gctcgtgggg cctgataaaa ttcaactctt agttgctctg agactcccat 7320
ggcattatat ctccaaaggt gccaggcgtg gtgctgggca ccaaggtgct ttccatccct 7380
tccctgctag gggattccct gagcctccac agtgggaggc cctttaccac ctatgacgct 7440
gaccacgatt caagcaacag caactgtgca gtgattgtcc acggtgcctg gtggtatgca 7500
tcctgttacc gatcaaatct caatggtcgc tatgcagtgt ctgaggctgc cgcccacaaa 7560
tatggcattg actgggcctc aggccgtggt gtgggccacc cctaccgcag ggttcggatg 7620
atgcttcgat agggcactct ggcagccagt gcccttatct ctcctgtaca gcttccggat 7680
cgtcagccac cttgcctttg ccaaccacct ctgcttgcct gtccacattt aaaaataaaa 7740
tcattttagc cctttcaaga ctcttccagt tactgggaaa aaaccctaga gggcagagca 7800
gggacacaag tttcagctcc attcagggaa gaatttcctt ccagtcagga agatggaaag 7860
gacagcctct aaagtagtga ggcttctgtc tctggaggtg tgcaagacaa ggccagaggg 7920
gcatatagtg gaaggactgg aggagattca agcatccatt gacaatttcc tgaggctctc 7980

tgggtcctgc aagtggatgg ctttgtggca ggaattgcac aattgcacag cttttttgtt 8040

tgtttgtttg tttgtttgtt tttgagacgc agtctcgctc tgtcgcccag gctggagtgc 8100

agtggcacca tcctggctca ctgcaagctc tgcctcccag gttcacgcca ttctcctgcc 8160

tcagcctccc gagtagctgg aactacaggc acccgttacc 8200

<210> 4
<211> 5
<212> PRT
<213> Homo sapiens

<400> 4

Gln Arg Arg Ala Ala
1               5

<210> 5
<211> 15
<212> DNA
<213> artificial

<220>
<223> degenerate code

<220>
<221> misc_feature
<222> (3)..(15)
<223> N = any nucleotide, W = A or C, Y = A or G and V = any
nucleotide if the first nucleotide in codon is C; A or G if the
first nucleotide in the codon is A

<400> 5
caywgvwgvg cngcn 15

<210> 6
<211> 5
<212> PRT
<213> Homo sapiens

<400> 6

Gln Lys Arg Ala Ala
1               5

<210> 7
<211> 15
<212> DNA
<213> artificial

<220>
<223> degenerate code

<220>
<221> misc_feature
<222> (3)..(15)
<223> N = any nucleotide, W = A or C, Y = A or G and V = any
nucleotide if the first nucleotide in codon is C; A or G if the
first nucleotide in the codon is A

<400>  7
cayaaywgvg cngcn                                                        15


<210>  8
<211>  5
<212>  PRT
<213>  Homo sapiens

<400>  8

Arg Arg Arg Ala Ala
1               5


<210>  9
<211>  15
<212>  DNA
<213>  artificial

<220>
<223>  degenerate code


<220>
<221>  misc_feature
<222>  (1)..(15)
<223>  N = any nucleotide, W = A or C,  Y = A or G and V = any
       nucleotide if the first nucleotide in codon is C; A or G if the
       first nucleotide in the codon is A

<400>  9
wgvwgvwgvg cngcn                                                        15


<210>  10
<211>  5
<212>  PRT
<213>  Homo sapiens

<400>  10

Asp Glu Arg Ala Ala
1               5


<210>  11
<211>  3615
<212>  DNA
<213>  Homo sapiens

<400>  11
agtggctttt tggaggtgtc tcggccatga cacacatttg acatgccctc cctcaaccta      60

cttatagact atttttcttg ctctgcagca tggaccaaag agaaattctg cagaagttcc     120

tggatgaggc ccaaagcaag aaaattacta aagaggagtt tgccaatgaa tttctgaagc     180

tgaaaaggca atctaccaag tacaaggcag acaaaaccta tcctacaact gtggctgaga     240

agcccaagaa tatcaagaaa aacagatata aggatatttt gccctatgat tatagccggg     300

tagaactatc cctgataacc tctgatgagg attccagcta catcaatgcc aacttcatta     360

agggagttta tggacccaag gcttatattg ccacccaggg tcctttatct acaaccctcc     420

tggacttctg gaggatgatt tgggaatata gtgtccttat cattgttatg gcatgcatgg     480

agtatgaaat gggaaagaaa aagtgtgagc gctactgggc tgagccagga gagatgcagc 540
tggaatttgg ccctttctct gtatcctgtg aagctgaaaa aaggaaatct gattatataa 600
tcaggactct aaaagttaag ttcaatagtg aaactcgaac tatctaccag tttcattaca 660
agaattggcc agaccatgat gtaccttcat ctatagaccc tattcttgag ctcatctggg 720
atgtacgttg ttaccaagag gatgacagtg ttcccatatg cattcactgc agtgctggct 780
gtggaaggac tggtgttatt tgtgctattg attatacatg gatgttgcta aaagatggga 840
taattcctga gaacttcagt gttttcagtt tgatccggga aatgcggaca cagaggcctt 900
cattagttca aacgcaggaa caatatgaac tggtctacaa tgctgtatta gaactattta 960
agagacagat ggatgttatc agagataaac attctggaac agagagtcaa gcaaagcatt 1020
gtattcctga gaaaaatcac actctccaag cagactctta ttctcctaat ttaccaaaaa 1080
gtaccacaaa agcagcaaaa atgatgaacc aacaaaggac aaaaatggaa atcaaagaat 1140
cttcttcctt tgactttagg acttctgaaa taagtgcaaa agaagagcta gttttgcacc 1200
ctgctaaatc aagcacttct tttgactttc tggagctaaa ttacagtttt gacaaaaatg 1260
ctgacacaac catgaaatgg cagacaaagg catttccaat agttggggag cctcttcaga 1320
agcatcaaag tttggatttg ggctctcttt tgtttgaggg atgttctaat tctaaacctg 1380
taaatgcagc aggaagatat tttaattcaa aggtgccaat aacacggacc aaatcaactc 1440
cttttgaatt gatacagcag agagaaacca aggaggtgga cagcaaggaa aacttttctt 1500
atttggaatc tcaaccacat gattcttgtt ttgtagagat gcaggctcaa aaagtaatgc 1560
atgtttcttc agcagaactg aattattcac tgccatatga ctctaaacac caaatacgta 1620
atgcctctaa tgtaaagcac catgactcta gtgctcttgg tgtatattct tacatacctt 1680
tagtggaaaa tccttatttt tcatcatggc ctccaagtgg taccagttct aagatgtctc 1740
ttgatttacc tgagaagcaa gatggaactg tttttccttc ttctctgttg ccaacatcct 1800
ctacatccct cttctcttat tacaattcac atgattcttt atcactgaat tctccaacca 1860
atatttcctc actattgaac caggagtcag ctgtactagc aactgctcca aggatagatg 1920
atgaaatccc ccctccactt cctgtatgga cacctgaatc atttattgtg gttgaggaag 1980
ctggagaatt ctcaccaaat gttcccaaat ccttatcctc agctgtgaag gtaaaaattg 2040
gaacatcact ggaatggggt ggaacatctg aaccaaagaa atttgatgac tctgtgatac 2100
ttagaccaag caagagtgta aaactccgaa gtcctaaatc agaactacat caagatcgtt 2160
cttctccccc acctcctctc ccagaaagaa ctctagagtc cttctttctt gccgatgaag 2220
attgtatgca ggcccaatct atagaaacat attctactag ctatcctgac accatggaaa 2280
attcaacatc ttcaaaacag acactgaaga ctcctggaaa aagtttcaca aggagtaaga 2340
gtttgaaaat tttgcgaaac atgaaaaaga gtatctgtaa ttcttgccca ccaaacaagc 2400
ctgcagaatc tgttcagtca aataactcca gctcatttct gaattttggt tttgcaaacc 2460
gtttttcaaa acccaaagga ccaaggaatc caccaccaac ttggaatatt taataaaact 2520

ccagatttat aataatatgg gctgcaagta cacctgcaaa taaaactact agaatactgc 2580

tagttaaaat aagtgctcta tatgcataat atcaaatatg aagatatgct aatgtgttaa 2640

tagcttttaa aagaaaagca aaatgccaat aagtgccagt tttgcatttt catatcattt 2700

gcattgagtt gaaaactgca aataaaagtt tgtcacttga gcttatgtac agaatgctat 2760

atgagaaaca cttttagaat ggatttattt ttcatttttg ccagttattt ttattttctt 2820

ttacttttct acataaacat aaacttcaaa aggtttgtaa gatttggatc tcaactaatt 2880

tctacattgc cagaatatac tataaaaagt taaaaaaaaa acttactttg tgggttgcaa 2940

tacaaactgc tcttgacaat gactattccc tgacagttat ttttgcctaa atggagtata 3000

ccttgtaaat cttcccaaat gttgtggaaa actggaatat taagaaaatg agaaattata 3060

tttattagaa taaaatgtgc aaataatgac aattatttga atgtaacaag gaattcaact 3120

gaaatcctga taagttttaa ccaaagtcat taaattacca attctagaaa agtaatcaat 3180

gaaatataat agctatcttt tggtagcaaa agatataaat tgtatatgtt tatacaggat 3240

ctttcagatc atgtgcaatt tttatctaac caatcagaaa tactagttta aaatgaattt 3300

ctatatgaat atggatctgc cataagaaaa tctagttcaa ctctaatttt atgtagtaaa 3360

taaattggca ggtaattgtt tttacaaaga atccacctga cttcccctaa tgcattaaaa 3420

atatttttat ttaaataact ttatttataa cttttagaaa catgtagtat tgtttaaaca 3480

tcatttgttc ttcagtattt ttcatttgga agtccaatag ggcaaattga atgaagtatt 3540

attatctgtc tcttgtagta caatgtatcc aacagacact caataaactt tttggttgtt 3600

aaaaaaaaaa aaaaa 3615

<210> 12
<211> 20
<212> DNA
<213> artificial

<220>
<223> oligo

<400> 12
agtggtgatg acaagcatcc 20

<210> 13
<211> 20
<212> DNA
<213> artificial

<220>
<223> oligo

<400> 13
agacatctaa gtgtcccagg 20

<210> 14
<211> 20
<212> DNA
<213> artificial

<220>
<223>  oligo

<400>  14
gctcagaggc aggatgtggt                                                   20


<210>  15
<211>  20
<212>  DNA
<213>  artificial

<220>
<223>  oligo

<400>  15
tggaggggtc ctggcccgtc                                                   20


<210>  16
<211>  20
<212>  DNA
<213>  artificial

<220>
<223>  oligo

<400>  16
cacaatcaac gataaccagg                                                   20


<210>  17
<211>  20
<212>  DNA
<213>  artificial

<220>
<223>  oligo

<400>  17
cctggttatc gttgattgtg                                                   20


<210>  18
<211>  20
<212>  DNA
<213>  artificial

<220>
<223>  oligo

<400>  18
cacaatcaat gataaccagg                                                   20


<210>  19
<211>  20
<212>  DNA
<213>  artificial

<220>
<223>  oligo

<400>  19
cctggttatc attgattgtg                                                   20


<210>  20

<211>  20
<212>  DNA
<213>  artificial

<220>
<223>  oligo

<400>  20
accaggctcc ttggaacttc                                                    20


<210>  21
<211>  20
<212>  DNA
<213>  artificial

<220>
<223>  oligo

<400>  21
gaagttccaa ggagcctggt                                                    20


<210>  22
<211>  20
<212>  DNA
<213>  artificial

<220>
<223>  oligo

<400>  22
accaggctcc ctggaacttc                                                    20


<210>  23
<211>  20
<212>  DNA
<213>  artificial

<220>
<223>  oligo

<400>  23
gaagttccag ggagcctggt                                                    20


<210>  24
<211>  15
<212>  DNA
<213>  Homo sapiens


<220>
<221>  misc_feature
<222>  (15)..(15)
<223>  N = any nucleotide

<400>  24
cagaggcggg ccgcn                                                         15


<210>  25
<211>  15
<212>  DNA
<213>  Homo sapiens

<220>
<221> misc_feature
<222> (15)..(15)
<223> N = any nucleotide

<400> 25
cagaagcggg ccgcn 15


<210> 26
<211> 15
<212> DNA
<213> Homo sapiens


<220>
<221> misc_feature
<222> (15)..(15)
<223> N = any nucleotide

<400> 26
cggaggcggg ccgcn 15


<210> 27
<211> 15
<212> DNA
<213> Homo sapiens


<220>
<221> misc_feature
<222> (15)..(15)
<223> N = any nucleotide

<400> 27
cggaggcgtg ccgcn 15


<210> 28
<211> 15
<212> DNA
<213> Homo sapiens


<220>
<221> misc_feature
<222> (15)..(15)
<223> N = any nucleotide

<400> 28
gacgagcggg ccgcn 15


<210> 29
<211> 15
<212> DNA
<213> artificial

<220>
<223> degenerate code


<220>
<221> misc_feature
<222> (3)..(15)
<223> N = any nucleotide, S = T or C, W = A or C, Y = A or G and V =
any nucleotide if the first nucleotide in codon is C; A or G if

the first nucleotide in the codon is A

<400>  29
gasgaywgvg cngcn                                                        15